# EUROPEAN PATENT SPECIFICATION

(11) **EP 2 376 490 B1**
(45) Date of publication and mention of the grant of the patent: **23.01.2013**
(21) Application number: 09768413.8
(22) Date of filing: 04.12.2009
(51) Int. Cl.: C07D 471/04, C07D 487/04, C07D 498/04, C07D 513/04, A61K 31/4188, A61K 31/437, A61K 31/4985, A61K 31/5025, A61K 31/519, A61P 29/00, A61P 37/02, A61P 19/02, A61P 9/10

(54) **IMIDAZOPYRIDINE COMPOUNDS**
IMIDAZOPYRIDINVERBINDUNGEN
COMPOSÉS IMIDAZOPYRIDINES

(30) Priority: 04.12.2008 SE 0850116; 23.12.2008 US 203562 P
(43) Date of publication of application: 19.10.2011
(73) Proprietor: Proximagen Limited, London EC1M 3LN (GB)
(72) Inventor: NILSSON, Marianne, S-762 94 Rimbo (SE); HARALDSSON, Martin, S-187 72 Täby (SE); HENRIKSSON, Sofia, S-169 30 Solna (SE); EMOND, Rikard, S-133 43 Saltsjöbaden (SE); SAVORY, Edward, Cambridgeshire CB3 5JA (GB); SIMPSON, Iain, Cambridge CB4 2LZ (GB)
(74) Representative: Clarke, Lionel Paul
(86) International application number: PCT/GB2009/002823
(87) International publication number: WO 2010/064020

(56) References cited:
- WO-A-02/38153

## Description

### FIELD OF THE INVENTION

The present invention relates to new fused imidazole compounds of formula (I), which are inhibitors of SSAO activity. The invention also relates to pharmaceutical compositions comprising these compounds and to the use of these compounds in the treatment or prevention of medical conditions wherein inhibition of SSAO activity is beneficial, such as inflammatory diseases and immune disorders.

### BACKGROUND ART

Semicarbazide-sensitive amine oxidase (SSAO), otherwise known as Vascular Adhesion Protein-1 (VAP-1) or Amine Oxidase, Copper Containing 3 (AOC3), belongs to the copper-containing amine oxidase family of enzymes (EC.1.4.3.6). Members of this enzyme family are sensitive to inhibition by semicarbazide and utilize cupric ion and protein-derived topa quinone (TPQ) cofactor in the oxidative deamination of primary amines to aldehydes, hydrogen peroxide, and ammonia according to the following reaction:

R-CH₂-NH₂ + O₂ → R-CHO + H₂O₂ + NH₃

Known substrates for human SSAO include endogenous methylamine and aminoacetone as well as some xenobiotic amines such as benzylamine [Lyles, Int. J. Biochem. Cell Biol. 1996, 28, 259-274; Klinman, Biochim. Biophys. Acta 2003, 1647(1-2), 131-137; Mátyus et al., Curr. Med. Chem. 2004, 11(10), 1285-1298; O'Sullivan et al., Neurotoxicology 2004, 25(1-2), 303-315]. In analogy with other copper-containing amine oxidases, DNA-sequence analysis and structure determination suggest that the tissue-bound human SSAO is a homodimeric glycoprotein consisting of two 90-100 kDa subunits anchored to the plasma membrane by a single N-terminal membrane spanning domain [Morris et al., J. Biol. Chem. 1997, 272, 9388-9392; Smith et al., J. Exp. Med. 1998, 188, 17-27; Airenne et al., Protein Science 2005, 14, 1964-1974; Jakobsson et al., Acta Crystallogr. D Biol. Crystallogr. 2005, 61(Pt 11), 1550-1562].

SSAO activity has been found in a variety of tissues including vascular and non-vascular smooth muscle tissue, endothelium, and adipose tissue [Lewinsohn, Braz. J. Med. Biol. Res. 1984, 17, 223-256; Nakos & Gossrau, Folia Histochem. Cytobiol. 1994, 32, 3-10; Yu et al., Biochem. Pharmacol. 1994, 47, 1055-1059; Castillo et al., Neurochem. Int. 1998, 33, 415-423; Lyles & Pino, J. Neural. Transm. Suppl. 1998, 52, 239-250; Jaakkola et al., Am. J. Pathol. 1999, 155, 1953-1965; Morin et al., J. Pharmacol. Exp. Ther. 2001, 297, 563-572; Salmi & Jalkanen, Trends Immunol. 2001, 22, 211-216]. In addition, SSAO protein is found in blood plasma and this soluble form appears to have similar properties as the tissue-bound form [Yu et al., Biochem. Pharmacol. 1994, 47, 1055-1059; Kurkijärvi et al., J. Immunol. 1998, 161, 1549-1557]. It has recently been shown that circulating human and rodent SSAO originates from the tissue-bound form [Göktürk et al., Am. J. Pathol. 2003, 163(5), 1921-1928; Abella et al., Diabetologia 2004, 47(3), 429-438; Stolen et al., Circ. Res. 2004, 95(1), 50-57], whereas in other mammals the plasma/serum SSAO is also encoded by a separate gene called AOC4 [Schwelberger, J. Neural. Transm. 2007, 114(6), 757-762].

The precise physiological role of this abundant enzyme has yet to be fully determined, but it appears that SSAO and its reaction products may have several functions in cell signalling and regulation. For example, recent findings suggest that SSAO plays a role in both GLUT4-mediated glucose uptake [Enrique-Tarancon et al., J. Biol. Chem. 1998, 273, 8025-8032; Morin et al., J. Pharmacol. Exp. Ther. 2001, 297, 563-572] and adipocyte differentiation [Fontana et al., Biochem. J. 2001, 356, 769-777; Mercier et al., Biochem. J. 2001, 358, 335-342]. In addition, SSAO has been shown to be involved in inflammatory processes where it acts as an adhesion protein for leukocytes [Salmi & Jalkanen, Trends Immunol. 2001, 22, 211-216; Salmi & Jalkanen, in "Adhesion Molecules: Functions and Inhibition" K. Ley (Ed.), 2007, pp. 237-251], and might also play a role in connective tissue matrix development and maintenance [Langford et al., Cardiovasc. Toxicol. 2002, 2(2), 141-150; Göktürk et al., Am. J. Pathol. 2003, 163(5), 1921-1928]. Moreover, a link between SSAO and angiogenesis has recently been discovered [Noda et al., FASEB J. 2008, 22(8), 2928-2935].

Several studies in humans have demonstrated that SSAO activity in blood plasma is elevated in conditions such as congestive heart failure, diabetes mellitus, Alzheimer's disease, and inflammation, [Lewinsohn, Braz. J. Med. Biol. Res. 1984, 17, 223-256; Boomsma et al., Cardiovasc. Res. 1997, 33, 387-391; Ekblom, Pharmacol. Res. 1998, 37, 87-92; Kurkijärvi et al., J. Immunol. 1998, 161, 1549-1557; Boomsma et al., Diabetologia 1999, 42, 233-237; Meszaros et al., Eur. J. Drug Metab. Pharmacokinet. 1999, 24, 299-302; Yu et al., Biochim. Biophys. Acta 2003, 1647(1-2), 193-199; Mátyus et al., Curr. Med. Chem. 2004, 11(10), 1285-1298; O'Sullivan et al., Neurotoxicology 2004, 25(1-2), 303-315; del Mar Hernandez et al., Neurosci. Lett. 2005, 384(1-2), 183-187]. The mechanisms underlying these alterations of enzyme activity are not clear. It has been suggested that reactive aldehydes and hydrogen peroxide produced by endogenous amine oxidases contribute to the progression of cardiovascular diseases, diabetic complications and Alzheimer's disease [Callingham et al., Prog. Brain Res. 1995, 106, 305-321; Ekblom, Pharmacol. Res. 1998, 37, 87-92; Yu et al., Biochim. Biophys. Acta 2003, 1647(1-2), 193-199; Jiang et al., Neuropathol Appl Neurobiol. 2008, 34(2), 194-204]. Furthermore, the enzymatic activity of SSAO is involved in the leukocyte extravasation process at sites of inflammation where SSAO has been shown to be strongly expressed on the vascular endothelium [Salmi et al., Immunity 2001, 14(3), 265-276; Salmi & Jalkanen, in "Adhesion Molecules: Functions and Inhibition" K. Ley (Ed.), 2007, pp. 237-251]. Accordingly, inhibition of SSAO has been suggested to have a therapeutic value in the prevention of diabetic complications and in inflammatory diseases [Ekblom, Pharmacol. Res. 1998, 37, 87-92; Salmi et al., Immunity 2001, 14(3), 265-276; Salter-Cid et al., J. Pharmacol. Exp. Ther. 2005, 315(2), 553-562].

SSAO knockout animals are phenotypically overtly normal but exhibit a marked decrease in the inflammatory responses evoked in response to various inflammatory stimuli [Stolen et al., Immunity 2005, 22(1), 105-115]. In addition, antagonism of its function in wild type animals in multiple animal models of human disease (e.g. carrageenan-induced paw inflammation, oxazolone-induced colitis, lipopolysaccharide-induced lung inflammation, collagen-induced arthritis, endotoxin-induced uveitis) by the use of antibodies and/or small molecules has been shown to be protective in decreasing the leukocyte infiltration, reducing the severity of the disease phenotype and reducing levels of inflammatory cytokines and chemokines [Kirton et al., Eur. J. Immunol. 2005, 35(11), 3119-3130; Salter-Cid et al., J. Pharmacol. Exp. Ther. 2005, 315(2), 553-562; McDonald et al., Annual Reports in Medicinal Chemistry 2007, 42, 229-243; Salmi & Jalkanen, in "Adhesion Molecules: Functions and Inhibition" K. Ley (Ed.), 2007, pp. 237-251; Noda et al., FASEB J. 2008 22(4), 1094-1103; Noda et al., FASEB J. 2008, 22(8), 2928-2935]. This anti-inflammatory protection seems to be afforded across a wide range of inflammatory models all with independent causative mechanisms, rather than being restricted to one particular disease or disease model. This would suggest that SSAO may be a key nodal point for the regulation of the inflammatory response, and it seems therefore likely that SSAO inhibitors may be effective anti-inflammatory drugs in a wide range of human diseases.

SSAO inhibitors of widely different structures have previously been disclosed. For example, WO 02/38153 describes tetrahydroimidazo[4,5-c]pyridine derivatives and WO 03/006003 describes 2-indanylhydrazine derivatives. Allylhydrazine and hydroxylamine (aminooxy) compounds are disclosed in WO 2005/014530, and allylamino compounds in WO 2007/120528.

The invention described here relates to novel fused imidazole derivatives as a new class of SSAO inhibitors with biological, pharmacological, and pharmacokinetic characteristics that make them suitable for use as prophylactic or therapeutic agents in a wide range of human inflammatory diseases and immune disorders. This therapeutic capacity is designed to block SSAO enzyme action, reducing the levels of pro-inflammatory enzyme products (aldehydes, hydrogen peroxide and ammonia) whilst also decreasing the adhesive capacity of immune cells and correspondingly their activation and final extra-vasation. Diseases where such an activity is expected to be therapeutically beneficial include all diseases where immune cells play a prominent role in the initiation, maintenance or resolution of the pathology, such as multiple sclerosis, arthritis and vasculitis.

### DISCLOSURE OF THE INVENTION

It has surprisingly been found that the new fused imidazole compounds of formula (I) are inhibitors of SSAO. They are therefore useful for the treatment or prevention of diseases in which inhibition of SSAO activity is beneficial, such as inflammation, inflammatory diseases, immune or autoimmune disorders.

In a first aspect, the invention relates to a compound of formula (I): 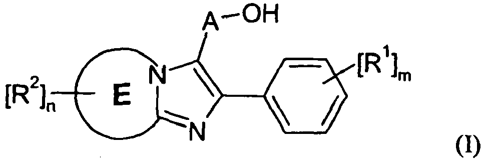 or a pharmaceutically acceptable salt, solvate, hydrate, geometrical isomer, tautomer, optical isomer or *N*-oxide thereof, wherein:
E is a 5- or 6-membered heteroaromatic ring;
A is C₁₋₃-alkylene, which is optionally substituted with C₁₋₃-alkyl;
each R¹ is independently selected from the group consisting of halogen, cyano, nitro, C₁₋₄-alkyl, fluoro-C₁₋₄-alkyl, C₃₋₆-cycloalkyl, heterocyclyl, phenyl, heteroaryl, -C₀₋₄-alkylene-OR³, -C₀₋₄-alkylene-SR³, -C₀₋₄-alkylene-NR^{4A}R^{4B}, -N(R⁵)-C₂₋₄-alkylene-NR^{4A}R^{4B}, -C(O)R³, -C(O)OR³, -C(O)NR^{4A}R^{4B}, -N(R⁵)C(O)R⁶, -N(R⁵)C(O)NR^{4A}R^{4B}, -N(R⁵)C(O)OR⁶, -N(R⁵)S(O)₂R⁶, -S(O)₂NR^{4A}R^{4B} and -S(O)₂R⁶, and wherein any ring residue is optionally substituted with one or more substituents independently selected from halogen, cyano, hydroxy, C₁₋₄-alkyl, C₁₋₄-alkoxy, trifluoromethyl and trifluoromethoxy; or two neighbouring substituents R¹, together with the carbon atoms to which they are attached, form a 5- or 6-membered aromatic or partially unsaturated ring, which optionally contains one or two heteroatoms selected from nitrogen, sulphur or oxygen, and which ring is optionally substituted with one or more substituents independently selected from halogen, cyano, hydroxy, C₁₋₄-alkyl, C₁₋₄-alkoxy, trifluoromethyl and trifluoromethoxy;
each R² is independently selected from the group consisting of halogen, cyano, nitro, C₁₋₄-alkyl, fluoro-C₁₋₄-alkyl, C₃₋₆-cycloalkyl, heterocyclyl, phenyl, heteroaryl, C₃₋₆-cycloalkyl-C₁₋₄-alkyl, C₃₋₆-cycloalkyl(hydroxy)C₁₋₄-alkyl, heterocyclyl-C₁₋₄-alkyl, C₃₋₆-cycloalkylcarbonyl, heterocyclylcarbonyl, benzoyl, heteroarylcarbonyl, -C₀₋₄-alkylene-OR³, -C₀₋₄-alkylene-SR³, -C₀₋₄-alkylene-NR^{7A}R^{7B}, -C₀₋₄-alkylene-C(O)R⁶, -C₀₋₄-alkylene-C(O)OR³, -C₀₋₄-alkylene-C(O)NR^{7A}R^{7B}, -C₀₋₄-alkylene-N(R⁵)C(O)R⁶, -C₀₋₄-alkylene-N(R⁵)C(O)NR^{7A}R^{7B}, -C₀₋₄-alkylene-N(R⁵)C(O)OR⁶, -C₀₋₄-alkylene-N(R⁵)S(O)₂R⁶, -C₀₋₄-alkylene-S(O)₂NR^{7A}R^{7B}, -C₀₋₄-alkylene-S(O)₂R⁶, amidino and guanidino, and wherein any ring residue is optionally substituted with one or more substituents independently selected from halogen, cyano, hydroxy, C₁₋₄-alkyl, C₁₋₄-alkoxy, trifluoromethyl, trifluoromethoxy and -C(O)NR^{8A}R^{8B};
each R³ is independently selected from hydrogen, C₁₋₄-alkyl, fluoro-C₁₋₄-alkyl, hydroxy-C₁₋₄-alkyl and C₃₋₆-cycloalkyl;
each R^{4A} and R^{4B} is independently selected from hydrogen, C₁₋₄-alkyl, hydroxy-C₁₋₄-alkyl and -C₁₋₄-alkylene-NR^{8A}R^{8B};
or independently R^{4A} and R^{4B} together with the nitrogen atom to which they are attached form a 4- to 6-membered saturated heterocyclic ring, which is optionally substituted with one or more substituents independently selected from halogen, hydroxy, C₁₋₄-alkyl, C₁₋₄-alkoxy, trifluoromethyl and trifluoromethoxy;
each R⁵ is independently selected from hydrogen and C₁₋₄-alkyl;
each R⁶ is independently selected from C₁₋₄-alkyl, fluoro-C₁₋₄-alkyl, hydroxy-C₁₋₄-alkyl and C₃₋₆-cycloalkyl;
each R^{7A} and R^{7B} is independently selected from the group consisting of hydrogen, C₁₋₄-alkyl, hydroxy-C₁₋₄-alkyl, C₁₋₄-alkoxy-C₁₋₄-alkyl, C₃₋₆-cycloalkyl, phenyl-C₁₋₄-alkyl, heteroaryl-C₁₋₄-alkyl, C₃₋₆-cycloalkyl-C₁₋₄-alkyl and heterocyclyl-C₁₋₄-alkyl, and wherein any ring residue is optionally substituted with one or more substituents independently selected from halogen, cyano, hydroxy, C₁₋₄-alkyl, C₁₋₄-alkoxy, trifluoromethyl and trifluoromethoxy;
or independently R^{7A} and R^{7B}, together with the nitrogen atom to which they are attached, form a 4- to 6-membered saturated heterocyclic ring, which optionally contains an additional heteroatom selected from nitrogen, sulphur or oxygen, and which ring is optionally substituted with one or more substituents independently selected from halogen, hydroxy, C₁₋₄-alkyl, C₁₋₄-alkoxy, hydroxy-C₁₋₄-alkyl, -S(O)₂NR^{8A}R^{8B} and -C(O)NR^{8A}R^{8B};
each R^{8A} and R^{8B} is independently selected from hydrogen and C₁₋₄-alkyl;
m is 1, 2, 3 or 4; and
n is 0, 1 or 2;
with the provision that the compound is not selected from the group consisting of:
   - 4-[3-(hydroxymethyl)-6-methylimidazo[1,2-a]pyridin-2-yl]-benzonitrile;
   - 2-(4-ethylphenyl)-6-methyl-imidazo[1,2-a]pyridine-3-methanol;
   - 2-(2-nitrophenyl)-imidazo[1,2-a]pyridine-3-methanol;
   - 2-(3,4-dimethylphenyl)-6-methyl-imidazo[1,2-a]pyridine-3-methanol;
   - 6-chloro-2-(4-chlorophenyl)-imidazo[1,2-a]pyridine-3-methanol;
   - 2-(4-fluorophenyl)-imidazo[1,2-a]pyridine-3-methanol;
   - 2-(2-chloro-4-methoxyphenyl)-6-(1-ethylpropyl)-8-methyl-imidazo[1,2-a]pyridine-3-methanol;
   - 2-(4-chlorophenyl)-imidazo[1,2-a]pyridine-3-propanol;
   - 2-(4-methylphenyl)-6-(trifluoromethyl)-imidazo[1,2-a]pyridine-3-methanol;
   - 7-methyl-2-(2-naphthalenyl)-imidazo[1,2-a]pyridine-3-methanol;
   - 2-(4-methylphenyl)-6-nitro-imidazo[1,2-a]pyridine-3-methanol;
   - 2-[1,1'-biphenyl]-4-yl-7-methyl-imidazo[1,2-a]pyridine-3-methanol;
   - 6-methyl-2-[4-(trifluoromethyl)phenyl]-imidazo[1,2-a]pyridine-3-methanol;
   - 2-(4-chlorophenyl)-imidazo[1,2-a]pyridine-3-methanol;
   - 6-methyl-2-(4-methylphenyl)-imidazo[1,2-a]pyridine-3-ethanol;
   - 6-methyl-2-(4-methylphenyl)-imidazo[1,2-a]pyridine-3-methanol;
   - ethyl 4-[3-(hydroxymethyl)-6-methylimidazo[1,2-a]pyridin-2-yl]-benzoate;
   - 2-[4-(methylsulfonyl)phenyl]-imidazo[1,2-a]pyridine-3-methanol;
   - 2-(4-chlorophenyl)-6-methyl-imidazo[1,2-a]pyridine-3-methanol;
   - 6-methyl-2-(4-nitrophenyl)-imidazo[1,2-a]pyridine-3-methanol;
   - 6-chloro-2-(4-methylphenyl)-imidazo[1,2-b]pyridazine-3-methanol;
   - 3-(diethylamino)-2-methyl-6-(4-methylphenyl)-imidazo[1,2-b][1,2,4]triazine-7-methanol;
   - 6-(4-chlorophenyl)-2,3-diphenyl-imidazo[1,2-b][1,2,4]triazine-7-methanol;
   - 6-(4-bromophenyl)-imidazo[2,1-b]thiazole-5-methanol;
   - 6-(4-chlorophenyl)-imidazo[2,1-b]thiazole-5-methanol;
   - 6-(4-chlorophenyl)-2-methyl-imidazo[2,1-b]thiazole-5-methanol;
   - 6-(4-methylphenyl)-imidazo[2,1-b]thiazole-5-methanol;
   - 6-[1,1'-biphenyl]-4-yl-imidazo[2,1-b]thiazole-5-methanol;
   - 6-(4-bromophenyl)-2-(2-furanyl)-imidazo[2,1-b]-1,3,4-thiadiazole-5-methanol;
   - 6-(4-bromophenyl)-2-(2-thienyl)-imidazo[2,1-b]-1,3,4-thiadiazole-5-methanol; and
   - 6-(4-bromophenyl)-2-cyclohexyl-imidazo[2,1-b]-1,3,4-thiadiazole-5-methanol.

In a preferred embodiment of the invention, the heteroaromatic ring E is selected from:

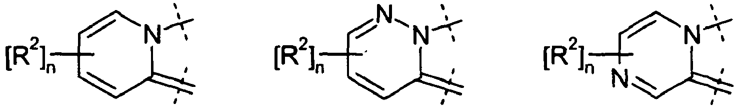

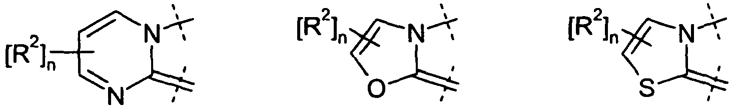

In another preferred embodiment, A is methylene (-CH₂-).

In another preferred embodiment, R¹ is 1, 2 or 3 substituents, more preferably 1 or 2 substituents, independently selected from halogen, C₁₋₄-alkyl, fluoro-C₁₋₄-alkyl and -C₀₋₄-alkylene-OR³, and wherein R³ is selected from hydrogen and C₁₋₄-alkyl.

More preferably, R¹ is independently selected from halogen, C₁₋₄-alkyl, fluoro-C₁₋₄-alkyl and C₁₋₄-alkoxy.

Most preferably, R¹ is independently selected from halogen, methyl, trifluoromethyl and methoxy.

In yet another preferred embodiment, R² is selected from 0, 1 or 2 substituents, more preferably 0 or I substituents, independently selected from halogen, cyano, nitro, C₁₋₄-alkyl, fluoro-C₁₋₄-alkyl, -C₀₋₄-alkylene-OR³, -C₀₋₄-alkylene-C(O)OR³, -C₀₋₄-alkylene-C(O)NR^{7A}R^{7B} and C₃₋₆-cycloalkyl(hydroxy)C₁₋₄-alkyl.

In a more preferred embodiment, R² is independently selected from halogen, cyano, nitro, C₁₋₄-alkyl, fluoro-C₁₋₄-alkyl, hydroxy-C₁₋₄-alkyl and C₁₋₄-alkoxycarbonyl and -C(O)NR^{7A}R^{7B}.

In a most preferred embodiment, R² is independently selected from halogen, cyano, nitro, methyl, ethyl, trifluoromethyl, hydroxymethyl, methoxycarbonyl and -C(O)NR^{7A}R^{7B}.

When R² is -C(O)NR^{7A}R^{7B}, each of R^{7A} and R^{7B} is preferably independently selected from hydrogen, C₁₋₄-alkyl, hydroxy-C₁₋₄-alkyl, C₁₋₄-alkoxy-C₁₋₄-alkyl, C₃₋₆-cycloalkyl, phenyl-C₁₋₄-alkyl, heteroaryl-C₁₋₄-alkyl and heterocyclyl-C₁₋₄-alkyl, and wherein any ring residue is optionally substituted with one or more substituents selected from hydroxy, C₁₋₄-alkyl and C₁₋₄-alkoxy.

In another preferred embodiment, said R^{7A} and R^{7B} form, together with the nitrogen atom to which they are attached, a 4- to 6-membered saturated heterocyclic ring, preferably an azetidine, pyrrolidine, piperidine, piperazine or morpholine ring, and which ring is optionally substituted with one or more substituents independently selected from hydroxy, C₁₋₄-alkyl, C₁₋₄-alkoxy, hydroxy-C₁₋₄-alkyl and -S(O)₂NR^{8A}R^{8B}.

Especially preferred compounds of formula (I) are the compounds selected from the group consisting of:
- [2-(4-Methylphenyl)imidazo[1,2-a]pyridin-3-yl]methanol;
- [2-(2,4-Dichlorophenyl)imidazo[1,2-a]pyridin-3-yl]methanol;
- [2-(4-Bromophenyl)-8-methylimidazo[1,2-a]pyridin-3-yl]methanol;
- [7-Methyl-2-(4-methylphenyl)imidazo[1,2-a]pyridin-3-yl]methanol;
- [2-(4-Bromophenyl)-7-methylimidazo[1,2-a]pyridin-3-yl]methanol;
- [2-(4-Bromophenyl)-7-ethylimidazo[1,2-a]pyridin-3-yl]methanol;
- [2-(2-Chlorophenyl)-7-methylimidazo[1,2-a]pyridin-3-yl]methanol;
- (2-(2,4-Dichlorophenyl)-7-methylimidazo[1,2-a]pyridin-3-yl]methanol;
- [2-(3,4-Dichlorophenyl)-7-methylimidazo[1,2-a]pyridin-3-yl]methanol;
- [6-Methyl-2-(2-naphthyl)imidazo[1,2-a]pyridin-3-yl]methanol;
- [2-(3-Methoxyphenyl)-6-methylimidazo[1,2-a]pyridin-3-yl]methanol;
- 4-[3-(Hydroxymethyl)-6-methylimidazo[1,2-a]pyridin-2-yl]benzonitrile;
- [6-Methyl-2-(3-nitrophenyl)imidazo[1,2-a]pyridin-3-yl]methanol;
- [2-(4-Fluorophenyl)-6-methylimidazo[1,2-a]pyridin-3-yl]methanol;
- [2-(4-Iodophenyl)-6-methylimidazo[1,2-a]pyridin-3-yl]methanol;
- [2-(2-Chlorophenyl)-6-methylimidazo[1,2-a]pyridin-3-yl]methanol;
- (2-{4-[(2-Aminoethyl)amino]phenyl}-6-methylimidazo[1,2-a]pyridin-3-yl)methanol;
- 1-[2-(4-Chlorophenyl)-6-methylimidazo[1,2-a]pyridin-3-yl]ethanol;
- [2-(2,4-Dichlorophenyl)-6-methylimidazo[1,2-a]pyridin-3-yl]methanol;
- [2-(3-Methoxyphenyl)-6-(trifluoromethyl)imidazo[1,2-a]pyridin-3-yl]methanol;
- [2-(4-Chlorophenyl)-6-(trifluoromethyl)imidazo[1,2-a]pyridin-3-yl]methanol;
- [2-(4-Bromophenyl)-6-(trifluoromethyl)imidazo[1,2-a]pyridin-3-yl]methanol;
- [7-Chloro-2-(4-chlorophenyl)imidazo[1,2-a]pyridin-3-yl]methanol;
- [2-(4-Bromophenyl)-7-chloroimidazo[1,2-a]pyridin-3-yl]methanol;
- [7-Chloro-2-(2,4-dichlorophenyl)imidazo[1,2-a]pyridin-3-yl]methanol;
- [7-Chloro-2-(2,4-difluorophenyl)imidazo[1,2-a]pyridin-3-yl]methanol;
- [6-Bromo-2-(3-methoxyphenyl)imidazo[1,2-a]pyridin-3-yl]methanol;
- [6-Bromo-2-(4-fluorophenyl)imidazo[1,2-a]pyridin-3-yl]methanol;
- [6-Bromo-2-(4-bromophenyl)imidazo[1,2-a]pyridin-3-yl]methanol;
- [2-(4-Bromophenyl)-6-chloroimidazo[1,2-a]pyridin-3-yl]methanol;
- [2-(4-Chlorophenyl)-6-fluoroimidazo[1,2-a]pyridin-3-yl]methanol;
- [6-Bromo-2-(2,4-difluorophenyl)imidazo[1,2-a]pyridin-3-yl]methanol;
- [6-Chloro-2-(2,4-difluorophenyl)imidazo[1,2-a]pyridin-3-yl]methanol;
- [6-Bromo-2-(2,4-dichlorophenyl)imidazo[1,2-a]pyridin-3-yl]methanol;
- [6-Chloro-2-(2,4-dichlorophenyl)imidazo[1,2-a]pyridin-3-yl]methanol;
- [6-Bromo-2-(3,4-difluorophenyl)imidazo[1,2-a]pyridin-3-yl]methanol;
- [6-Bromo-2-(3-chloro-4-fluorophenyl)imidazo[1,2-a]pyridin-3-yl]methanol;
- [6-Chloro-2-(3-chloro-4-fluorophenyl)imidazo[1,2-a]pyridin-3-yl]methanol;
- [6,8-Dichloro-2-(3-methoxyphenyl)imidazo[1,2-a]pyridin-3-yl]methanol;
- [2-(4-Bromophenyl)-6,8-dichloroimidazo[1,2-a]pyridin-3-yl]methanol;
- 2-(4-Bromophenyl)-3-(hydroxymethyl)imidazo[1,2-a]pyridine-6-carbonitrile;
- Methyl 2-(4-bromophenyl)-3-(hydroxymethyl)imidazo[1,2-a]pyridine-6-carboxylate;
- Methyl 2-(4-chlorophenyl)-3-(hydroxymethyl)imidazo[1,2-a]pyridine-6-carboxylate;
- [2-(4-Bromophenyl)imidazo[1,2-a]pyridine-3,7-diyl]dimethanol;
- [2-(4-Chlorophenyl)imidazo[1,2-a]pyridine-3,6-diyl]dimethanol;
- [2-(4-Chlorophenyl)-6-nitroimidazo[1,2-a]pyridin-3-yl]methanol;
- [2-(4-Bromophenyl)-6-nitroimidazo[1,2-a]pyridin-3-yl]methanol;
- {2-(4-Chlorophenyl)-6-[(4-methoxypiperidin-1-yl)carbonyl]imidazo[1,2-a]pyridin-3-yl}methanol;
- 2-(4-Chlorophenyl)-3-(hydroxymethyl)-N-(3-methoxypropyl)imidazo[1,2-a]pyridine-6-carboxamide;
- 2-(4-Chlorophenyl)-3-(hydroxymethyl)-N-(2-methoxyethyl)imidazo[1,2-a]pyridine-6-carboxamide;
- [2-(4-Chlorophenyl)-6-(morpholin-4-ylcarbonyl)imidazo[1,2-a]pyridin-3-yl]methanol;
- 2-(4-Chlorophenyl)-3-(hydroxymethyl)-N,N-dimethylimidazo[1,2-a]pyridine-6-carboxamide;
- 2-(4-Chlorophenyl)-3-(hydroxymethyl)-N-methylimidazo[1,2-a]pyridine-6-carboxamide;
- 2-(4-Chlorophenyl)-3-(hydroxymethyl)-N-[2-(1-methylpyrrolidin-2-yl)ethyl]imidazo[1,2-a]pyridine-6-carboxamide;
- {2-(4-Chlorophenyl)-6-[(4-methylpiperazin-1-yl)carbonyl]imidazo[1,2-a]pyridin-3-yl}methanol;
- 2-(4-Chlorophenyl)-N-(3,4-dimethoxybenzyl)-3-(hydroxymethyl)imidazo[1,2-a]pyridine-6-carboxamide;
- 2-(4-Chlorophenyl)-3-(hydroxymethyl)-N-[2-(1H-imidazol-4-yl)ethyl]imidazo[1,2-a]pyridine-6-carboxamide;
- 2-(4-Chlorophenyl)-3-(hydroxymethyl)-N-(pyridin-3-ylmethyl)imidazo[1,2-a]pyridine-6-carboxamide;
- 2-(4-Chlorophenyl)-3-(hydroxymethyl)-N-(3-hydroxypropyl)imidazo[1,2-a]pyridine-6-carboxamide;
- (1- {[2-(4-Chlorophenyl)-3-(hydroxymethyl)imidazo[1,2-a]pyridin-6-yl]carbonyl}piperidin-4-yl)methanol;
- 2-(4-Chlorophenyl)-3-(hydroxymethyl)-N-(2-hydroxypropyl)imidazo[1,2-a]pyridine-6-carboxamide;
- 2-(4-Chlorophenyl)-N-(*trans*-4-hydroxycyclohexyl)-3-(hydroxymethyl)imidazo[ 1,2-a]pyridine-6-carboxamide;
- 1-{[2-(4-Chlorophenyl)-3-(hydroxymethyl)imidazo[1,2-a]pyridin-6-yl]carbonyl}piperidin-4-ol;
- (3R)-1-{[2-(4-Chlorophenyl)-3-(hydroxymethyl)imidazo[1,2-a]pyridin-6-yl]carbonyl}pyrrolidin-3-ol;
- 1-{[2-(4-Chlorophenyl)-3-(hydroxymethyl)imidazo[1,2-a]pyridin-6-yl]carbonyl}pyrrolidin-3-ol;
- 1-{[2-(4-Chlorophenyl)-3-(hydroxymethyl)imidazo[1,2-a]pyridin-6-yl]carbonyl}azetidin-3-ol;
- 2-(4-Chlorophenyl)-3-(hydroxymethyl)imidazo[1,2-a]pyridine-7-carboxamide;
- 3-(Hydroxymethyl)-2-(3-methoxyphenyl)imidazo[1,2-a]pyridine-6-carboxamide;
- 2-(4-Chlorophenyl)-3-(hydroxymethyl)imidazo[1,2-a]pyridine-6-carboxamide;
- 2-(4-Fluorophenyl)-3-(hydroxymethyl)imidazo[1,2-a]pyridine-6-carboxamide;
- 2-(2,4-Difluorophenyl)-3-(hydroxymethyl)imidazo[1,2-a]pyridine-6-carboxamide;
- 2-(2,4-Dichlorophenyl)-3-(hydroxymethyl)imidazo[1,2-a]pyridine-6-carboxamide;
- 2-(3,4-Difluorophenyl)-3-(hydroxymethyl)imidazo[1,2-a]pyridine-6-carboxamide;
- [6-amino-2-(4-chlorophenyl)imidazo[1,2-a]pyridin-3-yl]methanol;
- N-[2-(4-chlorophenyl)-3-(hydroxymethyl)imidazo[1,2-a]pyridin-6-yl]acetamide;
- [6-amino-2-(4-bromophenyl)imidazo[1,2-a]pyridin-3-yl]methanol;
- [6-chloro-2-(4-chlorophenyl)imidazo[1,2-b]pyridazin-3-yl]methanol;
- [2-(4-Chlorophenyl)imidazo[1,2-a]pyrazin-3-yl]methanol;
- [6-Bromo-2-(3-methoxyphenyl)imidazo[1,2-a]pyrazin-3-yl]methanol;
- {6-Bromo-2-[4-(trifluoromethyl)phenyl]imidazo[1,2-a]pyrazin-3-yl}methanol;
- [6-Bromo-2-(4-fluorophenyl)imidazo[1,2-a]pyrazin-3-yl]methanol;
- [6-Bromo-2-(4-chlorophenyl)imidazo[1,2-a]pyrazin-3-yl]methanol;
- [6-Bromo-2-(4-bromophenyl)imidazo[1,2-a]pyrazin-3-yl]methanol;
- [6-Bromo-2-(2,4-dichlorophenyl)imidazo[1,2-a]pyrazin-3-yl]methanol;
- [6-Bromo-2-(2,4-difluorophenyl)imidazo[1,2-a]pyrazin-3-yl]methanol;
- [6-Bromo-2-(4-chloro-2-fluoro-5-methylphenyl)imidazo[1,2-a]pyrazin-3-yl]methanol;
- [2-(1-Benzofuran-5-yl)-6-bromoimidazo[1,2-a]pyrazin-3-yl]methanol;
- [6-Bromo-2-(2,3-dihydro-1,4-benzodioxin-5-yl)imidazo[1,2-a]pyrazin-3-yl]methanol;
- [6-amino-2-(4-fluorophenyl)imidazo[1,2-a]pyrazin-3-yl]methanol;
- [6-amino-2-(4-chlorophenyl)imidazo[1,2-a]pyrazin-3-yl]methanol;
- [6-Amino-2-(4-bromophenyl)imidazo[1,2-a]pyrazin-3-yl]methanol;
- [6-(Azetidin-1-yl)-2-(4-fluorophenyl)imidazo[1,2-a]pyrazin-3-yl]methanol;
- [2-(4-Chlorophenyl)imidazo[1,2-a]pyrimidin-3-yl]methanol;
- [2-(2,4-Dichlorophenyl)imidazo[1,2-a]pyrimidin-3-yl]methanol;
- [6-(4-fluorophenyl)-2-methylimidazo[2,1-b][1,3]oxazol-5-yl]methanol;
- [6-(2,4-dichlorophenyl)imidazo[2,1-b][1,3]thiazol-5-yl]methanol;
- [6-(4-Bromophenyl)-2-methylimidazo[2,1-b][1,3]thiazol-5-yl]methanol;
- [6-(2,4-Dichlorophenyl)-2-methylimidazo[2,1-b][1,3]thiazol-5-yl]methanol;
- [2-Chloro-6-(4-chlorophenyl)imidazo[2,1-b][1,3]thiazol-5-yl]methanol;
- Methyl 6-(4-chlorophenyl)-5-(hydroxymethyl)imidazo[2,1-b][1,3]thiazole-2-carboxylate;
- [6-(4-Chlorophenyl)imidazo[2,1-b][1,3]thiazole-2,5-diyl]dimethanol;
- 1-[6-(4-Chlorophenyl)-5-(hydroxymethyl)imidazo[2,1-b][1,3]thiazol-2-yl]ethanol;
- [6-(4-Chlorophenyl)-5-(hydroxymethyl)imidazo[2,1-b][1,3]thiazol-2-yl](cyclopropyl)methanol;
- 2-[6-(4-Chlorophenyl)-5-(hydroxymethyl)imidazo[2,1-b][1,3]thiazol-2-yl]propan-2-ol;
- 6-(4-Chlorophenyl)-N-ethyl-5-(hydroxymethyl)-N-methylimidazo[2,1-b][1,3]thiazole-2-carboxamide;
- [6-(4-Chlorophenyl)-2-(morpholin-4-ylcarbonyl)imidazo[2,1-b][1,3]thiazol-5-yl]methanol;
- {6-(4-Chlorophenyl)-2-[(4-methylpiperazin-1-yl)carbonyl]imidazo[2,1-b][1,3]thiazol-5-yl}methanol; and
- 6-(4-Chlorophenyl)-5-(hydroxymethyl)-N-propylimidazo[2,1-b][1,3]thiazole-2-carboxamide.

In another aspect, the invention relates to a compound of formula (I) for use in therapy. The compounds as defined above are useful as inhibitors of SSAO activity. As such, they are useful in the treatment or prevention of conditions and diseases in which inhibition of SSAO activity is beneficial. More specifically, they are useful for the treatment or prevention of inflammation, inflammatory diseases, immune or autoimmune disorders.

In particular, it is believed that compounds of formula (I) are useful for the treatment or prevention of arthritis (such as rheumatoid arthritis, juvenile rheumatoid arthritis, osteoarthritis and psoriatic arthritis), synovitis, vasculitis, conditions associated with inflammation of the bowel (such as Crohn's disease, ulcerative colitis, inflammatory bowel disease and irritable bowel syndrome), atherosclerosis, multiple sclerosis, Alzheimer's disease, vascular dementia, pulmonary inflammatory diseases (such as asthma, chronic obstructive pulmonary disease and acute respiratory distress syndrome), fibrotic diseases (including idiopathic pulmonary fibrosis, cardiac fibrosis and systemic sclerosis (scleroderma)), inflammatory diseases of the skin (such as contact dermatitis, atopic dermatitis and psoriasis), systemic inflammatory response syndrome, sepsis, inflammatory and/or autoimmune conditions of the liver (such as autoimmune hepatitis, primary biliary cirrhosis, alcoholic liver disease, sclerosing cholangitis, and autoimmune cholangitis), diabetes (type I or II) and/or the complications thereof, chronic heart failure, congestive heart failure, ischemic diseases (such as stroke and ischemia-reperfusion injury), and myocardial infarction and/or the complications thereof.

It is believed that the compounds of the invention are especially useful for the treatment or prevention of vasculitis, including, but not limited to, giant cell arteritis, Takayasu's arteritis, Polyarteritis nodosa, Kawasaki disease, Wegener's granulomatosis, Churg-Strauss syndrome, microscopic polyangiitis, Henoch-Schönlein purpura, cryoglobulinemia, cutaneous leukocytoclastic angiitis and primary angiitis of the central nervous system.

The invention thus includes a compound of formula (I) 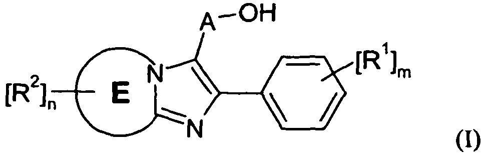 or a pharmaceutically acceptable salt, solvate, hydrate, geometrical isomer, tautomer, optical isomer or *N*-oxide thereof, wherein:
E is a 5- or 6-membered heteroaromatic ring;
A is C₁₋₃-alkylene, which is optionally substituted with C₁₋₃-alkyl;
each R¹ is independently selected from the group consisting of halogen, cyano, nitro, C₁₋₄-alkyl, fluoro-C₁₋₄-alkyl, C₃₋₆-cycloalkyl, heterocyclyl, phenyl, heteroaryl, -C₀₋₄-alkylene-OR³, -C₀₋₄-alkylene-SR³, -C₀₋₄-alkylene-NR^{4A}R^{4B}, -N(R⁵)-C₂₋₄-alkylene-NR^{4A}R^{4B}, -C(O)R³, -C(O)OR³, -C(O)NR^{4A}R^{4B}, -N(R⁵)C(O)R⁶, -N(R⁵)C(O)NR^{4A}R^{4B}, -N(R⁵)C(O)OR⁶, -N(R⁵)S(O)₂R⁶, -S(O)₂NR^{4A}R^{4B} and -S(O)₂R⁶, and wherein any ring residue is optionally substituted with one or more substituents independently selected from halogen, cyano, hydroxy, C₁₋₄-alkyl, C₁₋₄-alkoxy, trifluoromethyl and trifluoromethoxy;
or two neighbouring substituents R', together with the carbon atoms to which they are attached, form a 5- or 6-membered aromatic or partially unsaturated ring, which optionally contains one or two heteroatoms selected from nitrogen, sulphur or oxygen, and which ring is optionally substituted with one or more substituents independently selected from halogen, cyano, hydroxy, C₁₋₄-alkyl, C₁₋₄-alkoxy, trifluoromethyl and trifluoromethoxy;
each R² is independently selected from the group consisting of halogen, cyano, nitro, C₁₋₄-alkyl, fluoro-C₁₋₄-alkyl, C₃₋₆-cycloalkyl, heterocyclyl, phenyl, heteroaryl, C₃₋₆-cycloalkyl-C₁₋₄-alkyl, C₃₋₆-cycloalkyl(hydroxy)C₁₋₄-alkyl, heterocyclyl-C₁₋₄-alkyl, C₃₋₆-cycloalkylcarbonyl, heterocyclylcarbonyl, benzoyl, heteroarylcarbonyl, -C₀₋₄-alkylene-OR³, -C₀₋₄-alkylene-SR³, -C₀₋₄-alkylene-NR^{7A}R^{7B}, -C₀₋₄-alkylene-C(O)R⁶, -C₀₋₄-alkylene-C(O)OR³, -C₀₋₄-alkylene-C(O)NR^{7A}R^{7B}, -C₀₋₄-alkylene-N(R⁵)C(O)R⁶, -C₀₋₄-alkylene-N(R⁵)C(O)NR^{7A}R^{7B}, -C₀₋₄-alkylene-N(R⁵)C(O)OR⁶, -C₀₋₄-alkylene-N(R⁵)S(O)₂R⁶, -C₀₋₄-alkylene-S(O)₂NR^{7A}R^{7B}, -C₀₋₄-alkylene-S(O)₂R⁶, amidino and guanidino, and wherein any ring residue is optionally substituted with one or more substituents independently selected from halogen, cyano, hydroxy, C₁₋₄-alkyl, C₁₋₄-alkoxy, trifluoromethyl, trifluoromethoxy and -C(O)NR^{8A}R^{8B};
each R³ is independently selected from hydrogen, C₁₋₄-alkyl, fluoro-C₁₋₄-alkyl, hydroxy-C₁₋₄-alkyl and C₃₋₆-cycloalkyl;
each R^{4A} and R^{4B} is independently selected from hydrogen, C₁₋₄-alkyl, hydroxy-C₁₋₄-alkyl and -C₁₋₄-alkylene-NR^{8A}R^{8B};
or independently R^{4A} and R^{4B} together with the nitrogen atom to which they are attached form a 4- to 6-membered saturated heterocyclic ring, which is optionally substituted with one or more substituents independently selected from halogen, hydroxy, C₁₋₄-alkyl, C₁₋₄-alkoxy, trifluoromethyl and trifluoromethoxy;
each R⁵ is independently selected from hydrogen and C₁₋₄-alkyl;
each R⁶ is independently selected from C₁₋₄-alkyl, fluoro-C₁₋₄-alkyl, hydroxy-C₁₋₄-alkyl and C₃₋₆-cycloalkyl;
each R^{7A} and R^{7B} is independently selected from the group consisting of hydrogen, C₁₋₄-alkyl, hydroxy-C₁₋₄-alkyl, C₁₋₄-alkoxy-C₁₋₄-alkyl, C₃₋₆-cycloalkyl, phenyl-C₁₋₄-alkyl, heteroaryl-C₁₋₄-alkyl, C₃₋₆-cycloalkyl-C₁₋₄-alkyl and heterocyclyl-C₁₋₄-alkyl, and wherein any ring residue is optionally substituted with one or more substituents independently selected from halogen, cyano, hydroxy, C₁₋₄-alkyl, C₁₋₄-alkoxy, trifluoromethyl and trifluoromethoxy;
or independently R^{7A} and R^{7B}, together with the nitrogen atom to which they are attached, form a 4- to 6-membered saturated heterocyclic ring, which optionally contains an additional heteroatom selected from nitrogen, sulphur or oxygen, and which ring is optionally substituted with one or more substituents independently selected from halogen, hydroxy, C₁₋₄-alkyl, C₁₋₄-alkoxy, hydroxy-C₁₋₄-alkyl, -S(O)₂NR^{8A}R^{8B} and -C(O)NR^{8A}R^{8B};
each R^{8A} and R^{8B} is independently selected from hydrogen and C₁₋₄-alkyl;
m is 1, 2, 3 or 4; and
n is 0, 1 or 2,
for use in the treatment or prevention of the above-mentioned conditions and diseases.

The invention also includes the use of said compounds in the manufacture of a medicament for the treatment or prevention of the above-mentioned conditions and diseases. The invention furthermore includes methods for treatment or prevention of such conditions and diseases, comprising administering to a mammal, including man, in need of such treatment an effective amount of a compound as defined above.

Methods delineated herein include those wherein the subject is identified as in need of a particular stated treatment. Identifying a subject in need of such treatment can be in the judgment of a subject or a health care professional and can be subjective (e.g. opinion) or objective (e.g. measurable by a test or diagnostic method).

In other aspects, the methods herein include those further comprising monitoring subject response to the treatment administrations. Such monitoring may include periodic sampling of subject tissue, fluids, specimens, cells, proteins, chemical markers, genetic materials, etc. as markers or indicators of the treatment regimen. In other methods, the subject is prescreened or identified as in need of such treatment by assessment for a relevant marker or indicator of suitability for such treatment.

In one embodiment, the invention provides a method of monitoring treatment progress. The method includes the step of determining a level of diagnostic marker (Marker) (e.g., any target or cell type delineated herein modulated by a compound herein) or diagnostic measurement (e.g., screen, assay) in a subject suffering from or susceptible to a disorder or symptoms thereof delineated herein, in which the subject has been administered a therapeutic amount of a compound herein sufficient to treat the disease or symptoms thereof. The level of Marker determined in the method can be compared to known levels of Marker in either healthy normal controls or in other afflicted patients to establish the subject's disease status. In preferred embodiments, a second level of Marker in the subject is determined at a time point later than the determination of the first level, and the two levels are compared to monitor the course of disease or the efficacy of the therapy. In certain preferred embodiments, a pre-treatment level of Marker in the subject is determined prior to beginning treatment according to this invention; this pre-treatment level of Marker can then be compared to the level of Marker in the subject after the treatment commences, to determine the efficacy of the treatment.

In certain method embodiments, a level of Marker or Marker activity in a subject is determined at least once. Comparison of Marker levels, e.g., to another measurement of Marker level obtained previously or subsequently from the same patient, another patient, or a normal subject, may be useful in determining whether therapy according to the invention is having the desired effect, and thereby permitting adjustment of dosage levels as appropriate. Determination of Marker levels may be performed using any suitable sampling/expression assay method known in the art or described herein. Preferably, a tissue or fluid sample is first removed from a subject. Examples of suitable samples include blood, urine, tissue, mouth or cheek cells, and hair samples containing roots. Other suitable samples would be known to the person skilled in the art. Determination of protein levels and/or mRNA levels (e.g., Marker levels) in the sample can be performed using any suitable technique known in the art, including, but not limited to, enzyme immunoassay, ELISA, radiolabeling/assay techniques, blotting/chemiluminescence methods, real-time PCR, and the like.

### DEFINITIONS

The following definitions shall apply throughout the specification and the appended claims, unless otherwise stated or indicated.

The term "C₁₋₄-alkyl" denotes a straight or branched alkyl group having from 1 to 4 carbon atoms. For parts of the range C₁₋₄-alkyl all subgroups thereof are contemplated such as C₁₋₃-alkyl, C₁₋₂-alkyl, C₂₋₄-alkyl, C₂₋₃-alkyl and C₃₋₄-alkyl. Examples of said C₁₋₄-alkyl include methyl, ethyl, *n*-propyl, isopropyl, *n*-butyl, isobutyl, *sec*-butyl and *tert*-butyl.

The term "fluoro-C₁₋₄-alkyl" denotes a straight or branched C₁₋₄-alkyl group substituted by one or more fluorine atoms. Examples of said fluoro-C₁₋₄-alkyl include fluoromethyl, trifluoromethyl, 2-fluoroethyl and 2,2,2-trifluoroethyl.

The term "hydroxy-C₁₋₄-alkyl" denotes a straight or branched C₁₋₄-alkyl group that has one or more hydrogen atoms thereof replaced with OH. Examples of said hydroxy-C₁₋₄-alkyl include hydroxymethyl, 2-hydroxy-2-methylpropyl and 2,3-dihydroxypropyl.

The term "C₁₋₄-alkoxy" refers to a straight or branched C₁₋₄-alkyl group which is attached to the remainder of the molecule through an oxygen atom. For parts of the range C₁₋₄-alkoxy, all subgroups thereof are contemplated such as C₁₋₃-alkoxy, C₁₋₂-alkoxy, C₂₋₄-alkoxy, C₂₋₃-alkoxy and C₃₋₄-alkoxy. Examples of said C₁₋₄-alkoxy include methoxy, ethoxy, *n*-propoxy, isopropoxy, *n*-butoxy, isobutoxy, sec-butoxy and *tert*-butoxy.

The term "C₁₋₄-alkoxy-C₁₋₄-alkyl" denotes a straight or branched alkoxy group having from 1 to 4 carbon atoms connected to a straight or branched alkyl group having from from 1 to 4 carbon atoms. Examples of said C₁₋₄-alkoxy-C₁₋₄-alkyl include methoxymethyl, methoxyethyl, ethoxyethyl, isopropoxyethyl, *n*-butoxyethyl and t-butoxyethyl.

The term "C₀₋₄-alkylene" denotes a bond (i.e. C₀-alkylene) or a straight or branched divalent saturated hydrocarbon chain (i.e. C₁₋₄-alkylene) having from 1 to 4 carbon atoms. The C₁₋₄-alkylene chain may be attached to the rest of the molecule and to the radical group through one carbon within the chain or through any two carbons within the chain. Examples of C₁₋₄-alkylene radicals include methylene [-CH₂-], 1,2-ethylene [-CH₂-CH₂-], 1,1-ethylene [-CH(CH₃)-], 1,2-propylene [-CH₂-CH(CH₃)-] and 1,3-propylene [-CH₂-CH₂-CH₂-]. When referring to a "C₀₋₄-alkylene" radical, all subgroups thereof are contemplated, such as C₀₋₃-alkylene, C₀₋₂-alkylene, C₀₋₁-alkylene, C₁₋₄-alkylene, C₁₋₃-alkylene, C₁₋₂-alkylene, C₂₋₄-alkylene, C₂₋₃-alkylene and C₃₋₄-alkylene.

The term "C₃₋₆-cycloalkyl" denotes a saturated monocyclic hydrocarbon ring having from 3 to 6 carbon atoms. Examples of said C₃₋₆-cycloalkyl include cyclopropyl, cyclobutyl, cyclopentyl and cyclohexyl. For parts of the range "C₃₋₆-cycloalkyl" all subgroups thereof are contemplated, such as C₃₋₅-cycloalkyl, C₃₋₄-cycloalkyl, C₄₋₆-cycloalkyl, C₄₋₅-cycloalkyl and C₅₋₆-cycloalkyl.

The terms "heterocyclyl" and "heterocyclic ring" denote a saturated or partially unsaturated monocyclic ring having from 4 to 6 ring atoms with at least one heteroatom such as nitrogen, sulphur or oxygen, and the remaining ring atoms are carbon. Examples of said heterocyclic rings include piperidinyl, tetrahydropyranyl, tetrahydrofuranyl, oxetanyl, azetidinyl, pyrrolidinyl, morpholinyl, imidazolinyl, imidazolidinyl, thiomorpholinyl, pyranyl, dioxanyl, piperazinyl, and 5,6-dihydro-4H-1,3-oxazin-2-yl. When present, the sulfur atom may be in an oxidized form (i.e., S=O or O=S=O). Exemplary heterocyclic groups containing sulfur in oxidized form are 1,1-dioxido-thiomorpholinyl and 1,1-dioxido-isothiazolidinyl.

The terms "heteroaryl" and "heteroaromatic ring" denote a monocyclic heteroaromatic ring comprising 5 to 6 ring atoms in which one or more of the ring atoms are other than carbon, such as nitrogen, sulphur or oxygen. The heteroaryl moiety can be linked to the remainder of the molecule via a carbon or nitrogen atom. Examples of heteroaryl groups include furyl, pyrrolyl, thienyl, oxazolyl, isoxazolyl, imidazolyl, thiazolyl, isothiazolyl, pyridinyl, pyrimidinyl, tetrazolyl, pyrazolyl, pyridazinyl, pyrazinyl and thiadiazolyl.

The term "phenyl-C₁₋₄-alkyl" refers to a phenyl group that is directly linked to a straight or branched C₁₋₄-alkyl group. Examples of said phenyl-C₁₋₄-alkyl include phenylmethyl (i.e. benzyl), 1-phenylethyl and 2-phenylpropyl.

The term "heteroaryl-C₁₋₄-alkyl" refers to a heteroaryl ring that is directly linked to a straight or branched C₁₋₄-alkyl group via a carbon or nitrogen atom of said ring. Examples of such groups include pyridinylmethyl, pyrazinylmethyl, thiazolylmethyl and isoxazolylmethyl.

The term "C₃₋₆-cycloalkyl-C₁₋₄-alkyl" denotes a C₃₋₆-cycloalkyl group that is directly attached to a straight or branched C₁₋₄-alkyl group. Examples of C₃₋₆-cycloalkyl-C₁₋₄-alkyl groups include cyclopentylmethyl and cyclohexylethyl.

The term "C₃₋₆-cycloalkyl(hydroxy)C₁₋₄-alkyl" denotes a C₃₋₆-cycloalkyl-C₁₋₄-alkyl group as defined above wherein the C₁₋₄-alkyl part is additionally substituted with a hydroxy group. Examples of such groups include cyclopropyl(hydroxy)methyl and 2-cyclopentyl-1-hydroxyethyl.

The term "heterocyclyl-C₁₋₄-alkyl" denotes a heterocyclic ring as defined above that is directly attached to a straight or branched C₁₋₄-alkyl group via a carbon or nitrogen atom of said ring. Examples of heterocyclyl-C₁₋₄-alkyl groups include oxetanylmethyl, tetrahydrofuranylmethyl and pyrrolidinylmethyl.

The term "heteroarylcarbonyl" refers to a heteroaryl ring that is directly linked to a carbonyl group via a carbon or nitrogen atom of said ring. Examples of such groups include pyridinylcarbonyl, pyridazinylcarbonyl and thienylcarbonyl.

The term "C₃₋₆-cycloalkylcarbonyl" refers to a C₃₋₆-cycloalkyl group that is directly attached to a carbonyl group. Examples of C₃₋₆-cycloalkylcarbonyl groups include cyclobutylcarbonyl and cyclopentylcarbonyl.

The term "heterocyclylcarbonyl" refers to a heterocyclic ring as defined above that is attached to a carbonyl group via a carbon or nitrogen atom of said ring. Exemplary heterocyclylcarbonyl groups include morpholin-4-ylcarbonyl and piperazin-1-ylcarbonyl. "Halogen" refers to fluorine, chlorine, bromine or iodine.
"Hydroxy" refers to the -OH radical.
"Nitro" refers to the -NO₂ radical.
"Cyano" refers to the -CN radical.
"Amidino" refers to the -C(=NH)NH₂ radical.
"Guanidino" refers to the -N(H)C(=NH)NH₂ radical.
"Optional" or "optionally" means that the subsequently described event or circumstance may but need not occur, and that the description includes instances where the event or circumstance occurs and instances in which it does not.
"Pharmaceutically acceptable" means being useful in preparing a pharmaceutical composition that is generally safe, non-toxic and neither biologically nor otherwise undesirable and includes being useful for veterinary use as well as human pharmaceutical use.
"Treatment" as used herein includes prophylaxis of the named disorder or condition, or amelioration or elimination of the disorder once it has been established.
"An effective amount" refers to an amount of a compound that confers a therapeutic effect on the treated subject. The therapeutic effect may be objective (i.e., measurable by some test or marker) or subjective (i.e., subject gives an indication of or feels an effect).
"Prodrugs" refers to compounds that may be converted under physiological conditions or by solvolysis to a biologically active compound of the invention. A prodrug may be inactive when administered to a subject in need thereof, but is converted *in vivo* to an active compound of the invention. Prodrugs are typically rapidly transformed *in vivo* to yield the parent compound of the invention, e.g. by hydrolysis in the blood. The prodrug compound usually offers advantages of solubility, tissue compatibility or delayed release in a mammalian organism (see Silverman, R. B., The Organic Chemistry of Drug Design and Drug Action, 2nd Ed., Elsevier Academic Press (2004), pp. 498-549). Prodrugs of a compound of the invention may be prepared by modifying functional groups, such as a hydroxy, amino or mercapto groups, present in a compound of the invention in such a way that the modifications are cleaved, either in routine manipulation or *in vivo,* to the parent compound of the invention. Examples of prodrugs include, but are not limited to, acetate, formate and succinate derivatives of hydroxy functional groups or phenyl carbamate derivatives of amino functional groups.

Throughout the specification and the appended claims, a given chemical formula or name shall also encompass all salts, hydrates, solvates, N-oxides and prodrug forms thereof. Further, a given chemical formula or name shall encompass all tautomeric and stereoisomeric forms thereof. Stereoisomers include enantiomers and diastereomers. Enantiomers can be present in their pure forms, or as racemic (equal) or unequal mixtures of two enantiomers. Diastereomers can be present in their pure forms, or as mixtures of diastereomers. Diastereomers also include geometrical isomers, which can be present in their pure *cis* or *trans* forms or as mixtures of those.

The compounds of formula (I) may be used as such or, where appropriate, as pharmacologically acceptable salts (acid or base addition salts) thereof. The pharmacologically acceptable addition salts mentioned below are meant to comprise the therapeutically active non-toxic acid and base addition salt forms that the compounds are able to form. Compounds that have basic properties can be converted to their pharmaceutically acceptable acid addition salts by treating the base form with an appropriate acid. Exemplary acids include inorganic acids, such as hydrogen chloride, hydrogen bromide, hydrogen iodide, sulphuric acid, phosphoric acid; and organic acids such as formic acid, acetic acid, propanoic acid, hydroxyacetic acid, lactic acid, pyruvic acid, glycolic acid, maleic acid, malonic acid, oxalic acid, benzenesulphonic acid, toluenesulphonic acid, methanesulphonic acid, trifluoroacetic acid, fumaric acid, succinic acid, malic acid, tartaric acid, citric acid, salicylic acid, p-aminosalicylic acid, pamoic acid, benzoic acid, ascorbic acid and the like. Exemplary base addition salt forms are the sodium, potassium, calcium salts, and salts with pharmaceutically acceptable amines such as, for example, ammonia, alkylamines, benzathine, and amino acids, such as, e.g. arginine and lysine. The term addition salt as used herein also comprises solvates which the compounds and salts thereof are able to form, such as, for example, hydrates, alcoholates and the like.

### COMPOSITIONS

For clinical use, the compounds of the invention are formulated into pharmaceutical formulations for various modes of administration. It will be appreciated that compounds of the invention may be administered together with a physiologically acceptable carrier, excipient, or diluent. The pharmaceutical compositions of the invention may be administered by any suitable route, preferably by oral, rectal, nasal, topical (including buccal and sublingual), sublingual, transdermal, intrathecal, transmucosal or parenteral (including subcutaneous, intramuscular, intravenous and intradermal) administration. Other formulations may conveniently be presented in unit dosage form, e.g., tablets and sustained release capsules, and in liposomes, and may be prepared by any method well known in the art of pharmacy. Pharmaceutical formulations are usually prepared by mixing the active substance, or a pharmaceutically acceptable salt thereof, with conventional pharmaceutically acceptable carriers, diluents or excipients. Examples of excipients are water, gelatin, gum arabicum, lactose, microcrystalline cellulose, starch, sodium starch glycolate, calcium hydrogen phosphate, magnesium stearate, talcum, colloidal silicon dioxide, and the like. Such formulations may also contain other pharmacologically active agents, and conventional additives, such as stabilizers, wetting agents, emulsifiers, flavouring agents, buffers, and the like. Usually, the amount of active compounds is between 0.1-95% by weight of the preparation, preferably between 0.2-20% by weight in preparations for parenteral use and more preferably between 1-50% by weight in preparations for oral administration.

The formulations can be further prepared by known methods such as granulation, compression, microencapsulation, spray coating, etc. The formulations may be prepared by conventional methods in the dosage form of tablets, capsules, granules, powders, syrups, suspensions, suppositories or injections. Liquid formulations may be prepared by dissolving or suspending the active substance in water or other suitable vehicles. Tablets and granules may be coated in a conventional manner. To maintain therapeutically effective plasma concentrations for extended periods of time, compounds of the invention may be incorporated into slow release formulations.

The dose level and frequency of dosage of the specific compound will vary depending on a variety of factors including the potency of the specific compound employed, the metabolic stability and length of action of that compound, the patient's age, body weight, general health, sex, diet, mode and time of administration, rate of excretion, drug combination, the severity of the condition to be treated, and the patient undergoing therapy. The daily dosage may, for example, range from about 0.001 mg to about 100 mg per kilo of body weight, administered singly or multiply in doses, e.g. from about 0.01 mg to about 25 mg each. Normally, such a dosage is given orally but parenteral administration may also be chosen.

### PREPARATION OF COMPOUNDS OF THE INVENTION

The compounds of formula (I) above may be prepared by, or in analogy with, conventional methods. The preparation of intermediates and compounds according to the examples of the present invention may in particular be illuminated by the following Scheme 1. Definitions of variables in the structures in schemes herein are commensurate with those of corresponding positions in the formulae delineated herein. 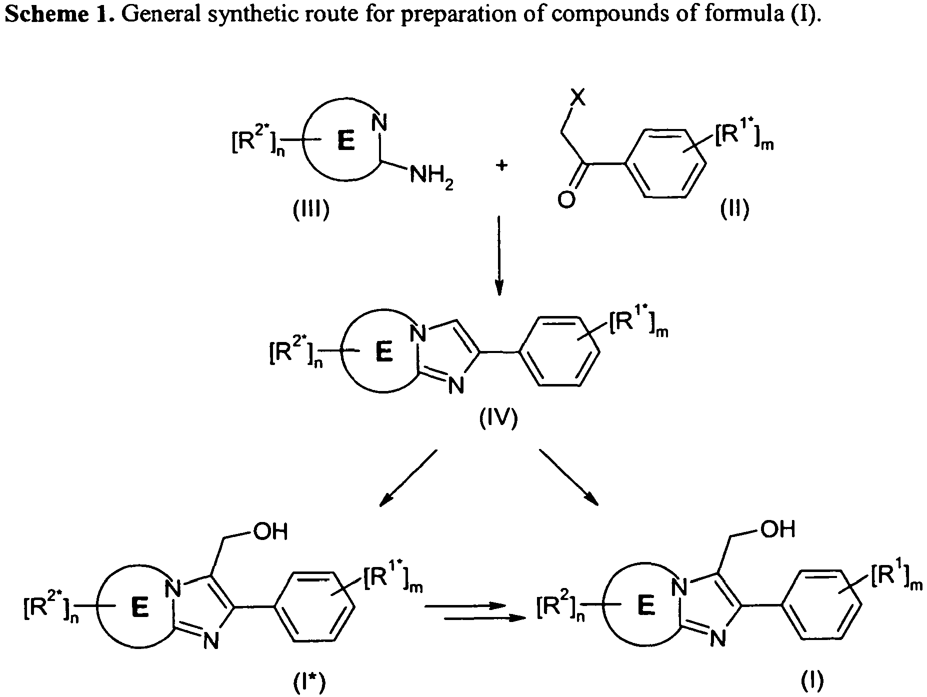 wherein E, m and n are as defined in formula (I);
X is halogen (e.g. bromo); and
R^{1*} and R^{2*} correspond to R' and R², respectively, as defined in formula (I), or to a group that in one or more steps can be transformed into a group R' or R².

Compounds of formula (I), wherein A is methylene, can easily be obtained in only a few simple steps. The key step in the preparation is the formation of the fused imidazole scaffold. Thus, condensation of an appropriately substituted 2-halo-1-phenylethanone derivative of formula (II) with an appropriately substituted heteroaryl-amine derivative of formula (III) results in the intermediate compound of formula (IV). Subsequent treatment of this intermediate with formaldehyde affords a compound of formula (I) or (I*), depending on the nature of R^{1*} and R^{2*}. One or more additional operations may be necessary to transform a compound of formula (I*) into the desired compound of formula (I).

Optionally, a compound of formula (I) can also be transformed into another compound of formula (I) in one or more synthetic steps. For example, if R² denotes a group -NH₂, the corresponding amide derivative can simply be obtained by acylation of the primary amino group.

Appropriate reaction conditions for the individual reaction steps are known to a person skilled in the art. Particular reaction conditions for examples of the invention are also described in the experimental section.

The necessary starting materials for preparing the compounds of formula (I) are either commercially available, or may be prepared by methods known in the art.

The processes described below in the experimental section may be carried out to give a compound of the invention in the form of a free base or as an acid addition salt. A pharmaceutically acceptable acid addition salt may be obtained by dissolving the free base in a suitable organic solvent and treating the solution with an acid, in accordance with conventional procedures for preparing acid addition salts from base compounds. Examples of addition salt forming acids are mentioned above.

The compounds of formula (I) may possess one or more chiral carbon atoms, and they may therefore be obtained in the form of optical isomers, e.g., as a pure enantiomer, or as a mixture of enantiomers (racemate) or as a mixture containing diastereomers. The separation of mixtures of optical isomers to obtain pure enantiomers is well known in the art and may, for example, be achieved by fractional crystallization of salts with optically active (chiral) acids or by chromatographic separation on chiral columns.

The chemicals used in the synthetic routes delineated herein may include, for example, solvents, reagents, catalysts, and protecting group and deprotecting group reagents. Examples of protecting groups are t-butoxycarbonyl (Boc), benzyl and trityl (triphenylmethyl). The methods described above may also additionally include steps, either before or after the steps described specifically herein, to add or remove suitable protecting groups in order to ultimately allow synthesis of the compounds. In addition, various synthetic steps may be performed in an alternate sequence or order to give the desired compounds. Synthetic chemistry transformations and protecting group methodologies (protection and deprotection) useful in synthesizing applicable compounds are known in the art and include, for example, those described in R. Larock, Comprehensive Organic Transformations, VCH Publishers (1989); T.W. Greene and P.G.M. Wuts, Protective Groups in Organic Synthesis, 3rd Ed., John Wiley and Sons (1999); L. Fieser and M. Fieser, Fieser and Fieser's Reagents for Organic Synthesis, John Wiley and Sons (1994); and L. Paquette, ed., Encyclopedia of Reagents for Organic Synthesis, John Wiley and Sons (1995) and subsequent editions thereof.

The following abbreviations have been used:
- aq.: aqueous
- conc.: concentrated
- CH₂Cl₂: dichloromethane
- DMF: *N,N-*dimethylformamide
- DMSO: dimethylsulfoxide
- ESI: electrospray ionisation
- h: hour(s)
- HBTU: *0*-(1H-Benzotriazol-1-yl)-*N,N,N',N'*-tetramethyluronium hexafluorophosphate
- HPLC: High Performance Liquid Chromatography
- HRMS: High Resolution Mass Spectrometry (Accurate MS)
- min: minute(s)
- MS: Mass Spectrometry
- NH₄OAc: ammonium acetate
- rt: room temperature
- sat.: saturated
- TBTU: *0*-(Benzotriazol-1-yl)-*N,N,N',N'*-tetramethyluronium tetrafluoroborate
- TFA: trifluoroacetic acid
- THF: tetrahydrofuran
- UV: ultraviolet

The recitation of a listing of chemical groups in any definition of a variable herein includes definitions of that variable as any single group or combination of listed groups. The recitation of an embodiment herein includes that embodiment as any single embodiment or in combination with any other embodiments or portions thereof.

The invention will now be further illustrated by the following non-limiting examples. The specific examples below are to be construed as merely illustrative, and not limitative of the remainder of the disclosure in any way whatsoever. Without further elaboration, it is believed that one skilled in the art can, based on the description herein, utilize the present invention to its fullest extent. All references and publications cited herein are hereby incorporated by reference in their entirety.

### EXAMPLES AND INTERMEDIATE COMPOUNDS

### Experimental Methods

Microwave reactions were performed with a Personal Chemistry/Biotage microwave reactor using process vials fitted with aluminum caps and septa. Preparative flash chromatography was performed on Merck silica gel 60 (230-400 mesh). Preparative HPLC was performed on a Gilson system equipped with a UV detector in accordance to the experimental details specified in the examples. The purest fractions were collected, concentrated and dried in vacuo. All targeted compounds were analyzed by analytical HPLC/MS to ensure acceptable purity. The analysis was performed using an Agilent 1100/1200 Series Liquid Chromatograph/Mass Selective Detector (MSD) (Single Quadrupole) (1946A/1946C/1956C/6110) equipped with an electrospray interface. Accurate masses (HRMS) were measured using an Agilent MSD-TOF connected to an Agilent 1100 HPLC system. During the analyses the calibration was checked by two masses and automatically corrected when needed. Spectra were acquired in positive electrospray mode. The acquired mass range was m/z 100-1100. Profile detection of the mass peaks was used. The compounds prepared were named using ACD Name 6.0, 7.0 or 10.0. In addition, the commercial names or trivial names were used for many of the commercial starting materials and reagents.

### EXAMPLE 1

### [2-(4-Methylphenyl)imidazo[1,2-a]pyridin-3-yl]methanol

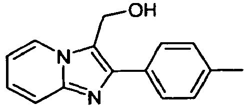

2-Amino-pyridine (14 mg, 0.15 mmol) and 2-bromo-4'-methylacetophenone (21 mg, 0.10 mmol) were mixed in ethanol (1 mL) and stirred at rt overnight. The reaction mixture was then warmed at 65 °C for 4 h. 0.5 M NaOH (3 mL) was added and a solid was collected by centrifugation. Sodium acetate (41 mg, 0.5 mmol) was added to this intermediate followed by 12.3 M formaldehyde in water (0.08 mL, 1.0 mmol) and acetic acid (1.0 mL). The reaction mixture was heated at 50 °C overnight. 4 M NaOH (4.5 mL) was added slowly with stirring and the reaction mixture was extracted with CH₂Cl₂ (3 mL). The crude product was purified by preparative HPLC (Xterra C18, 50 mM NH₄HCO₃ (pH 10) - CH₃CN) to give the title compound as a white solid (3 mg). HRMS (ESI+) calcd for C₁₅H₁₄N₂O 238.1106, found 238.1114.

### EXAMPLE 2

### [2-(2,4-Dichlorophenyl)imidazo[1,2-a]pyridin-3-yl]methanol

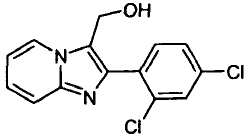

2-Aminopyridine (71 mg, 0.75 mmol) and 2,4-dichlorophenacyl chloride (112 mg, 0.50 mmol) were mixed in ethanol (1 mL) and stirred at rt over the weekend. The mixture was then heated at 70 °C overnight. 0.5 M NaOH (3 mL) was added slowly with stirring. The precipitate that formed was collected by centrifugation and washed with water (3.5 mL) to give the intermediate compound 2-(2,4-dichlorophenyl)imidazo[1,2-a]pyridine. MS (ESI+) m/z 263, 265 [M+H]+.

To this intermediate was added sodium acetate (205 mg, 2.5 mmol) followed by 12.3 M formaldehyde in water (0.41 mL, 5.0 mmol) and acetic acid (1.0 mL). The mixture was heated at 50 °C for 6 h and then 4 M NaOH (4.5 mL) was added slowly with stirring. The mixture was extracted with CH₂Cl₂ (2 x 2 mL). The organic phase was washed with brine (2 mL) and dried (MgSO₄). A portion of the organic solution (∼0.5 - 1 mL) was taken out and concentrated. The material was purified by preparative HPLC (Xterra C18, 50 mM NH₄HCO₃ (pH 10) - CH₃CN) to give the title compound as a white solid (14.3 mg). HRMS (ESI+) calcd for C₁₄H₁₀Cl₂N₂O 292.0170, found 292.0181.

### EXAMPLE 3

### [2-(4-Bromophenyl)-8-methylimidazo[1,2-a]pyridin-3-yl]methanol

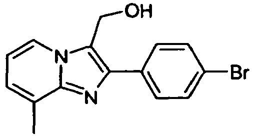

2-Bromo-1-(4-bromophenyl)ethanone (1.39 g, 5 mmol) in ethanol (5 mL) was treated with 3-methylpyridin-2-amine (0.51 mL, 7.5 mmol) at rt over the weekend. HBr in acetic acid (0.6 mL) was added and the crystals were filtered and washed with cold ethanol to yield a light yellow solid. The solid was stirred in ethanol (10 mL) for 1 h. The solid material was collected by filtration, washed with ethanol (3 mL) and dried *in vacuo* to give the intermediate compound 2-(4-bromophenyl)-8-methylimidazo[1,2-a]pyridine hydrobromide as a light yellow solid (1.25 g).

The intermediate (0.37 g, 1.0 mmol) was treated with 12.3 M formaldehyde in water (0.66 mL, 8 mmol) and sodium acetate (0.41 g, 5 mmol) in acetic acid (4 mL) at 50 °C for 3 h. 1 M NaOH (80 mL) was gently added and the crystals were collected by filtration, washed with water and dried *in vacuo* to give the title compound as a white solid (281 mg). HRMS (ESI+) calcd for C₁₅H₁₃BrN₂O 316.0211, found 316.0224.

### EXAMPLE 4

### [7-Methyl-2-(4-methylphenyl)imidazo[1,2-a]pyridin-3-yl]methanol

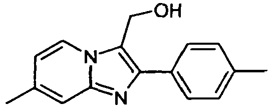

2-Amino-4-methylpyridine (16 mg, 0.15 mmol) and 2-bromo-4'-methylacetophenone (21 mg, 0.10 mmol) were mixed in ethanol (1 mL) and stirred at rt overnight. The reaction mixture was then heated at 65 °C for 4 h. 0.5 M NaOH (3 mL) was added slowly with stirring. The precipitate that formed was collected by centrifugation and washed with water (3.5 mL) to give the intermediate 7-methyl-2-(4-methylphenyl)imidazo[1,2-a]pyridine.

Sodium acetate (41 mg, 0.5 mmol) was added to the intermediate followed by 12.3 M formaldehyde in water (0.08 mL, 1.0 mmol) and acetic acid (1.0 mL). The reaction mixture was heated at 50 °C overnight. 4 M NaOH (4.5 mL) was added slowly with stirring and the reaction mixture was extracted with CH₂Cl₂ (3 mL). The organic phase was collected and concentrated. The crude product was purified by preparative HPLC (Xterra C 18, 50 mM NH₄HCO₃ (pH 10) - CH₃CN) to give the title compound as a white solid (2 mg). HRMS (ESI+) calcd for C₁₆H₁₆N₂O 252.1263, found 252.1272.

### EXAMPLE 5

### [2-(4-Bromophenyl)-7-methylimidazo[1,2-a]pyridin-3-yl]methanol

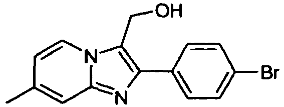

A mixture of 2-bromo-1-(4-bromophenyl)ethanone (208 mg, 0.75 mmol) and 4-methylpyridin-2-amine (85 mg, 0.79 mmol) were stirred in ethanol (1 mL) at rt overnight. An additional portion of 4-methylpyridin-2-amine (42 mg, 0.4 mmol) was added. After 6 h HBr in acetic acid (0.1 mL, 37%) was added with stirring. The mixture was then placed in the freezer overnight. The crystals were collected by filtration and washed with a small volume of cold acetone to give the intermediate compound 2-(4-bromophenyl)-7-methylimidazo[1,2-a]pyridine hydrobromide as an off-white solid (171 mg).

The intermediate (0.15 g, 0.40 mmol) was treated with 12.3 M formaldehyde in water (0.26 mL, 3.2 mmol) and sodium acetate (166 mg, 2 mmol) in acetic acid (1 mL) at rt for 4 days. 1 M NaOH (∼ 10 mL) was added with stirring. The crystals were collected by filtration and recrystallized from methanol to give the title compound as an off-white solid (69 mg). HRMS (ESI+) calcd for C₁₅H₁₃BrN₂O 316.0211, found 316.0219.

### EXAMPLE 6

### [2-(4-Bromophenyl)-7-ethylimidazo[1,2-a]pyridin-3-yl]methanol

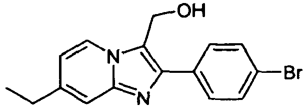

2-Bromo-1-(4-bromophenyl)ethanone (0.56 g, 2.0 mmol) and 4-ethylpyridin-2-amine (0.26 g, 2.1 mmol) were stirred in ethanol (2 mL) at rt overnight. The precipitate was collected by filtration and washed with ethanol. The material was recrystallized from ethanol/ethyl acetate to give the intermediate compound 2-(4-bromophenyl)-7-ethylimidazo[1,2-a]pyridine hydrobromide as a white solid (105 mg).

This intermediate (95 mg, 0.25 mmol) was treated with 12.3 M formaldehyde in water (0.16 mL, 2.0 mmol) and sodium acetate (0.10 g, 1.3 mmol) in acetic acid (1 mL) at 50 °C for 4 h. The mixture was added to 1 M NaOH (100 mL) and was left with stirring overnight. The precipitate was collected by filtration, washed with water until the filtrate was neutral, and *dried in vacuo* to give the title compound as an off-white solid (57 mg). HRMS (ESI+) calcd for C₁₆H₁₅BrN₂O 330.0368, found 330.0384.

### EXAMPLE 7

### [2-(2-Chlorophenyl)-7-methylimidazo[1,2-a]pyridin-3-yl]methanol

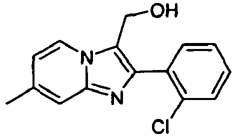

2-Amino-4-methylpyridine (81 mg, 0.75 mmol) and 2-bromo-1-(2-chlorophenyl)ethan-1-one (117 mg, 0.50 mmol) were mixed in ethanol (1 mL) and stirred at rt over the weekend. 0.5 M NaOH (3 mL) was added slowly with stirring. The precipitate that formed was collected by centrifugation and washed with water (3.5 mL) to give the intermediate 2-(2-chlorophenyl)-7-methylimidazo[1,2-a]pyridine. MS (ESI+) m/z 243, 245 [M+H]+.

To this intermediate was added sodium acetate (205 mg, 2.5 mmol) followed by 12.3 M formaldehyde in water (0.41 mL, 5.0 mmol) and acetic acid (1.0 mL). The mixture was heated at 50 °C for 6 h and then 4 M NaOH (4.5 mL) was added slowly with stirring. The mixture was extracted with CH₂Cl₂ (2 x 2 mL). The organic phase was washed with brine (2 mL) and dried (MgSO₄). A portion of the organic solution (∼0.5 - 1 mL) was taken out and concentrated. The material was purified by preparative HPLC (Xterra C18, 50 mM NH₄HCO₃ (pH 10) - CH₃CN) to give the title compound as a white solid (12 mg). HRMS (ESI+) calcd for C₁₅H₁₃ClN₂O 272.0716, found 272.0726.

### EXAMPLE 8

### [2-(2,4-Dichlorophenyl)-7-methylimidazo[1,2-a]pyridin-3-yl]methanol

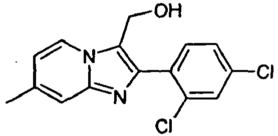

2-Amino-4-methylpyridine (81 mg, 0.75 mmol) and 2,4-dichlorophenacyl chloride (112 mg, 0.50 mmol) were mixed in ethanol (1 mL) and stirred at rt over the weekend. The mixture was then heated at 70 °C for 2 days. 0.5 M NaOH (3 mL) was added slowly with stirring. The precipitate that formed was collected by centrifugation and washed with water (3.5 mL) to give the intermediate compound 2-(2,4-dichlorophenyl)-7-methylimidazo[1,2-a]pyridine. MS (ESI+) m/z 277, 279 [M+H]+.

To the intermediate was added sodium acetate (205 mg, 2.5 mmol) followed by 12.3 M formaldehyde in water (0.41 mL, 5.0 mmol) and acetic acid (1.0 mL). The mixture was heated at 50 °C for 6 h and then 4 M NaOH (4.5 mL) was added slowly with stirring. The mixture was extracted with CH₂Cl₂ (2 x 2 mL). The organic phase was washed with brine (2 mL) and dried (MgSO₄). A portion of the organic solution (∼0.5 - 1 mL) was taken out and concentrated. The material was purified by preparative HPLC (Xterra C18, 50 mM NH₄HCO₃ (pH 10) - CH₃CN) to give the title compound as a white solid (9.9 mg). HRMS (ESI+) calcd for C₁₅H₁₂Cl₂N₂O 306.0327, found 306.0337.

### EXAMPLE 9

### [2-(3,4-Dichlorophenyl)-7-methylimidazo[1,2-a]pyridin-3-yl] methanol

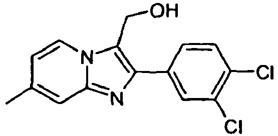

2-Amino-4-methylpyridine (16 mg, 0.15 mmol) and 2-bromo-3',4'-dichloroacetophenone (27 mg, 0.10 mmol) were mixed in ethanol (1 mL) and stirred at rt overnight. The reaction mixture was then heated at 65 °C for 4 h. 0.5 M NaOH (3 mL) was added was added slowly with stirring. The precipitate that formed was collected by centrifugation and washed with water (3.5 mL) to give the intermediate compound 2-(3,4-dichlorophenyl)-7-methylimidazo[1,2-a]pyridine.

To this intermediate was added sodium acetate (41 mg, 0.5 mmol) followed by 12.3 M formaldehyde in water (0.08 mL, 1.0 mmol) and acetic acid (1.0 mL). The reaction mixture was heated at 50 °C overnight. 4 M NaOH (4.5 mL) was added slowly with stirring and the reaction mixture was extracted with CH₂Cl₂ (3 mL). The organic phas was collected and concentrated. The crude product was purified by preparative HPLC (Xterra C18, 50 mM NH₄HCO₃ (pH 10) - CH₃CN) to give the title compound as a white solid (2 mg). HRMS (ESI+) calcd for C₁₅H₁₂Cl₂N₂O 306.0327, found 306.0339.

### EXAMPLE 10

### [6-Methyl-2-(2-naphthyl)imidazo[1,2-a]pyridin-3-yl]methanol

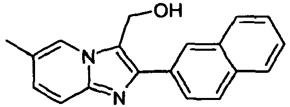

2-Amino-5-methylpyridine (81 mg, 0.75 mmol) and 2-bromo-2'-acetonaphthone (125 mg, 0.50 mmol) were mixed in ethanol (1 mL) and stirred at rt over the weekend. 0.5 M NaOH (3 mL) was added slowly with stirring. A precipitate formed which was collected by centrifugation and washed with water (3.5 mL) to give the intermediate compound 6-methyl-2-(2-naphthyl)imidazo[1,2-a]pyridine. MS (ESI+) m/z 259 [M+H]+.

To the intermediate was added sodium acetate (205 mg, 2.5 mmol) followed by 12.3 M formaldehyde in water (0.41 mL, 5.0 mmol) and acetic acid (1.0 mL). The mixture was heated at 50 °C for 2 h and then 4 M NaOH (4.5 mL) was added slowly with stirring. The mixture was extracted with CH₂Cl₂ (2 x 2 mL). The organic phase was washed with brine (2 mL) and dried (MgSO₄). A portion of the organic solution (∼0.5 - 1 mL) was taken out and concentrated. The material was purified by preparative HPLC (Xterra C18, 50 mM NH₄HCO₃ (pH 10) - CH₃CN) to give the title compound as a white solid (0.1 mg). HRMS (ESI+) calcd for C₁₉H₁₆N₂O 288.1263, found 288.1275.

### EXAMPLE 11

### [2-(3-Methoxyphenyl)-6-methylimidazo[1,2-a]pyridin-3-yl]methanol

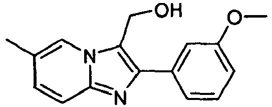

2-Amino-5-methylpyridine (81 mg, 0.75 mmol) and 2-bromo-3'-methoxyacetophenone (115 mg, 0.50 mmol) were mixed in ethanol (1 mL) and stirred at rt over the weekend. 0.5 M NaOH (3 mL) was added slowly with stirring. A precipitate formed which was collected by centrifugation and washed with water (3.5 mL) to give the intermediate compound 2-(3-methoxyphenyl)-6-methylimidazo[1,2-a]pyridine. MS (ESI+) m/z 239 [M+H]+.

To the intermediate was added sodium acetate (205 mg, 2.5 mmol) followed by 12.3 M formaldehyde in water (0.41 mL, 5.0 mmol) and acetic acid (1.0 mL). The mixture was heated at 50 °C for 2 h and then 4 M NaOH (4.5 mL) was added slowly with stirring. The mixture was extracted with CH₂Cl₂ (2 x 2 mL). The organic phase was washed with brine (2 mL) and dried (MgSO₄). A portion of the organic solution (∼0.5 - 1 mL) was taken out and concentrated. The material was purified by preparative HPLC (Xterra C18, 50 mM NH₄HCO₃ (pH 10) - CH₃CN) to give the title compound as a white solid (16.1 mg). HRMS (ESI+) calcd for C₁₆H₁₆N₂O₂ 268.1212, found 268.1221.

### EXAMPLE 12

### 4-[3-(Hydroxymethyl)-6-methylimidazo[1,2-a]pyridin-2-yl]benzonitrile

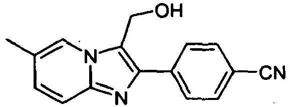

2-Amino-5-methylpyridine (324 mg, 3.0 mmol) and 4-(2-bromoacetyl)benzonitrile (448 mg, 2.0 mmol) were mixed in ethanol (2.5 mL) and stirred at 70 °C for 2 h. The reaction mixture was cooled and the precipitate was collected by filtration and washed with cold ethanol (2 x 2 mL). The precipitate was dried *in vacuo* to give the intermediate 4-(6-methylimidazo[1,2-a]pyridin-2-yl)benzonitrile hydrobromide as a yellow solid (287 mg).

To the intermediate (250 mg, 0.80 mmol) was added sodium acetate (326 mg, 4.0 mmol), 12.3 M formaldehyde in water (0.78 mL, 9.6 mmol), acetic acid (1.5 mL) and acetonitrile (1.5 mL). The mixture was heated at 50 °C for 2 h. After cooling the mixture was added to 2 M NaOH (20 mL) with stirring. The mixture was stirred for 1 h at rt. The precipitate was collected by filtration, washed with water (3 x 15 mL) and dried *in vacuo* to give the crude compound as a beige solid (200 mg). A small portion of the material (10 mg) was purified by preparative HPLC (Xterra C18, 50 mM NH₄HCO₃ (pH 10) - CH₃CN) to give the title compound as a white solid (1.7 mg). HRMS (ESI+) calcd for C₁₆H₁₃N₃O 263.1059, found 263.1066.

### EXAMPLE 13

### [6-Methyl-2-(3-nitrophenyl)imidazo[1,2-a]pyridin-3-yl]methanol

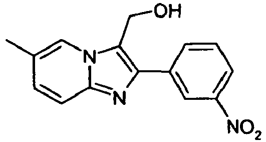

Sodium acetate (204 mg, 2.5 mmol), 12.3 M formaldehyde in water (0.48 mL, 6.0 mmol), acetic acid (1 mL) and acetonitrile (1 mL) were added to 6-methyl-2-(3-nitrophenyl)imidazo[1,2-a]pyridine (126 mg, 0.50 mmol), and the mixture was heated at 50 °C for 1.5 h. After cooling, the mixture was partitioned between 2 M NaOH (15 mL) and CH₂Cl₂ (30 mL) in the presence of methanol (10 mL). The organic phase was collected, washed with water (10 mL), dried (MgSO₄) and concentrated. The residue was finally dried *in vacuo* to give the title compound as a light yellow solid (114 mg). HRMS (ESI+) calcd for C₁₅H₁₃N₃O₃ 283.0957, found 283.0965.

### EXAMPLE 14

### [2-(4-Chlorophenyl)-6-methylimidazo[1,2-a]pyridin-3-yl]methanol

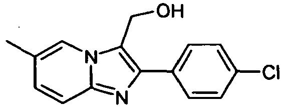

2-Amino-5-methylpyridine (81 mg, 0.75 mmol) and 4-chlorophenacyl bromide (117 mg, 0.50 mmol) were mixed in ethanol (1 mL) and stirred at rt over the weekend. 0.5 M NaOH (3 mL) was added slowly with stirring. A precipitate formed which was collected by centrifugation and washed with water (3.5 mL) to give the intermediate product 2-(4-chlorophenyl)-6-methylimidazo[1,2-a]pyridine. MS (ESI+) m/z 243, 245 [M+H]+.

To the intermediate was added sodium acetate (205 mg, 2.5 mmol) followed by 12.3 M formaldehyde in water (0.41 mL, 5.0 mmol) and acetic acid (1.0 mL). The mixture was heated at 50 °C for 2 h and then 4 M NaOH (4.5 mL) was added slowly with stirring. The mixture was extracted with CH₂Cl₂ (2 x 2 mL). The organic phase was washed with brine (2 mL) and dried (MgSO₄). A portion of the organic solution (∼0.5 - 1 mL) was taken out and concentrated. The material was purified by preparative HPLC (Xterra C18, 50 mM NH₄HCO₃ (pH 10) - CH₃CN) to give the title compound as a white solid (9.2 mg). HRMS (ESI+) calcd for C₁₅H₁₃ClN₂O 272.0716, found 272.0730.

### EXAMPLE 15

### [2-(4-Fluorophenyl)-6-methylimidazo[1,2-a]pyridin-3-yl]methanol

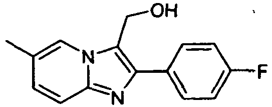

2-Amino-5-methylpyridine (16 mg, 0.15 mmol) and 2-bromo-4'-fluoroacetophenone (22 mg, 0.10 mmol) were mixed in ethanol (1 mL) and stirred at rt overnight. The reaction mixture was then warmed at 65 °C for 4 h. 0.5 M NaOH (3 mL) was added was added slowly with stirring. The precipitate that formed was collected by centrifugation and washed with water (3.5 mL) to give the intermediate compound 2-(4-fluorophenyl)-6-methylimidazo[1,2-a]pyridine.

To the intermediate was added sodium acetate (41 mg, 0.5 mmol) followed by 12.3 M formaldehyde in water (0.08 mL, 1.0 mmol) and acetic acid (1.0 mL). The reaction mixture was heated at 50 °C overnight. 4 M NaOH (4.5 mL) was added slowly with stirring and the reaction mixture was extracted with CH₂Cl₂ (3 mL). The organic phase was collected and concentrated. The crude product was purified by preparative HPLC (Xterra C18, 50 mM NH₄HCO₃ (pH 10) - CH₃CN) to give the title compound as a white solid (2 mg). HRMS (ESI+) calcd for C₁₅H₁₃FN₂O 256.1012, found 256.1020.

### EXAMPLE 16

### [2-(4-Iodophenyl)-6-methylimidazo[1,2-a] pyridin-3-yl]methanol

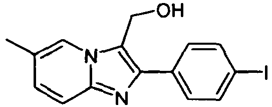

2-Amino-5-methylpyridine (162 mg, 1.5 mmol) and 4-iodophenacyl bromide (325 mg, 1.0 mmol) were mixed in ethanol (2.0 mL) and stirred at 70 °C for 1.5 h. The reaction mixture was cooled and the precipitate was collected by filtration and washed with cold ethanol (2 x 2 mL). The precipitate was partitioned between 2 M NaOH (2 mL) and CH₂Cl₂ (6 mL). The organic phase was collected, washed with water, dried (MgS04) and concentrated. The residue was dried *in vacuo* to give the intermediate compound 2-(4-iodophenyl)-6-methylimidazo[1,2-a]pyridine as a white solid (219 mg).

To the intermediate (200 mg, 0.60 mmol) was added sodium acetate (245 mg, 3.0 mmol), 12.3 M formaldehyde in water (0.58 mL, 7.2 mmol), acetic acid (1.5 mL) and acetonitrile (1.5 mL). The mixture was heated at 50 °C for 1.5 h. After cooling, the mixture was partitioned between 2 M NaOH (20 mL) and CH₂Cl₂ (40 mL). The organic phase was collected, washed with water, dried (MgSO4) and concentrated. The residue was dried in *vacuo* to give a white solid (208 mg). A small portion of the material (14 mg) was purified by preparative HPLC (Xterra C18, 50 mM NH₄HCO₃ (pH 10) - CH₃CN) to give the title compound as a white solid (10.4 mg). HRMS (ESI+) calcd for C₁₅H₁₃IN₂O 364.0073, found 364.0084.

### EXAMPLE 17

### [2-(2-Chlorophenyl)-6-methylimidazo[1,2-a]pyridin-3-yl]methanol

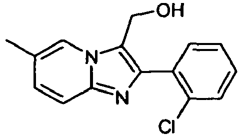

2-Amino-5-methylpyridine (81 mg, 0.75 mmol) and 2-bromo-1-(2-chlorophenyl)ethan-1-one (117 mg, 0.50 mmol) were mixed in ethanol (1 mL) and stirred at rt over the weekend. 0.5 M NaOH (3 mL) was added slowly with stirring. A precipitate formed which was collected by centrifugation and washed with water (3.5 mL) to give the intermediate compound 2-(2-chlorophenyl)-6-methylimidazo[1,2-a]pyridine. MS (ESI+) m/z 243, 245 [M+H]+.

To the intermediate was added sodium acetate (205 mg, 2.5 mmol) followed by 12.3 M formaldehyde in water (0.41 mL, 5.0 mmol) and acetic acid (1.0 mL). The mixture was heated at 50 °C for 6 h and then 4 M NaOH (4.5 mL) was added slowly with stirring. The mixture was extracted with CH₂Cl₂ (2 x 2 mL). The organic phase was washed with brine (2 mL) and dried (MgSO₄). A portion of the organic solution (∼0.5 - 1 mL) was taken out and concentrated. The material was purified by preparative HPLC (Xterra C18, 50 mM NH₄HCO₃ (pH 10) - CH₃CN) to give the title compound as a white solid (6.9 mg). HRMS (ESI+) calcd for C₁₅H₁₃ClN₂O 272.0716, found 272.0726.

### EXAMPLE 18

### (2-{4-[(2-Aminoethyl)amino]phenyl}-6-methylimidazo[1,2-a]pyridin-3-yl)methanol

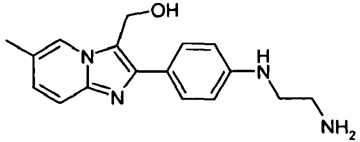

A mixture of [2-(4-iodophenyl)-6-methylimidazo[1,2-a]pyridin-3-yl]methanol (Example 16; 19 mg, 0.05 mmol), CuI (5 mg, 0.026 mmol) and ethylenediamine (174 µL, 2.6 mmol) was stirred at rt for 1 h. The mixture was diluted with MeOH (1.5 mL) and water (1 mL) and purified by preparative HPLC (Xterra C18, 50 mM NH₄HCO₃ (pH 10) - CH₃CN) to give the title compound as a white solid (1.8 mg). HRMS (ESI+) calcd for C₁₇H₂₀N₄O 296.1637, found 296.1644.

### EXAMPLE 19

### 1-[2-(4-Chlorophenyl)-6-methylimidazo[1,2-a]pyridin-3-yl]ethanol

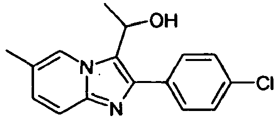

2-(4-Chlorophenyl)-6-methylimidazo[1,2-a]pyridine (100 mg, 0.41 mmol; see Example 14, intermediate product) was suspended in DMF (1 mL) and POCl₃ (92 µL, 0.99 mmol) was added under nitrogen. The reaction mixture was stirred at rt for 10 min and then heated at 100 °C for 20 min. 0.5 M Na₂CO₃ (5 mL) was added dropwise to the reaction mixture. A precipitate formed which was collected by centrifugation and washed with water (5 mL). The material was then partitioned between CH₂Cl₂ and water. The organic phase was dried (MgSO₄) and concentrated, and the residue was dried *in vacuo* to give the intermediate 2-(4-chlorophenyl)-6-methylimidazo[1,2-a]pyridine-3-carbaldehyde as a yellow solid (98 mg).

To the intermediate (14 mg, 46 µmol) was added methyl magnesiumbromide (3M in diethyl ether, 0.5 mL) at rt. The reaction mixture was stirred at rt for 1 h. The reaction was then quenched by addition of saturated NH₄Cl (2 mL) and extracted with ethyl acetate (3 x 5 mL). The solvent was removed under reduced pressure and the crude product was purified by preparative HPLC (Xterra C18, 50 mM NH₄HCO₃ (pH 10) - CH₃CN) to give the title compound as a white solid (7 mg). HRMS (ESI+) calcd for C₁₆H₁₅ClN₂O 286.0873, found 286.0886.

### EXAMPLE 20

### [2-(2,4-Dichlorophenyl)-6-methylimidazo[1,2-a]pyridin-3-yl]methanol

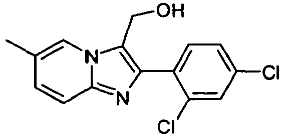

2-Amino-5-methylpyridine (81 mg, 0.75 mmol) and 2,4-dichlorophenacyl chloride (112 mg, 0.50 mmol) were mixed in ethanol (1 mL) and stirred at rt over the weekend. The mixture was then heated at 70 °C for 2 days. 0.5 M NaOH (3 mL) was added slowly with stirring. A precipitate formed which was collected by centrifugation and washed with water (3.5 mL) to give the intermediate 2-(2,4-dichlorophenyl)-6-methylimidazo[1,2-a]pyridine. MS (ESI+) m/z 277, 279 [M+H]+.

To the intermediate was added sodium acetate (205 mg, 2.5 mmol) followed by 12.3 M formaldehyde in water (0.41 mL, 5.0 mmol) and acetic acid (1.0 mL). The mixture was heated at 50 °C for 2 h and then 4 M NaOH (4.5 mL) was added slowly with stirring. The mixture was extracted with CH₂Cl₂ (2 x 2 mL). The organic phase was washed with brine (2 mL) and dried (MgSO₄). A portion of the organic solution (∼0.5 - 1 mL) was taken out and concentrated. The material was purified by preparative HPLC (Xterra C18, 50 mM NH₄HCO₃ (pH 10) - CH₃CN) to give the title compound as a white solid (10.9 mg). HRMS (ESI+) calcd for C₁₅H₁₂Cl₂N₂O 306.0327, found 306.0340.

### EXAMPLE 21

### [2-(3-Methoxyphenyl)-6-(trifluoromethyl)imidazo[1,2-a]pyridin-3-yl] methanol

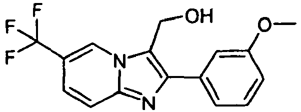

2-Amino-5-(trifluoromethyl)pyridine (49 mg, 0.30 mmol) and 2-bromo-3'-methoxyacetophenone (46 mg, 0.20 mmol) were mixed in ethanol (0.5 mL) and stirred at rt over the weekend. The mixture was then heated at 70 °C for 2 h. 0.5 M NaOH (1.2 mL) was added slowly with stirring. A precipitate that formed was collected by centrifugation and washed with water (1.5 mL) to give the intermediate product 2-(3-methoxyphenyl)-6-(trifluoromethyl)imidazo[1,2-a]pyridine. MS (ESI+) m/z 293 [M+H]+.

To the intermediate was added sodium acetate (82 mg, 1.0 mmol) followed by 12.3 M formaldehyde in water (0.16 mL, 2.0 mmol), acetic acid (0.4 mL) and acetonitrile (0.5 mL). The reaction mixture was heated at 50 °C for 3 h and then concentrated. Part of the material (∼50%) was purified by preparative HPLC (Xterra C18, 50 mM NH₄HCO₃ (pH 10) - CH₃CN) to give the title compound as a white solid (18 mg). HRMS (ESI+) calcd for C₁₆H₁₃F₃N₂O₂ 322.0929, found 322.0931.

### EXAMPLE 22

### [2-(4-Chlorophenyl)-6-(trifluoromethyl)imidazo[1,2-a]pyridin-3-yl]methanol

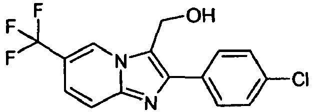

2-Amino-5-(trifluoromethyl)pyridine (49 mg, 0.30 mmol) and 4-chlorophenacyl bromide (47 mg, 0.20 mmol) were mixed in ethanol (0.5 mL) and stirred at rt over the weekend. The mixture was then heated at 70 °C for 4 h. 0.5 M NaOH (1.2 mL) was added slowly with stirring. A precipitate formed which was collected by centrifugation and washed with water (1.5 mL) to give the intermediate product 2-(4-chlorophenyl)-6-(trifluoromethyl)imidazo[1,2-a]pyridine. MS (ESI+) m/z 297, 299 [M+H]+.

To the intermediate was added sodium acetate (82 mg, 1.0 mmol) followed by 12.3 M formaldehyde in water (0.16 mL, 2.0 mmol), acetic acid (0.4 mL) and acetonitrile (0.5 mL). The reaction mixture was heated at 50 °C for 3 h. 4 M NaOH (1.8 mL) was added slowly with stirring and the mixture was extracted with CH₂Cl₂ (2 x 2 mL). The organic phase was washed with brine (2 mL) and concentrated. Part of the material (∼50%) was purified by preparative HPLC (Xterra C18, 50 mM NH₄HCO₃ (pH 10) - CH₃CN) to give the title compound as a white solid (15 mg). HRMS (ESI+) calcd for C₁₅H₁₀ClF₃N₂O 326.0434, found 326.0446.

### EXAMPLE 23

### [2-(4-Bromophenyl)-6-(trifluoromethyl)imidazo[1,2-a]pyridin-3-yl] methanol

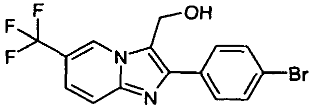

2-Bromo-1-(4-bromophenyl)ethanone (0.56 g, 2.0 mmol) and 5-(trifluoromethyl)pyridin-2-amine (0.34 g, 2.1 mmol) were stirred in ethanol (2 mL) at 60 °C overnight. The precipitate that formed was collected by filtration and washed with ethanol to give the intermediate 2-(4-bromophenyl)-6-(trifluoromethyl)imidazo[1,2-a]pyridine hydrobromide as a white solid (562 mg).

The intermediate (0.21 g, 0.50 mmol) was treated with 12.3 M formaldehyde in water (0.32 mL, 4.0 mmol) and sodium acetate (0.20 g, 2.5 mmol) in acetic acid (2 mL) at 50 °C for 4 h. The mixture was added to 1 M NaOH (100 mL) and was left with stirring overnight. The precipitate was collected by filtration, washed with water until the filtrate was neutral, and dried *in vacuo* to give the title compound as an off-white solid (163 mg). HRMS (ESI+) calcd for C₁₅H₁₀BrF₃N₂O 369.9929, found 369.9938.

### EXAMPLE 24

### [7-Chloro-2-(4-chlorophenyl)imidazo[1,2-a]pyridin-3-yl]methanol

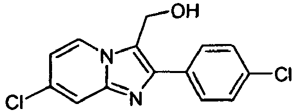

2-Amino-4-chloropyridine (39 mg, 0.30 mmol) and 4-chlorophenacyl bromide (47 mg, 0.20 mmol) were mixed in ethanol (0.5 mL) and stirred at rt over the weekend. The mixture was then heated at 70 °C for 2 h. 0.5 M NaOH (1.2 mL) was added slowly with stirring. A precipitate formed which was collected by centrifugation and washed with water (1.5 mL) to give the intermediate 7-chloro-2-(4-chlorophenyl)imidazo[1,2-a]pyridine. MS (ESI+) m/z 263, 265 [M+H]+.

To the intermediate was added sodium acetate (82 mg, 1.0 mmol) followed by 12.3 M formaldehyde in water (0.16 mL, 2.0 mmol), acetic acid (0.4 mL) and acetonitrile (0.5 mL). The reaction mixture was heated at 50 °C for 4 h. 4 M NaOH (1.8 mL) was added slowly with stirring and the mixture was extracted with CH₂Cl₂ (2 x 2 mL). The organic phase was washed with brine (2 mL) and concentrated. Part of the material (∼50%) was purified by preparative HPLC (Xterra C18, 50 mM NH₄HCO₃ (pH 10) - CH₃CN) to give the title compound as a white solid (20 mg). HRMS (ESI+) calcd for C₁₄H₁₀Cl₂N₂O 292.0170, found 292.0183.

### EXAMPLE 25

### [2-(4-Bromophenyl)-7-chloroimidazo[1,2-a]pyridin-3-yl]methanol trifluoroacetate

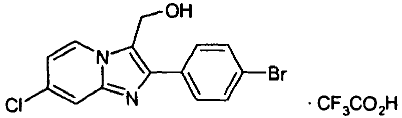

A mixture of 2-amino-4-chloropyridine (0.26 g, 2.0 mmol) and 2,4'-dibromoacetophenone (0.37 g, 1.5 mmol) in ethanol (5 mL) was heated at 65 °C overnight. The precipitate that formed was collected by filtration, washed with ethanol (10 mL) and dried under reduced pressure to give the intermediate 2-(4-bromophenyl)-7-chloroimidazo[1,2-a]pyridine hydrobromide as a white solid (0.32 g).

To the intermediate (0.32 g, 0.83 mmol) in acetic acid (5 mL) was added 12.3 M formaldehyde in water (0.54 mL, 6.6 mmol) and sodium acetate (340 mg, 4.2 mmol). The reaction mixture was heated at 45 °C for 4 h and then allowed to cool to rt. 1 M NaOH (50 mL) was added whereby the product precipitated. The precipitate was isolated by filtration to give the crude product (270 mg). A small amount was purified by preparative HPLC (ACE C8, water containing 0.1% TFA - CH₃CN) to give the title compound (7 mg). HRMS (ESI+) calcd for C₁₄H₁₀BrClN₂O 335.9665, found 335.9680.

### EXAMPLE 26

### [7-Chloro-2-(2,4-dichlorophenyl)imidazo[1,2-a]pyridin-3-yl]methanol

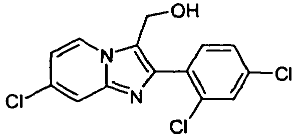

2-Amino-4-chloropyridine (39 mg, 0.30 mmol) and 2,4-dichlorophenacyl chloride (45 mg, 0.20 mmol) were mixed in ethanol (0.5 mL) and stirred at rt over the weekend. The mixture was then heated at 70 °C for 2 days. 0.5 M NaOH (1.2 mL) was added slowly with stirring. The precipitate that formed was collected by centrifugation and washed with water (1.5 mL) to give the intermediate 7-chloro-2-(2,4-dichlorophenyl)imidazo[1,2-a]pyridine. MS (ESI+) m/z 297, 299 [M+H]+.

To the intermediate (∼0.20 mmol) was added sodium acetate (82 mg, 1.0 mmol) followed by 12.3 M formaldehyde in water (0.16 mL, 2.0 mmol), acetic acid (0.4 mL) and acetonitrile (0.5 mL). The reaction mixture was heated at 50 °C for 2 days and then concentrated. Part of the material (∼50%) was purified by preparative HPLC (Xterra C18, 50 mM NH₄HCO₃ (pH 10) - CH₃CN) to give the title compound as a white solid (12 mg). HRMS (ESI+) calcd for C₁₄H₉Cl₃N₂O 325.9780, found 325.9791.

### EXAMPLE 27

### [7-Chloro-2-(2,4-difluorophenyl)imidazo[1,2-a]pyridin-3-yl]methanol

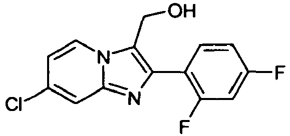

2-Amino-4-chloropyridine (39 mg, 0.30 mmol) and 2,4-difluorophenacyl bromide (47 mg, 0.20 mmol) were mixed in ethanol (0.5 mL) and stirred at rt over the weekend. The mixture was then heated at 70 °C for 2 h. 0.5 M NaOH (0.6 mL) was added slowly with stirring. The precipitate that formed was collected by centrifugation and washed with water (1.5 mL) to give the intermediate 7-chloro-2-(2,4-difluorophenyl)imidazo[1,2-a]pyridine. MS (ESI+) m/z 265 [M+H]+.

To the intermediate was added sodium acetate (82 mg, 1.0 mmol) followed by 12.3 M formaldehyde in water (0.20 mL, 2.4 mmol), acetic acid (0.4 mL) and acetonitrile (0.5 mL). The reaction mixture was heated at 50 °C for 3 h and then concentrated. Part of the material (∼50%) was purified by preparative HPLC (Xterra C18, 50 mM NH₄HCO₃ (pH 10) - CH₃CN) to give the title compound as a white solid (12 mg). HRMS (ESI+) calcd for C₁₄H₉ClF₂N₂O 294.0371, found 294.0378.

### EXAMPLE 28

### [6-Bromo-2-(3-methoxyphenyl)imidazo[1,2-a]pyridin-3-yl]methanol

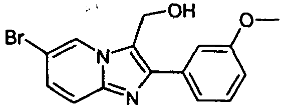

2-Amino-5-bromopyridine (52 mg, 0.30 mmol) and 2-bromo-3'-methoxyacetophenone (46 mg, 0.20 mmol) were mixed in ethanol (0.5 mL) and stirred at rt over the weekend. The mixture was then heated at 70 °C for 1 h. 0.5 M NaOH (1.2 mL) was added slowly with stirring. The precipitate that formed was collected by centrifugation and washed with water (1.5 mL) to give the intermediate 6-bromo-2-(3-methoxyphenyl)imidazo[1,2-a]pyridine. MS (ESI+) m/z 303, 305 [M+H]+.

To the intermediate was added sodium acetate (82 mg, 1.0 mmol) followed by 12.3 M formaldehyde in water (0.16 mL, 2.0 mmol), acetic acid (0.4 mL) and acetonitrile (0.5 mL). The reaction mixture was heated at 50 °C for 4 h and then concentrated. Part of the material (∼50%) was purified by preparative HPLC (Xterra C18, 50 mM NH₄HCO₃ (pH 10) - CH₃CN) to give the title compound as a white solid (23 mg). HRMS (ESI+) calcd for C₁₅H₁₃BrN₂O₂ 332.0160, found 332.0173.

### EXAMPLE 29

### [6-Chloro-2-(4-chlorophenyl)imidazo[1,2-a]pyridin-3-yl]methanol

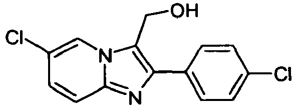

2-Amino-5-chloropyridine (39 mg, 0.30 mmol) and 4-chlorophenacyl bromide (47 mg, 0.20 mmol) were mixed in ethanol (0.5 mL) and stirred at rt over the weekend. The mixture was then heated at 70 °C overnight. 0.5 M NaOH (1.2 mL) was added slowly with stirring. The precipitate that formed was collected by centrifugation and washed with water (1.5 mL) to give the intermediate compound 6-chloro-2-(4-chlorophenyl)imidazo[1,2-a]pyridine. MS (ESI+) m/z 263, 265 [M+H]+.

To the intermediate was added sodium acetate (82 mg, 1.0 mmol) followed by 12.3 M formaldehyde in water (0.16 mL, 2.0 mmol), acetic acid (0.4 mL) and acetonitrile (0.5 mL). The reaction mixture was heated at 50 °C for 8 h. 4 M NaOH (1.8 mL) was added slowly with stirring and the mixture was extracted with CH₂Cl₂ (2 x 2 mL). The organic phase was washed with brine (2 mL) and concentrated. Part of the material (∼50%) was purified by preparative HPLC (Xterra C18, 50 mM NH₄HCO₃ (pH 10) - CH₃CN) to give the title compound as a white solid (12.1 mg). HRMS (ESI+) calcd for C₁₄H₁₀Cl₂N₂O 292.0170, found 292.0183.

### EXAMPLE 30

### [6-Bromo-2-(4-fluorophenyl)imidazo[1,2-a]pyridin-3-yl]methanol

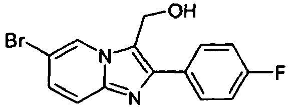

2-Amino-5-bromopyridine (52 mg, 0.30 mmol) and 4-fluorophenacyl bromide (43 mg, 0.20 mmol) were mixed in ethanol (0.5 mL) and stirred at rt over the weekend. The mixture was then heated at 70 °C for 2 h. 0.5 M NaOH (0.6 mL) was added slowly with stirring. The precipitate that formed was collected by centrifugation and washed with water (1.5 mL) to give the intermediate 6-bromo-2-(4-fluorophenyl)imidazo[1,2-a]pyridine. MS (ESI+) m/z 291, 293 [M+H]+.

To the intermediate was added sodium acetate (82 mg, 1.0 mmol) followed by 12.3 M formaldehyde in water (0.20 mL, 2.4 mmol), acetic acid (0.4 mL) and acetonitrile (0.5 mL). The reaction mixture was heated at 50 °C for 1 h and then concentrated. Part of the material (∼50%) was purified by preparative HPLC (Xterra C18, 50 mM NH₄HCO₃ (pH 10) - CH₃CN) to give the title compound as a white solid (17 mg). HRMS (ESI+) calcd for C₁₄H₁₀BrFN₂O 319.9961, found 319.9973.

### EXAMPLE 31

### [6-Bromo-2-(4-bromophenyl)imidazo[1,2-a]pyridin-3-yl]methanol trifluoroacetate

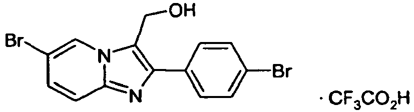

A mixture of 2-amino-5-bromopyridine (0.35 g, 2.0 mmol) and 2,4'-dibromoacetophenone (0.37 g, 1.5 mmol) in ethanol (5 mL) was heated at 65 °C overnight. The precipitate that formed was collected by filtration, washed with ethanol (10 mL) and dried under reduced pressure to give the intermediate 6-bromo-2-(4-bromophenyl)imidazo[1,2-a]pyridine hydrobromide as a white solid (0.33 g).

To the intermediate (0.33 g, 0.76 mmol) in acetic acid (5 mL) was added 12.3 M formaldehyde in water (0.37 mL, 6.1 mmol) and sodium acetate (310 mg, 3.8 mmol). The reaction mixture was heated at 45 °C for 4 h and then allowed to cool to rt. To the reaction mixture was added 1 M NaOH (50 mL) whereby the product precipitated. The precipitate was collected by filtration to give the crude product (326 mg). A small amount was purified by preparative HPLC (ACE C8, water containing 0.1 % TFA - CH₃CN) to give the title compound as a solid (21 mg). HRMS (ESI+) calcd for C₁₄H₁₀Br₂N₂O 379.9160, found 379.9172.

### EXAMPLE 32

### [2-(4-Bromophenyl)-6-chloroimidazo[1,2-a]pyridin-3-yl]methanol trifluoroacetate

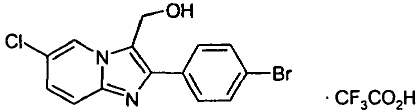

A mixture of 2-amino-5-chloropyridine (0.26 g, 2.0 mmol) and 2,4'-dibromoacetophenone (0.28 g, 1.0 mmol) in ethanol (5 mL) was heated at 65 °C overnight. The precipitate that formed was collected by filtration, washed with ethanol (10 mL) and dried under reduced pressure to give the intermediate 2-(4-bromophenyl)-6-chloroimidazo[1,2-a]pyridine hydrobromide as a white solid (211 mg).

To the intermediate (211 mg, 0.54 mmol) in acetic acid (5 mL) was added 12.3 M formaldehyde in water (0.36 mL, 4.3 mmol) and sodium acetate (222 mg, 2.71 mmol). The reaction mixture was heated at 45 °C for 5 h and then allowed to cool to rt. To the reaction mixture was added 1 M NaOH (50 mL) whereby the product precipitated. The precipitate was collected by filtration and recrystallized from methanol to give a white solid (172 mg). A portion of the material was purified by preparative HPLC (Xterra C18, 50 mM NH₄HCO₃ (pH 10) - CH₃CN) and then repurified (ACE C8, water containing 0.1% TFA - CH₃CN) to give the title compound as a white solid (5.4 mg). HRMS (ESI+) calcd for C₁₄H₁₀BrClN₂O 335.9665, found 335.9668.

### EXAMPLE 33

### [2-(4-Chlorophenyl)-6-fluoroimidazo[1,2-a]pyridin-3-yl]methanol

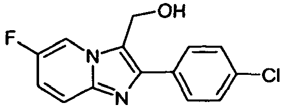

2-Amino-5-fluoropyridine (34 mg, 0.30 mmol) and 4-chlorophenacyl bromide (47 mg, 0.20 mmol) were mixed in ethanol (0.5 mL) and stirred at rt over the weekend. The mixture was then heated at 70 °C for 2 h. 0.5 M NaOH (1.2 mL) was added slowly with stirring. The precipitate that formed was collected by centrifugation and washed with water (1.5 mL) to give the intermediate 2-(4-chlorophenyl)-6-fluoroimidazo[1,2-a]pyridine. MS (ESI+) m/z 247, 249 [M+H]+.

To the intermediate was added sodium acetate (82 mg, 1.0 mmol) followed by 12.3 M formaldehyde in water (0.16 mL, 2.0 mmol), acetic acid (0.4 mL) and acetonitrile (0.5 mL). The reaction mixture was heated at 50 °C for 4 h. 4 M NaOH (1.8 mL) was added slowly with stirring and the mixture was extracted with CH₂Cl₂ (2 x 2 mL). The organic phase was washed with brine (2 mL) and concentrated. Part of the material (∼50%) was purified by preparative HPLC (Xterra C18, 50 mM NH₄HCO₃ (pH 10) - CH₃CN) to give the title compound as a light yellow solid (10 mg). HRMS (ESI+) calcd for C₁₄H₁₀ClFN₂O 276.0466, found 276.0476.

### EXAMPLE 34

### [6-Bromo-2-(2,4-difluorophenyl)imidazo[1,2-a] pyridin-3-yl]methanol

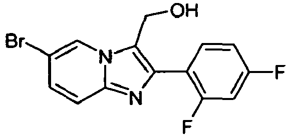

2-Amino-5-bromopyridine (52 mg, 0.30 mmol) and 2,4-difluorophenacyl bromide (47 mg, 0.20 mmol) were mixed in ethanol (0.5 mL) and stirred at rt over the weekend. The mixture was then heated at 70 °C for 2 h. 0.5 M NaOH (0.6 mL) was added slowly with stirring. A precipitate formed which was collected by centrifugation and washed with water (1.5 mL) to give the intermediate 6-bromo-2-(2,4-difluorophenyl)imidazo[1,2-a]pyridine. MS (ESI+) m/z 309, 311 [M+H]+.

To the intermediate was added sodium acetate (82 mg, 1.0 mmol) followed by 12.3 M formaldehyde in water (0.20 mL, 2.4 mmol), acetic acid (0.4 mL) and acetonitrile (0.5 mL). The reaction mixture was heated at 50 °C for 6 h and then concentrated. Part of the material (∼50%) was purified by preparative HPLC (Xterra C18, 50 mM NH₄HCO₃ (pH 10) - CH₃CN) to give the title compound as a white solid (17 mg). HRMS (ESI+) calcd for C₁₄H₉BrF₂N₂O 337.9867, found 337.9880.

### EXAMPLE 35

### [6-Chloro-2-(2,4-difluorophenyl)imidazo[1,2-a]pyridin-3-yl]methanol

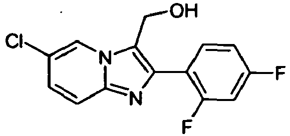

2-Amino-5-chloropyridine (39 mg, 0.30 mmol) and 2,4-difluorophenacyl bromide (47 mg, 0.20 mmol) were mixed in ethanol (0.5 mL) and stirred at rt over the weekend. The mixture was then heated at 70 °C for 2 h. 0.5 M NaOH (0.6 mL) was added slowly with stirring. The precipitate that formed was collected by centrifugation and washed with water (1.5 mL) to give the intermediate 6-chloro-2-(2,4-difluorophenyl)imidazo[1,2-a]pyridine. MS (ESI+) m/z 265 [M+H]+.

To the intermediate was added sodium acetate (82 mg, 1.0 mmol) followed by 12.3 M formaldehyde in water (0.20 mL, 2.4 mmol), acetic acid (0.4 mL) and acetonitrile (0.5 mL). The reaction mixture was heated at 50 °C for 4 h and then concentrated. Part of the material (∼50%) was purified by preparative HPLC (Xterra C18, 50 mM NH₄HCO₃ (pH 10) - CH₃CN) to give the title compound as a white solid (14 mg). HRMS (ESI+) calcd for C₁₄H₉ClF₂N₂O 294.0371, found 294.0385.

### EXAMPLE 36

### [6-Bromo-2-(2,4-dichlorophenyl)imidazo[1,2-a]pyridin-3-yl]methanol

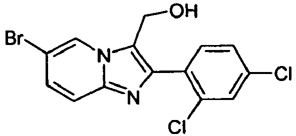

2-Amino-5-bromopyridine (52 mg, 0.30 mmol) and 2,4-dichlorophenacyl chloride (45 mg, 0.20 mmol) were mixed in ethanol (0.5 mL) and stirred at rt over the weekend. The mixture was then heated at 70 °C for 2 days. 0.5 M NaOH (1.2 mL) was added slowly with stirring. The precipitate that formed was collected by centrifugation and washed with water (1.5 mL) to give the intermediate 6-bromo-2-(2,4-dichlorophenyl)imidazo[1,2-a]pyridine. MS (ESI+) m/z 341, 343 [M+H]+.

To the intermediate was added sodium acetate (82 mg, 1.0 mmol) followed by 12.3 M formaldehyde in water (0.16 mL, 2.0 mmol), acetic acid (0.4 mL) and acetonitrile (0.5 mL). The reaction mixture was heated at 50 °C for 2 days and then concentrated. Part of the material (∼50%) was purified by preparative HPLC (Xterra C18, 50 mM NH₄HCO₃ (pH 10) - CH₃CN) to give the title compound as a white solid (9.5 mg). HRMS (ESI+) calcd for C₁₄H₉BrCl₂N₂O 369.9275, found 369.9285.

### EXAMPLE 37

### [6-Chloro-2-(2,4-dichlorophenyl)imidazo[1,2-a]pyridin-3-yl]methanol

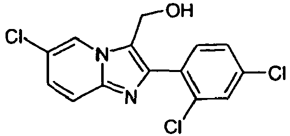

2-Amino-5-chloropyridine (39 mg, 0.30 mmol) and 2,4-dichlorophenacyl chloride (45 mg, 0.20 mmol) were mixed in ethanol (0.5 mL) and stirred at rt over the weekend. The mixture was then heated at 70 °C for 2 days. 0.5 M NaOH (1.2 mL) was added slowly with stirring. The precipitate that formed was collected by centrifugation and washed with water (1.5 mL) to give the intermediate 6-chloro-2-(2,4-dichlorophenyl)imidazo[1,2-a]pyridine. MS (ESI+) m/z 297, 299 [M+H]+.

To the intermediate was added sodium acetate (82 mg, 1.0 mmol) followed by 12.3 M formaldehyde in water (0.16 mL, 2.0 mmol), acetic acid (0.4 mL) and acetonitrile (0.5 mL). The reaction mixture was heated at 50 °C for 2 days and then concentrated. Part of the material (∼50%) was purified by preparative HPLC (Xterra C18, 50 mM NH₄HCO₃ (pH 10) - CH₃CN) to give the title compound as a white solid (16 mg). HRMS (ESI+) calcd for C₁₄H₉Cl₃N₂O 325.9780, found 325.9796.

### EXAMPLE 38

### [6-Bromo-2-(3,4-difluorophenyl)imidazo[1,2-a]pyridin-3-yl]methanol

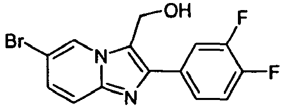

2-Amino-5-bromopyridine (52 mg, 0.30 mmol) and 2-bromo-3',4'-difluoroacetophenone (47 mg, 0.20 mmol) were mixed in ethanol (0.5 mL) and stirred at rt over the weekend. The mixture was then heated at 70 °C for 2 h. 0.5 M NaOH (0.6 mL) was added slowly with stirring. The precipitate that formed was collected by centrifugation and washed with water (1.5 mL) to give the intermediate 6-bromo-2-(3,4-difluorophenyl)imidazo[1,2-a]pyridine. MS (ESI+) m/z 309, 311 [M+H]+.

To the intermediate was added sodium acetate (82 mg, 1.0 mmol) followed by 12.3 M formaldehyde in water (0.20 mL, 2.4 mmol), acetic acid (0.4 mL) and acetonitrile (0.5 mL). The reaction mixture was heated at 50 °C for 2 h and then concentrated. Part of the material (∼50%) was purified by preparative HPLC (Xterra C18, 50 mM NH₄HCO₃ (pH 10) - CH₃CN) to give the title compound as a white solid (7.1 mg). HRMS (ESI+) calcd for C₁₄H₉BrF₂N₂O 337.9866, found 337.9879.

### EXAMPLE 39

### [6-Bromo-2-(3-chloro-4-fluorophenyl)imidazo[1,2-a]pyridin-3-yl]methanol

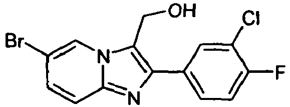

2-Amino-5-bromopyridine (52 mg, 0.30 mmol) and 2-bromo-1-(3-chloro-4-fluorophenyl)ethanone (50 mg, 0.20 mmol) were mixed in ethanol (0.5 mL) and stirred at rt over the weekend. The mixture was then heated at 70 °C for 2 h. 0.5 M NaOH (0.6 mL) was added slowly with stirring. The precipitate that formed was collected by centrifugation and washed with water (1.5 mL) to give the intermediate 6-bromo-2-(3-chloro-4-fluorophenyl)imidazo[1,2-a]pyridine. MS (ESI+) m/z 325, 327 [M+H]+.

To the intermediate was added sodium acetate (82 mg, 1.0 mmol) followed by 12.3 M formaldehyde in water (0.20 mL, 2.4 mmol), acetic acid (0.4 mL) and acetonitrile (0.5 mL). The reaction mixture was heated at 50 °C for 6 h and then concentrated. Part of the material (∼50%) was purified by preparative HPLC (Xterra C18, 50 mM NH₄HCO₃ (pH 10) - CH₃CN) to give the title compound as a white solid (2.5 mg). HRMS (ESI+) calcd for C₁₄H₉BrClFN₂O 353.9571, found 353.9577.

### EXAMPLE 40

### [6-Chloro-2-(3-chloro-4-fluorophenyl)imidazo[1,2-a]pyridin-3-yl]methanol

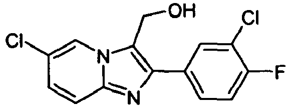

2-Amino-5-chloropyridine (39 mg, 0.30 mmol) and 2-bromo-1-(3-chloro-4-fluorophenyl)ethanone (50 mg, 0.20 mmol) were mixed in ethanol (0.5 mL) and stirred at rt over the weekend. The mixture was then heated at 70 °C for 2 h. 0.5 M NaOH (0.6 mL) was added slowly with stirring. The precipitate that formed was collected by centrifugation and washed with water (1.5 mL) to give the intermediate 6-chloro-2-(3-chloro-4-fluorophenyl)imidazo[1,2-a]pyridine. MS (ESI+) m/z 281, 283 [M+H]+.

To the intermediate was added sodium acetate (82 mg, 1.0 mmol) followed by 12.3 M formaldehyde in water (0.20 mL, 2.4 mmol), acetic acid (0.4 mL) and acetonitrile (0.5 mL). The reaction mixture was heated at 50 °C for 6 h and then concentrated. Part of the material (∼50%) was purified by preparative HPLC (Xterra C18, 50 mM NH₄HCO₃ (pH 10) - CH₃CN) to give the title compound as a white solid (10 mg). HRMS (ESI+) calcd for C₁₄H₉Cl₂FN₂O 310.0076, found 310.0084.

### EXAMPLE 41

### [6,8-Dichloro-2-(3-methoxyphenyl)imidazo[1,2-a]pyridin-3-yl]methanol

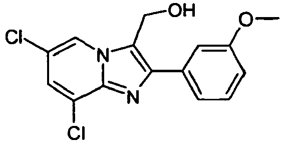

2-Amino-3,5-dichloropyridine (49 mg, 0.30 mmol) and 2-bromo-3'-methoxyacetophenone (46 mg, 0.20 mmol) were mixed in ethanol (0.5 mL) and stirred at rt over the weekend. The mixture was then heated at 70 °C overnight. 0.5 M NaOH (1.2 mL) was added slowly with stirring. The precipitate that formed was collected by centrifugation and washed with water (1.5 mL) to give the intermediate 6,8-dichloro-2-(3-methoxyphenyl)imidazo[1,2-a]pyridine. MS (ESI+) m/z 293, 295 [M+H]+.

To the intermediate was added sodium acetate (82 mg, 1.0 mmol) followed by 12.3 M formaldehyde in water (0.16 mL, 2.0 mmol), acetic acid (0.4 mL) and acetonitrile (0.5 mL). The reaction mixture was heated at 50 °C for 8 h and then concentrated. Part of the material (∼50%) was purified by preparative HPLC (Xterra C18, 50 mM NH₄HCO₃ (pH 10) - CH₃CN) to give the title compound as a white solid (6.8 mg). HRMS (ESI+) calcd for C₁₅H₁₂Cl₂N₂O₂ 322.0276, found 322.0290.

### EXAMPLE 42

### [2-(4-Bromophenyl)-6,8-dichtoroimidazo[1,2-a]pyridin-3-yl]methanol

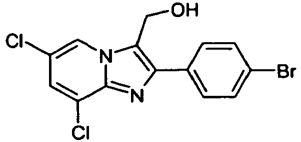

A mixture of 2-amino-3,5-dichloropyridine (0.33 g, 2.0 mmol) and 2,4'-dibromoacetophenone (0.28 g, 1.0 mmol) in ethanol (5 mL) was heated at 65 °C overnight. The mixture was purified by preparative HPLC (Xterra C18, 50 mM NH₄HCO₃ (pH 10) - CH₃CN) to give the intermediate 2-(4-bromophenyl)-6,8-dichloroimidazo[1,2-a]pyridine as a white solid (70 mg).

To the intermediate (60 mg, 0.18 mmol) in acetic acid (5 mL) was added 12.3 M formaldehyde in water, (0.12 mL, 1.40 mmol) and sodium acetate (72 mg, 0.88 mmol). The mixture was heated at 45 °C for 5 h and then allowed to cool to rt. 1 M NaOH (50 mL) was added whereby the product precipitated. The precipitate was collected by filtration and recrystallized from methanol to give the title compound as a white solid (49 mg). HRMS (ESI+) calcd for C₁₄H₉BrCl₂N₂O 369.9275, found 369.9275.

### EXAMPLE 43

### 2-(4-Bromophenyl)-3-(hydroxymethyl)imidazo[1,2-a]pyridine-6-carbonitrile

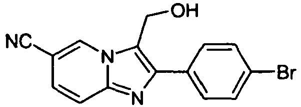

2-Bromo-1-(4-bromophenyl)ethanone (0.56 g, 2.0 mmol) and 6-aminopyridine-3-carbonitrile (0.25 g, 2.1 mmol) were stirred in ethanol (2 mL) at 60 °C overnight. The precipitate that formed was collected by filtration and washed with ethanol to give the intermediate 2-(4-bromophenyl)imidazo[1,2-a]pyridine-6-carbonitrile hydrobromide as a beige solid (507 mg).

This intermediate (0.19 g, 0.50 mmol) was treated with 12.3 M formaldehyde in water (0.32 mL, 4.0 mmol) and sodium acetate (0.20 g, 2.5 mmol) in acetic acid (2 mL) at 50 °C over the weekend. The solvents were evaporated and the residue was crystallized from methanol. The product was then recrystallized from ethyl acetate and finally purified by preparative HPLC (ACE C8, 10 mM NH₄OAc (pH 7) - CH₃CN) to give the title compound as a white solid (8 mg). HRMS (ESI+) calcd for C₁₅H₁₀BrN₃O 327.0007, found 327.0011.

### EXAMPLE 44

### Methyl 2-(4-bromophenyl)-3-(hydroxymethyl)imidazo[1,2-a]pyridine-6-carboxylate

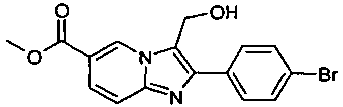

A mixture of 2-bromo-1-(4-bromophenyl)ethanone (0.56 g, 2.0 mmol) and methyl 6-aminopyridine-3-carboxylate (0.32 g, 2.1 mmol) was stirred in ethanol (2 mL) at 60 °C overnight. The precipitate that formed was collected by filtration and washed with ethanol to give the intermediate methyl 2-(4-bromophenyl)imidazo[1,2-a]pyridine-6-carboxylate hydrobromide as a white solid (458 mg).

This intermediate (0.21 g, 0.50 mmol) was treated with 12.3 M formaldehyde in water (0.32 mL, 4.0 mmol) and sodium acetate (0.20 g, 2.5 mmol) in acetic acid (2 mL) at 50 °C over the weekend. The solvents were evaporated and the residue was crystallized from methanol. Recrystallization from ethyl acetate gave the title compound as a white solid (129 mg). HRMS (ESI+) calcd for C₁₆H₁₃BrN₂O₃ 360.0110, found 360.0113.

### EXAMPLE 45

### Methyl 2-(4-chlorophenyl)-3-(hydroxymethyl)imidazo[1,2-a]pyridine-6-carboxylate hydrobromide

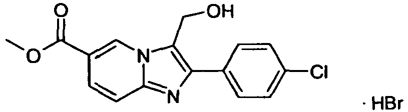

A mixture of methyl 6-aminopyridine-3-carboxylate (0.84 g, 5.5 mmol) and 2-bromo-1-(4-chlorophenyl)ethanone (1.17 g, 5.0 mmol) in ethanol (5 mL) was stirred at 40 °C overnight and then at 50 °C for 24 h. The product was allowed to crystallize upon cooling to -15 °C. Isolation by filtration and drying *in vacuo* gave the intermediate methyl 2-(4-chlorophenyl)imidazo[1,2-a]pyridine-6-carboxylate hydrobromide as white solid (1.53 g). This intermediate (1.47 g, 4.0 mmol) was treated with 12.3 M formaldehyde in water (2.5 mL, 32 mmol) and sodium acetate (1.6 g, 20 mmol) in acetic acid (30 mL) at 50 °C overnight. The solvent was evaporated and the residue was treated with aqueous methanol to give a white solid. Recrystallization from methanol gave material with ∼90% purity. A sample (15 mg) was recrystallized a second time from methanol to give the title compound as a white solid (3 mg). HRMS (ESI+) calcd for C₁₆H₁₃ClN₂O₃ 316.061, found 316.062.

### EXAMPLE 46

### [2-(4-Bromophenyl)imidazo[1,2-a]pyridine-3,7-diyl]dimethanol

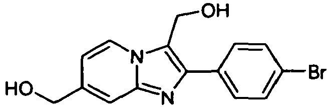

2-Bromo-1-(4-bromophenyl)ethanone (0.56 g, 2.0 mmol) and methyl 2-aminopyridine-4-carboxylate (0.32 g, 2.1 mmol) were stirred in ethanol (2 mL) at 60° C overnight and then at 75° C for 24 h. The precipitate was collected by filtration and washed with ethanol to give the intermediate methyl 2-(4-bromophenyl)imidazo[1,2-a]pyridine-7-carboxylate hydrobromide as a light orange solid (518 mg).

The intermediate (0.21 g, 0.50 mmol) was treated with 12.3 M formaldehyde in water (0.32 mL, 4.0 mmol) and sodium acetate (0.20 g, 2.5 mmol) in acetic acid (2 mL) at 50° C over the weekend. The solvents were evaporated and the solid residue was crystallized from methanol to give methyl 2-(4-bromophenyl)-3-(hydroxymethyl)imidazo[1,2-a]pyridine-7-carboxylate as a beige solid (148 mg).

Part of this material (36 mg, 0.1 mmol) was treated with NaBH₄ (38 mg, 1 mmol) in ethanol (1 mL) at 40 °C overnight. Water (4 mL) was added and the mixture was stirred for 30 min. The product was collected by filtration and washed with water. Trituration in methanol and drying in vacuo gave the title compound as an off-white solid (9 mg). HRMS (ESI+) calcd for C₁₅H₁₃BrN₂O₂ 332.0160, found 332.0171.

### EXAMPLE 47

### [2-(4-Chlorophenyl)imidazo[1,2-a]pyridine-3,6-diyl]dimethanol

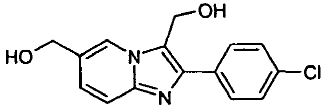

Methyl 2-(4-chlorophenyl)-3-(hydroxymethyl)imidazo[1,2-a]pyridine-6-carboxylate hydrobromide (Example 45; 72 mg, 0.18 mmol) was treated with lithium aluminum hydride (76 mg, 2.0 mmol) in THF (2 mL) at 40 °C for 30 minutes. Water (80 µL), 1 M NaOH (80µL) and more water (240 µL) were added carefully and after 1 h the mixture was filtered. The solid material was washed with ethyl acetate and methanol and the combined filtrates were evaporated to give 80 mg of a semisolid. Part of the material (∼50%) was purified by preparative HPLC (ACE C8, 10 mM NH₄OAc (pH 7) - CH₃CN) to give the title compound as a white solid (2 mg). HRMS (ESI+) calcd for C₁₅H₁₃ClN₂O₂ 288.0666, found 288.0672.

### EXAMPLE 48

### [2-(4-Chlorophenyl)-6-nitroimidazo[1,2-a]pyridin-3-yl]methanol

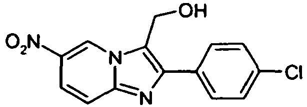

A mixture of 5-nitropyridin-2-amine (765 mg, 5.5 mmol) and 2-bromo-1-(4-chlorophenyl)ethanone (1.17 g, 5.0 mmol) in ethanol (5 mL) was stirred at 40 °C overnight and then at 50 °C for another 24 h. The mixture was cooled in the freezer and the solid material was collected by filtration. Recrystalisation from aqueous ethanol gave the intermediate compound 2-(4-chlorophenyl)-6-nitroimidazo[1,2-a]pyridine hydrobromide as a beige solid (0.61 g).

The intermediate (1.74 g, 4.91 mmol) was treated with 12.3 M formaldehyde in water (3.91 mL, 39.2 mmol) and sodium acetate (2.01 g, 24.5 mmol) in acetic acid (30 mL) at 50 °C overnight. The solvent was evaporated and to the solid residue was 1 M NaOH added. The product was extracted with ethyl acetate. The organic phase was separated, dried (Na₂SO₄), filtered and concentrated. The solid was triturated in methanol and dried in vacuo to give the title compound (1.05 g). HRMS (ESI+) calcd for C₁₄H₁₀ClN₃O₃ 303.0411, found 303.0413.

### EXAMPLE 49

### [2-(4-Bromophenyl)-6-nitroimidazo[1,2-a]pyridin-3-yl]methanol hydrochloride

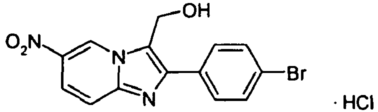

2-Bromo-1-(4-bromophenyl)ethanone (0.56 g, 2.0 mmol) and 5-nitropyridin-2-amine (0.29 g, 2.1 mmol) were stirred in ethanol (2 mL) at 60 °C overnight and then at 75 °C for 24 h. The precipitate was collected by filtration and washed with ethanol to give the intermediate 2-(4-bromophenyl)-6-nitroimidazo[1,2-a]pyridine hydrobromide as a beige solid (416 mg). The intermediate (0.20 g, 0.50 mmol) was treated with 12.3 M formaldehyde in water (0.32 mL, 4.0 mmol) and sodium acetate (0.20 g, 2.5 mmol) in acetic acid (2 mL) at 50 °C over the weekend. The mixture was added to 1 M NaOH (100 mL) and was left with stirring overnight whereby all material had gone into solution. Acidification with conc. HCl and extraction with CH₂Cl₂, drying (Na₂SO₄) and evaporation gave a yellow solid. Crystallization from methanol gave the title compound as a yellow solid (69 mg). HRMS (ESI+) calcd for C₁₄H₁₀BrN₃O₃ 346.9906, found 346.9912.

### INTERMEDIATE 1

### Sodium 2-(4-chlorophenyl)-3-(hydroxymethyl)imidazo[1,2-a]pyridine-6-carboxylate

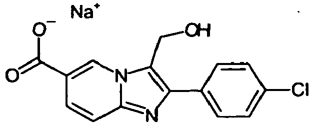

Methyl 2-(4-chlorophenyl)-3-(hydroxymethyl)imidazo[1,2-a]pyridine-6-carboxylate hydrobromide (Example 45; 0.63 g, 2.0 mmol) was treated with 1 M NaOH (10 mL, 10 mmol) in methanol (10 mL) at 50 °C overnight. The methanol was slowly allowed to evaporate and the resulting crystals were collected by filtration, washed with water and dried *in vacuo* to give the title compound as a white solid (0.58 g). MS (ESI+) m/z 303 [M+H]+.

### EXAMPLE 50

### {2-(4-Chlorophenyl)-6-[(4-methoxypiperidin-1-yl)carbonyl]imidazo[1,2-a]pyridin-3-yl}methanol

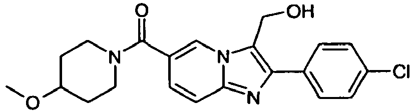

Diisopropylethylamine (52 µL, 0.3 mmol) and 1-propanephosphonic acid cyclic anhydride (50% in ethyl acetate, 90 µL, 0.15 mmol) were added to a solution of sodium 2-(4-chlorophenyl)-3-(hydroxymethyl)imidazo[1,2-a]pyridine-6-carboxylate (Intermediate 1; 10 mg, 0.03 mmol) in DMF (0.3 mL). 4-Methoxypiperidine (7 mg, 0.06 mmol) was added and the reaction mixture was left at rt overnight. Water (0.2 mL) and methanol (0.5 mL) were added and the mixture was purified by preparative HPLC (Xterra C18, 50 mM NH₄HCO₃ (pH 10) - CH₃CN) to give the title compound as a white solid (9 mg). HRMS (ESI+) calcd for C₂₁H₂₂ClN₃O₃ 399.1350, found 399.1356.

### EXAMPLE 51

### 2-(4-Chlorophenyl)-3-(hydroxymethyl)-N-(3-methoxypropyl)imidazo[1,2-a]pyridine-6-carboxamide

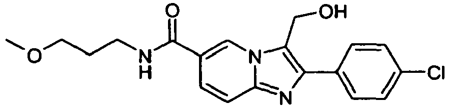

Diisopropylethylamine (52 µL, 0.3 mmol) and 1-propanephosphonic acid cyclic anhydride (50% in ethyl acetate, 90 µL, 0.15 mmol) were added to a solution of sodium 2-(4-chlorophenyl)-3-(hydroxymethyl)imidazo[1,2-a]pyridine-6-carboxylate (Intermediate 1; 10 mg, 0.03 mmol) in DMF (0.3 mL). 3-Methoxypropan-1-amine (6 µL, 0.06 mmol) was added and the reaction mixture was left at rt overnight. Water (0.2 mL) and methanol (0.5 mL) were added and the mixture was purified by preparative HPLC (Xterra C18, 50 mM NH₄HCO₃ (pH 10) - CH₃CN) to give the title compound as a white solid (4 mg). HRMS (ESI+) calcd for C₁₉H₂₀ClN₃O₃ 373.1193, found 373.1198.

### EXAMPLE 52

### 2-(4-Chlorophenyl)-3-(hydroxymethyl)-N-(2-methoxyethyl)imidazo[1,2-a]pyridine-6-carboxamide

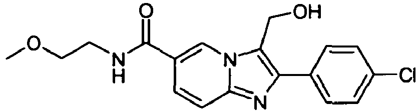

TBTU (12 mg, 0.036 mmol), diisopropylethylamine (16 µL, 0.09 mmol) and 2-methoxyethylamine (5 µL, 0.06 mmol) were added to a solution of sodium 2-(4-chlorophenyl)-3-(hydroxymethyl)imidazo[1,2-a]pyridine-6-carboxylate (Intermediate 1; 10 mg, 0.03 mmol) in DMF (0.3 mL). The mixture was stirred at rt overnight. The reaction was quenched with water and methanol, filtered and purified by preparative HPLC (Xterra C18, 50 mM NH₄HCO₃ (pH 10) - CH₃CN) to give the title compound as a white solid (5 mg). HRMS (ESI+) calcd for C₁₈H₁₈ClN₃O₃ 359.1037, found 359.1042.

### EXAMPLE 53

### [2-(4-Chlorophenyl)-6-(morpholin-4-ylcarbonyl)imidazo[1,2-a]pyridin-3-yl]methanol

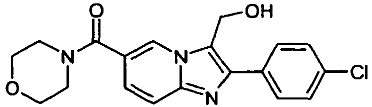

Diisopropylethylamine (52 µL, 0.3 mmol) and 1-propanephosphonic acid cyclic anhydride (50% in ethyl acetate, 90 µL, 0.15 mmol) were added to a solution of sodium 2-(4-chlorophenyl)-3-(hydroxymethyl)imidazo[1,2-a]pyridine-6-carboxylate (Intermediate 1; 10 mg, 0.03 mmol) in DMF (0.3 mL). Morpholine (5 mg, 0.06 mmol) was added and the reaction mixture was left at rt overnight. Water (0.2 mL) and methanol (0.5 mL) were added and the mixture was purified by preparative HPLC (Xterra C18, 50 mM NH₄HCO₃ (pH 10) - CH₃CN) to give the title compound as a white solid (9 mg). HRMS (ESI+) calcd for C₁₉H₁₈ClN₃O₃ 371.1037, found 371.1041.

### EXAMPLE 54

### 2-(4-Chlorophenyl)-3-(hydroxymethyl)-N,N-dimethylimidazo[1,2-a]pyridine-6-carboxamide

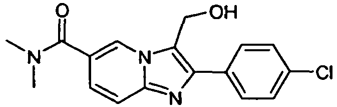

1-Propanephosphonic acid cyclic anhydride (50% in ethyl acetate, 18 µL, 0.12 mmol) was added to a solution of sodium 2-(4-chlorophenyl)-3-(hydroxymethyl)imidazo[1,2-a]pyridine-6-carboxylate (Intermediate 1; 10 mg, 0.03 mmol) in DMF (0.3 mL). After 10 minutes diisopropylethylamine (26µL, 0.15 mmol) and dimethylamine (0.1 mL, 0.6 mmol, 5.6 M in ethanol) were added. After 30 minutes the reaction was quenched by addition of water (1 mL) and the crude product was purified by preparative HPLC (ACE C8, water containing 0.1 % TFA - CH₃CN) to give the title compound as a white solid (6 mg). HRMS (ESI+) calcd for C₁₇H₁₆ClN₃O₂ 329.0931, found 329.0939.

### EXAMPLE 55

### 2-(4-Chlorophenyl)-3-(hydroxymethyl)-N-methylimidazo[1,2-a]pyridine-6-carboxamide

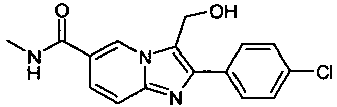

1-Propanephosphonic acid cyclic anhydride (50% in ethyl acetate, 18 µL, 0.12 mmol), diisopropylethylamine (26 µL, 0.15 mmol) and methylamine (30% in methanol, 8 µL, 0.06 mmol) were added to a solution of sodium 2-(4-chlorophenyl)-3-(hydroxymethyl)imidazo[1,2-a]pyridine-6-carboxylate (Intermediate 1; 10 mg, 0.03 mmol) in DMF (0.3 mL). After 30 min the reaction was quenched with water (0.5 mL) to give a clear solution. A precipitate then formed, which after an hour was collected and purified by preparative HPLC (Xterra C18, 50 mM NH₄HCO₃ (pH 10) - CH₃CN) to give the title compound as a white solid (4 mg). HRMS (ESI+) calcd for C₁₆H₁₄ClN₃O₂ 315.0775, found 315.0775.

### EXAMPLE 56

### 2-(4-Chlorophenyl)-3-(hydroxymethyl)-N-[2-(1-methylpyrrolidin-2-yl)ethyl]imidazo[1,2-a]pyridine-6-carboxamide

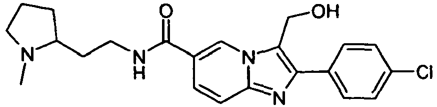

Diisopropylethylamine (52 µL, 0.3 mmol) and 1-propanephosphonic acid cyclic anhydride (50% in ethyl acetate, 90 µL, 0.15 mmol) were added to a solution of sodium 2-(4-chlorophenyl)-3-(hydroxymethyl)imidazo[1,2-a]pyridine-6-carboxylate (Intermediate 1; 10 mg, 0.03 mmol) in DMF (0.3 mL). 2-(1-Methylpyrrolidin-2-yl)ethanamine (9 µL, 0.06 mmol) was added and the reaction mixture was left at rt overnight. Water (0.2 mL) and methanol (0.5 mL) were added and the mixture was purified by preparative HPLC (Xterra C18, 50 mM NH₄HCO₃ (pH 10) - CH₃CN) to give the title compound as a white solid (6 mg). HRMS (ESI+) calcd for C₂₂H₂₅ClN₄O₂ 412.1666, found 412.1671.

### EXAMPLE 57

### {2-(4-Chlorophenyl)-6-[(4-methylpiperazin-1-yl)carbonyl]imidazo[1,2-a]pyridin-3-yl}methanol

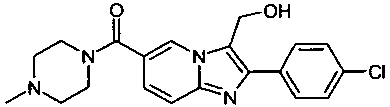

Diisopropylethylamine (52 µL, 0.3 mmol) and 1-propanephosphonic acid cyclic anhydride (50% in ethyl acetate, 90 µL, 0.15 mmol) were added to a solution of sodium 2-(4-chlorophenyl)-3-(hydroxymethyl)imidazo[1,2-a]pyridine-6-carboxylate (Intermediate 1; 10 mg, 0.03 mmol) in DMF (0.3 mL). 1-Methylpiperazine (7 µL, 0.06 mmol) was added and the reaction mixture was left at rt overnight. Water (0.2 mL) and methanol (0.5 mL) were added and the mixture was purified by preparative HPLC (Xterra C18, 50 mM NH₄HCO₃ (pH 10) - CH₃CN) to give the title compound as a white solid (10 mg). HRMS (ESI+) calcd for C₂₀H₂₁ClN₄O₂ 384.1353, found 384.1358.

### EXAMPLE 58

### 2-(4-Chlorophenyl)-N-(3,4-dimethoxybenzyl)-3-(hydroxymethyl)imidazo[1,2-a]pyridine-6-carboxamide

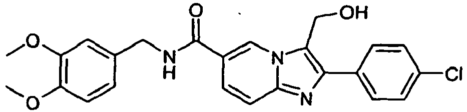

Diisopropylethylamine (52 µL, 0.3 mmol) and 1-propanephosphonic acid cyclic anhydride (50% in ethyl acetate, 90 µL, 0.15 mmol) were added to a solution of sodium 2-(4-chlorophenyl)-3-(hydroxymethyl)imidazo[1,2-a]pyridine-6-carboxylate (Intermediate 1; 10 mg, 0.03 mmol) in DMF (0.3 mL). 1-(3,4-Dimethoxyphenyl)methanamine (9 µL, 0.06 mmol) was added and the reaction mixture was stirred at rt overnight. Water (0.2 mL) and methanol (0.5 mL) were added and the mixture was purified by preparative HPLC (Xterra C18, 50 mM NH₄HCO₃ (pH 10) - CH₃CN) to give the title compound as a white solid (9 mg). HRMS (ESI+) calcd for C₂₄H₂₂ClN₃O₄ 451.1299, found 451.1302.

### EXAMPLE 59

### 2-(4-Chlorophenyl)-3-(hydroxymethyl)-N-[2-(1H-imidazol-4-yl)ethyl]imidazo[1,2-a]pyridine-6-carboxamide

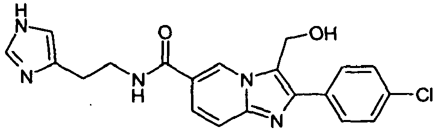

Diisopropylethylamine (52 µL, 0.3 mmol) and 1-propanephosphonic acid cyclic anhydride (50% in ethyl acetate, 90 µL, 0.15 mmol) were added to a solution of sodium 2-(4-chlorophenyl)-3-(hydroxymethyl)imidazo[1,2-a]pyridine-6-carboxylate (Intermediate 1; 10 mg, 0.03 mmol) in DMF (0.3 mL). 2-(1H-Imidazol-4-yl)ethanamine (7 mg, 0.06 mmol) was added and the reaction mixture was left at rt overnight. Water (0.2 mL) and methanol (0.5 mL) were added and the mixture was purified by preparative HPLC (Xterra C18, 50 mM NH₄HCO₃ (pH 10) - CH₃CN) to give the title compound as a white solid (4 mg). HRMS (ESI+) calcd for C₂₀H₁₈ClN₅O₂ 395.1149, found 395.1149.

### EXAMPLE 60

### 2-(4-Chlorophenyl)-3-(hydroxymethyl)-N-(pyridin-3-ylmethyl)imidazo[1,2-a]pyridine-6-carboxamide

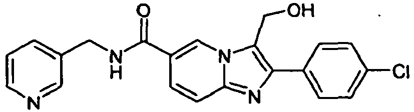

Diisopropylethylamine (52 µL, 0.3 mmol) and 1-propanephosphonic acid cyclic anhydride (50% in ethyl acetate, 90 µL, 0.15 mmol) were added to a solution of sodium 2-(4-chlorophenyl)-3-(hydroxymethyl)imidazo[1,2-a]pyridine-6-carboxylate (Intermediate 1; 10 mg, 0.03 mmol) in DMF (0.3 mL). 1-(Pyridin-3-yl)methanamine (6 µL, 0.06 mmol) was added and the reaction mixture was left at rt overnight. Water (0.2 mL) and methanol (0.5 mL) were added and the mixture was purified by preparative HPLC (Xterra C18, 50 mM NH₄HCO₃ (pH 10) - CH₃CN) to give the title compound as a white solid (9 mg). HRMS (ESI+) calcd for C₂₁H₁₇ClN₄O₂ 392.1040, found 392.1042.

### EXAMPLE 61

### 2-(4-Chlorophenyl)-3-(hydroxymethyl)-N-(3-hydroxypropyl)imidazo[1,2-a]pyridine-6-carboxamide

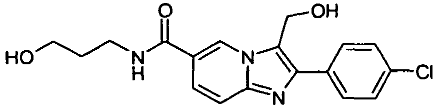

Diisopropylethylamine (52 µL, 0.3 mmol) and 1-propanephosphonic acid cyclic anhydride (50% in ethyl acetate, 90 µL, 0.15 mmol) were added to a solution of sodium 2-(4-chlorophenyl)-3-(hydroxymethyl)imidazo[1,2-a]pyridine-6-carboxylate (Intermediate 1; 10 mg, 0.03 mmol) in DMF (0.3 mL). 3-Aminopropan-1-ol (5 µL, 0.06 mmol) was added and the reaction mixture was left at rt overnight. Water (0.2 mL) and methanol (0.5 mL) were added and the mixture was purified by preparative HPLC (Xterra C18, 50 mM NH₄HCO₃ (pH 10) - CH₃CN) to give the title compound as a white solid (6 mg). HRMS (ESI+) calcd for C₁₈H₁₈ClN₃O₃ 359.1037, found 359.1038.

### EXAMPLE 62

### (1-{[2-(4-Chlorophenyl)-3-(hydroxymethyl)imidazo[1,2-alpyridin-6-yl]carbonyl}piperidin-4-yl)methanol

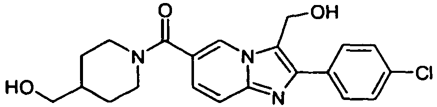

Diisopropylethylamine (52 µL, 0.3 mmol) and 1-propanephosphonic acid cyclic anhydride (50% in ethyl acetate, 90 µL, 0.15 mmol) were added to a solution of sodium 2-(4-chlorophenyl)-3-(hydroxymethyl)imidazo[1,2-a]pyridine-6-carboxylate (Intermediate 1; 10 mg, 0.03 mmol) in DMF (0.3 mL). Piperidin-4-ylmethanol (7 mg, 0.06 mmol) was added and the reaction mixture was stirred at rt overnight. Water(0.2 mL) and methanol (0.5 mL) were added and the mixture was purified by preparative HPLC (Xterra C18, 50 mM NH₄HCO₃ (pH 10) - CH₃CN) to give the title compound as a colourless liquid (7 mg). HRMS (ESI+) calcd for C₂₁H₂₂ClN₃O₃ 399.1350, found 399.1352.

### EXAMPLE 63

### 2-(4-Chlorophenyl)-3-(hydroxymethyl)-N-(2-hydroxypropyl)imidazo[1,2-a]pyridine-6-carboxamide

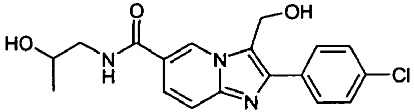

Diisopropylethylamine (52 µL, 0.3 mmol) and 1-propanephosphonic acid cyclic anhydride (50% in ethyl acetate, 90 µL, 0.15 mmol) were added to a solution of sodium 2-(4-chlorophenyl)-3-(hydroxymethyl)imidazo[1,2-a]pyridine-6-carboxylate (Intermediate 1; 10 mg, 0.03 mmol) in DMF (0.3 mL). 1-Aminopropan-2-ol (5 µL, 0.06 mmol) was added and the reaction mixture was left at rt overnight. Water (0.2 mL) and methanol (0.5 mL) were added and the mixture was purified by preparative HPLC (Xterra C18, 50 mM NH₄HCO₃ (pH 10) - CH₃CN) to give the title compound as a white solid (6 mg). HRMS (ESI+) calcd for C₁₈H₁₈ClN₃O₃ 359.1037, found 359.1039.

### EXAMPLE 64

### 2-(4-Chlorophenyl)-N-(trans-4-hydroxycyclohexyl)-3-(hydroxymethyl)imidazo[1,2-a]pyridine-6-carboxamide

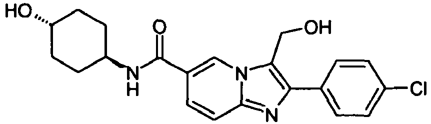

Diisopropylethylamine (52 µL, 0.3 mmol) and 1-propanephosphonic acid cyclic anhydride (50% in ethyl acetate, 90 µL, 0.15 mmol) were added to a solution of sodium 2-(4-chlorophenyl)-3-(hydroxymethyl)imidazo[1,2-a]pyridine-6-carboxylate (Intermediate 1; 10 mg, 0.03 mmol) in DMF (0.3 mL). *trans*-4-Aminocyclohexanol (7 mg, 0.06 mmol) was added and the reaction mixture was left at rt overnight. Water (0.2 mL) and methanol (0.5 mL) were added and the mixture was purified by preparative HPLC (Xterra C18, 50 mM NH₄HCO₃ (pH 10) - CH₃CN) to give the title compound as a white solid (7 mg). HRMS (ESI⁺) calcd for C₂₁H₂₂ClN₃O₃ 399.1350, found 399.1350.

### EXAMPLE 65

### 1-{[2-(4-Chlorophenyl)-3-(hydroxymethyl)imidazo[1,2-a]pyridin-6-yl]carbonyl}piperidin-4-ol

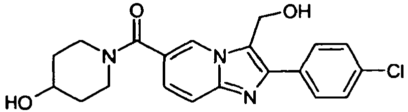

Diisopropylethylamine (52 µL, 0.3 mmol) and 1-propanephosphonic acid cyclic anhydride (50% in ethyl acetate, 90 µL, 0.15 mmol) were added to a solution of sodium 2-(4-chlorophenyl)-3-(hydroxymethyl)imidazo[1,2-a]pyridine-6-carboxylate (Intermediate 1; 10 mg, 0.03 mmol) in DMF (0.3 mL). Piperidin-4-ol (6 mg, 0.06 mmol) was added and the reaction mixture was stirred at rt overnight. Water (0.2 mL) and methanol (0.5 mL) were added and the mixture was purified by preparative HPLC (Xterra C18, 50 mM NH₄HCO₃ (pH 10) - CH₃CN) to give the title compound as a white solid (6 mg). HRMS (ESI+) calcd for C₂₀H₂₀ClN₃O₃ 385.1193, found 385.1194.

### EXAMPLE 66

### (3R)-1-{[2-(4-Chlorophenyl)-3-(hydroxymethyl)imidazo[1,2-a]pyridin-6-yl]carbonyl}pyrrolidin-3-ol

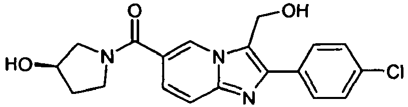

Diisopropylethylamine (52 µL, 0.3 mmol) and 1-propanephosphonic acid cyclic anhydride (50% in ethyl acetate, 90 µL, 0.15 mmol) were added to a solution of sodium 2-(4-chlorophenyl)-3-(hydroxymethyl)imidazo[1,2-a]pyridine-6-carboxylate (Intermediate 1; 10 mg, 0.03 mmol) in DMF (0.3 mL). (3R)-Pyrrolidin-3-ol hydrochloride (7 mg, 0.06 mmol) was added and the reaction mixture was left at rt overnight. Water (0.2 mL) and methanol (0.5 mL) were added and the mixture was purified by preparative HPLC (Xterra C18, 50 mM NH₄HCO₃ (pH 10) - CH₃CN) to give the title compound as a white solid (7 mg). HRMS (ESI+) calcd for C₁₉H₁₈ClN₃O₃ 371.1037, found 371.1042.

### EXAMPLE 67

### 1-{[2-(4-Chlorophenyl)-3-(hydroxymethyl)imidazo[1,2-a]pyridin-6-yl]carbonyl}pyrrolidin-3-ol

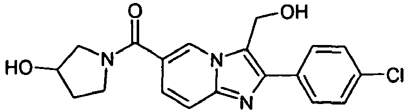

Diisopropylethylamine (52 µL, 0.3 mmol) and 1-propanephosphonic acid cyclic anhydride (50% in ethyl acetate, 90 µL, 0.15 mmol) were added to a solution of sodium 2-(4-chlorophenyl)-3-(hydroxymethyl)imidazo[1,2-a]pyridine-6-carboxylate (Intermediate 1; 10 mg, 0.03 mmol) in DMF (0.3 mL). Pyrrolidin-3-ol (5 µL, 0.06 mmol) was added and the reaction mixture was stirred at rt overnight. Water (0.2 mL) and methanol (0.5 mL) were added and the mixture was purified by preparative HPLC (Xterra C18, 50 mM NH₄HCO₃ (pH 10) - CH₃CN) to give the title compound as a white solid (6 mg). HRMS (ESI+) calcd for C₁₉H₁₈ClN₃O₃ 371.1037, found 371.1038.

### EXAMPLE 68

### 1-{[2-(4-Chlorophenyl)-3-(hydroxymethyl)imidazo[1,2-a]pyridin-6-yl]carbonyl}azetidin-3-ol

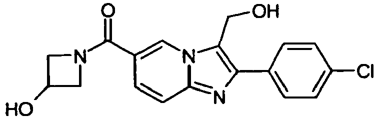

Diisopropylethylamine (52 µL, 0.3 mmol) and 1-propanephosphonic acid cyclic anhydride (50% in ethyl acetate, 90 µL, 0.15 mmol) were added to a solution of sodium 2-(4-chlorophenyl)-3-(hydroxymethyl)imidazo[1,2-a]pyridine-6-carboxylate (Intermediate 1; 10 mg, 0.03 mmol) in DMF (0.3 mL). Azetidin-3-ol hydrochloride (7 mg, 0.06 mmol) was added and the reaction mixture was left at rt overnight. Water (0.2 mL) and methanol (0.5 mL) were added and the mixture was purified by preparative HPLC (Xterra C18, 50 mM NH₄HCO₃ (pH 10) - CH₃CN) to give the title compound as a white solid (6 mg). HRMS (ESI+) calcd for C₁₈H₁₆ClN₃O₃ 357.0880, found 357.0880.

### INTERMEDIATE 2

### 2-(4-Chlorophenyl)-3-(hydroxymethyl)imidazo[1,2-a]pyridine-7-carboxylic acid hydrochloride

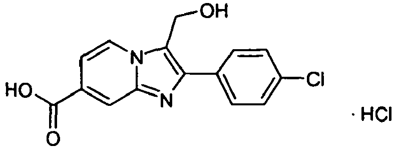

A mixture ofmethyl-2-aminopyridine-4-carboxylate (0.80 g, 5.5 mmol) and 2-bromo-1-(4-chlorophenyl)ethanone (1.17 g, 5.0 mmol) in ethanol (10 mL) was stirred at 40 °C for 5 days. The solid was collected by filtration, washed with ethanol and dried *in vacuo* to give the intermediate compound methyl 2-(4-chlorophenyl)imidazo[1,2-a]pyridine-7-carboxylate hydrobromide as an orange solid (1.1 g).

The intermediate (0.92 g, 2.5 mmol) was treated with 12.3 M formaldehyde in water (1.6 mL, 20 mmol) and sodium acetate (1.0 g, 20 mmol) in acetic acid (20 mL) at 50 °C overnight. Water (50 mL) was added and the mixture was evaporated to almost dryness. More water (50 mL) and aqueous HCl (4 M, 10 mL) were added and the mixture was evaporated to dryness and dried *in vacuo.* The residue was partitioned between ethyl acetate and 0.5 M NaOH solution. The organic phase was dried (Na₂SO₄) and partly evaporated whereupon crystals precipitated. The crystals were collected by filtration and dried *in vacuo* to give methyl 2-(4-chlorophenyl)-3-(hydroxymethyl)imidazo[1,2-a]pyridine-7-carboxylate as a beige solid (0.52 g).

Part of this material (0.32 g, 1.0 mmol) was treated with 1 M NaOH solution (5 mL, 5 mmol) in methanol (5 mL) at 50 °C overnight. The methanol was slowly allowed to evaporate. Water (∼50 mL) was added and the mixture was acidified by aqueous HCl. The precipitate that formed was collected by filtration, washed with water and dried *in vacuo* to give the title compound as a cream coloured solid (0.20 g). MS (ESI+) m/z 303 [M+H]+.

### EXAMPLE 69

### 2-(4-Chlorophenyl)-3-(hydroxymethyl)imidazo[1,2-a]pyridine-7-carboxamide

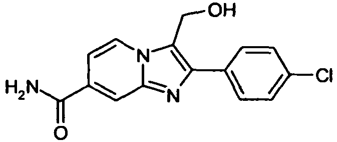

TBTU (14 mg), diisopropylethylamine (16 µL, 0.09 mmol) and ammmonia (2 M in methanol, 30 µL, 0.06 mmol) were added to a solution of 2-(4-chlorophenyl)-3-(hydroxymethyl)imidazo[1,2-a]pyridine-7-carboxylic acid hydrochloride (Intermediate 2; 10 mg, 0.03 mmol) in DMF (0.3 mL) and stirred at rt overnight. The reaction was quenched with water (0.1 mL) and methanol (0.3 mL), filtered and purified by preparative HPLC (Xterra C 18, 50 mM NH₄HCO₃ (pH 10) - CH₃CN) to give the title compound as a white solid (4 mg). HRMS (ESI+) calcd for C₁₅H₁₂ClN₃O₂ 301.0618, found 301.0619.

### EXAMPLE 70

### 3-(Hydroxymethyl)-2-(3-methoxyphenyl)imidazo(1,2-a]pyridine-6-carboxamide

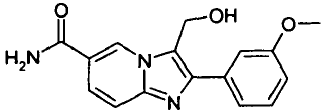

6-Aminonicotinamide (41 mg, 0.30 mmol) and 2-bromo-3'-methoxyacetophenone (46 mg, 0.20 mmol) were mixed in ethanol (0.5 mL) and stirred at rt over the weekend. The mixture was then heated at 70 °C for 2 h. 0.5 M NaOH (1.2 mL) was added slowly with stirring. The precipitate that formed was collected by centrifugation and washed with water (1.5 mL) to give the intermediate 2-(3-methoxyphenyl)imidazo[1,2-a]pyridine-6-carboxamide. MS (ESI+) m/z 268 [M+H]+.

To the intermediate was added sodium acetate (82 mg, 1.0 mmol) followed by 12.3 M formaldehyde in water (0.16 mL, 2.0 mmol), acetic acid (0.4 mL) and acetonitrile (0.5 mL). The reaction mixture was heated at 50 °C for 3 h and then concentrated. Part of the material (∼50%) was purified by preparative HPLC (Xterra C18, 50 mM NH₄HCO₃ (pH 10) - CH₃CN). The material was dissolved in methanol (1 mL) and treated with 1 M NaOH (1 mL) for 1 h at rt. The mixture was extracted with CH₂Cl₂ (2 mL) and the organic phase was washed with water and concentrated. The residue was dried *in vacuo* to give the title compound as a white solid (6.0 mg). HRMS (ESI+) calcd for C₁₆H₁₅N₃O₃ 297.1113, found 297.1120.

### EXAMPLE 71

### 2-(4-Chlorophenyl)-3-(hydroxymethyl)imidazo[1,2-a]pyridine-6-carboxamide

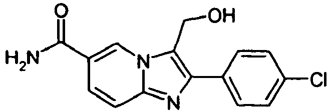

6-Aminonicotinamide (41 mg, 0.30 mmol) and 4-chlorophenacyl bromide (47 mg, 0.20 mmol) were mixed in ethanol (0.5 mL) and stirred at rt over the weekend. The mixture was then heated at 70 °C overnight. 0.5 M NaOH (1.2 mL) was added slowly with stirring. The precipitate that formed was collected by centrifugation and washed with water (1.5 mL) to give the intermediate compound 2-(4-chlorophenyl)imidazo[1,2-a]pyidine-6-carboxamide. MS (ESI+) m/z 272, 274 [M+H]+.

To the intermediate was added sodium acetate (82 mg, 1.0 mmol) followed by 12.3 M formaldehyde in water (0.16 mL, 2.0 mmol), acetic acid (0.4 mL) and acetonitrile (0.5 mL). The reaction mixture was heated at 50 °C for 3 h. 4 M NaOH (1.8 mL) was added slowly with stirring and the mixture was extracted with CH₂Cl₂ (2 x 2 mL). The organic phase was washed with brine (2 mL) and concentrated. Part of the material (∼50%) was purified by preparative HPLC (Xterra C18, 50 mM NH₄HCO₃ (pH 10) - CH₃CN) to give the title compound as a white solid (5.3 mg). HRMS (ESI+) calcd for C₁₅H₁₂ClN₃O₂ 301.0618, found 301.0626.

### EXAMPLE 72

### 2-(4-Fluorophenyl)-3-(hydroxymethyl)imidazo[1,2-a]pyridine-6-carboxamide

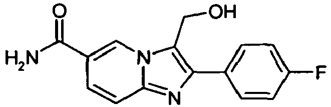

6-Aminonicotinamide (41 mg, 0.30 mmol) and 4-fluorophenacyl bromide (43 mg, 0.20 mmol) were mixed in ethanol (0.5 mL) and stirred at rt over the weekend. The mixture was then heated at 70 °C overnight. 0.5 M NaOH (1.2 mL) was added slowly with stirring. The precipitate that formed was collected by centrifugation and washed with water (1.5 mL) to give the intermediate compound 2-(4-fluorophenyl)imidazo[1,2-a]pyridine-6-carboxamide. MS (ESI+) m/z 256 [M+H]+.

To the intermediate was added sodium acetate (82 mg, 1.0 mmol) followed by 12.3 M formaldehyde in water (0.20 mL, 2.4 mmol), acetic acid (0.4 mL) and acetonitrile (0.5 mL). The reaction mixture was heated at 50 °C for 2 h and then concentrated. Part of the material (∼50%) was purified by preparative HPLC (Xterra C18, 50 mM NH₄HCO₃ (pH 10) - CH₃CN) to give the title compound as a white solid (2.4 mg). HRMS (ESI+) calcd for C₁₅H₁₂FN₃O₂ 285.0914, found 285.0918.

### EXAMPLE 73

### 2-(2,4-Difluorophenyl)-3-(hydroxymethyl)imidazo[1,2-a]pyridine-6-carboxamide

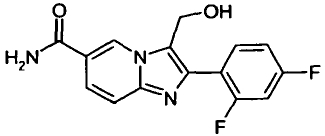

6-Aminonicotinamide (41 mg, 0.30 mmol) and 2,4-difluorophenacyl bromide (47 mg, 0.20 mmol) were mixed in ethanol (0.5 mL) and stirred at rt over the weekend. The mixture was then heated at 70 °C overnight. 0.5 M NaOH (1.2 mL) was added slowly followed by CH₂Cl₂ (2 mL). The aqueous phase was collected, concentrated and dried *in vacuo* to give the intermediate compound 2-(2,4-difluorophenyl)imidazo[1,2-a]pyridine-6-carboxamide. MS (ESI+) m/z 274 [M+H]+.

To the intermediate was added sodium acetate (82 mg, 1.0 mmol) followed by 12.3 M formaldehyde in water (0.20 mL, 2.4 mmol), acetic acid (0.4 mL) and acetonitrile (0.5 mL). The reaction mixture was heated at 50 °C for 10 h and then concentrated. Part of the material (∼50%) was purified by preparative HPLC (Xterra C18, 50 mM NH₄HCO₃ (pH 10) - CH₃CN) to give the title compound as a white solid (7.2 mg). HRMS (ESI+) calcd for C₁₅H₁₁F₂N₃O₂ 303.0819, found 303.0829.

### EXAMPLE 74

### 2-(2,4-Dichlorophenyl)-3-(hydroxymethyl)imidazo[1,2-a]pyridine-6-carboxamide

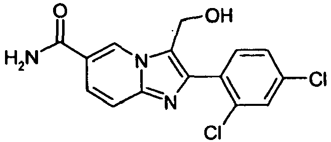

6-Aminonicotinamide (41 mg, 0.30 mmol) and 2,4-dichlorophenacyl chloride (45 mg, 0.20 mmol) were mixed in ethanol (0.5 mL) and stirred at rt over the weekend. The mixture was then heated at 70 °C for 2 days. 0.5 M NaOH (1.2 mL) was added slowly with stirring. The precipitate that formed was collected by centrifugation and washed with water (1.5 mL) to give the intermediate compound 2-(2,4-dichlorophenyl)imidazo[1,2-a]pyridine-6-carboxamide. MS (ESI+) m/z 306, 308 [M+H]+.

To the intermediate was added sodium acetate (82 mg, 1.0 mmol) followed by 12.3 M formaldehyde in water (0.16 mL, 2.0 mmol), acetic acid (0.4 mL) and acetonitrile (0.5 mL). The reaction mixture was heated at 50 °C overnight and was then concentrated. Part of the material (∼50%) was purified by preparative HPLC (Xterra C18, 50 mM NH₄HCO₃ (pH 10) - CH₃CN). This material was dissolved in methanol (1 mL) and treated with 1 M NaOH (1 mL) for 1 h at rt. The mixture was extracted with CH₂Cl₂ (2 mL) and the organic phase was washed with water and concentrated. The residue was dried in vacuo to give the title compound as a white solid (0.7 mg). HRMS (ESI+) calcd for C₁₅H₁₁Cl₂N₃O₂ 335.0228, found 335.0228.

### EXAMPLE 75

### 2-(3,4-Difluorophenyl)-3-(hydroxymethyl)imidazo[1,2-a]pyridine-6-carboxamide

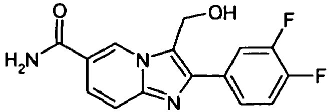

6-Aminonicotinamide (41 mg, 0.30 mmol) and 2-bromo-3',4'-difluoroacetophenone (47 mg, 0.20 mmol) were mixed in ethanol (0.5 mL) and stirred at rt over the weekend. The mixture was then heated at 70 °C overnight. 0.5 M NaOH (1.2 mL) was added slowly with stirring. The precipitate that formed was collected by centrifugation and washed with water (1.5 mL) to give the intermediate compound 2-(3,4-difluorophenyl)imidazo[1,2-a]pyridine-6-carboxamide. MS (ESI+) m/z 274 [M+H]+.

To the intermediate was added sodium acetate (82 mg, 1.0 mmol) followed by 12.3 M formaldehyde in water (0.20 mL, 2.4 mmol), acetic acid (0.4 mL) and acetonitrile (0.5 mL). The reaction mixture was heated at 50 °C for 6 h and then concentrated. Part of the material (∼50%) was purified by preparative HPLC (Xterra C18, 50 mM NH₄HCO₃ (pH 10) - CH₃CN) to give the title compound as a white solid (2.3 mg). HRMS (ESI+) calcd for C₁₅H₁₁F₂N₃O₂ 303.0819, found 303.0827.

### EXAMPLE 76

### [6-amino-2-(4-chlorophenyl)imidazo[1,2-a]pyridin-3-yl]methanol

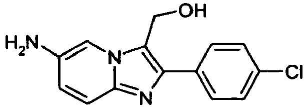

[2-(4-Chlorophenyl)-6-nitroimidazo[1,2-a]pyridin-3-yl]methanol (Example 48; 0.20 g, 0.66 mmol) was dissolved in THF (4 mL) and DMF (1 mL) and treated with Raney 2800 Nickel, (slurry in water, active catalyst; 100 mg) overnight. The mixture was filtered through a layer of Celite and the filtrate was concentrated. The residue was purified by preparative HPLC (Xterra C18, 50 mM NH₄HCO₃ (pH 10) - CH₃CN) to give the title compound as a white solid (180 mg). HRMS (ESI+) calcd for C₁₄H₁₂ClN₃O 273.0669, found 273.0674.

### EXAMPLE 77

### N-[2-(4-chlorophenyl)-3-(hydroxymethyl)imidazo[1,2-a]pyridin-6-yl]acetamide

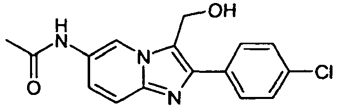

[6-Amino-2-(4-chlorophenyl)imidazo[1,2-a]pyridin-3-yl]methanol (Example 76; 0.50 g, 0.18 mmol) was dissolved in THF (2 mL) and treated with acetic anhydride (20 µL, 0.20 mmol) for 2 h. The mixture was purified by preparative HPLC (Xterra C18, 50 mM NH₄HCO₃ (pH 10) - CH₃CN) to give the title compound (8 mg). HRMS (ESI+) calcd for C₁₆H₁₄ClN₃O₂ 315.0774, found 315.0779.

### INTERMEDIATE 3

### tert-Butyl (6-aminopyridin-3-yl)carbamate

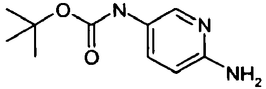

2,5-Diaminopyridine (0.546 g, 5.00 mmol) was suspended in chloroform (20 mL) and treated with di-tert-butyl dicarbonate (1.09 g, 5.00 mmol) at rt for 4 h. To the reaction was added *N,N-*dimethylethylenediamine (5 mmol) in order to trap remaining di-*tert*-butyl dicarbonate and the mixture was filtered through a bed of SiO₂. The product was eluted with 5% methanol in chloroform (250 mL). The solvent was evaporated and the residue was purified by preparative HPLC (Xterra C18, 50 mM NH₄HCO₃ (pH 10) - CH₃CN) to give the title compound as a solid (262 mg).

### EXAMPLE 78

### [6-amino-2-(4-bromophenyl)imidazo[1,2-a]pyridin-3-yl]methanol

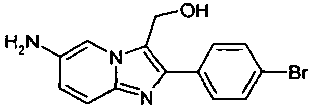

*tert*-Butyl (6-aminopyridin-3-yl)carbamate (Intermediate 3; 127 mg, 0.60 mmol) and 2,4-dibromoacetophenone (169 mg, 0.47 mmol) were dissolved in ethanol (2 mL) and the mixture was stirred at 60 °C for 5 h. The solvent was evaporated and the crude residue was purified by preparative HPLC (Xterra C18, 50 mM NH₄HCO₃ (pH 10) - CH₃CN) to give the intermediate compound *tert*-butyl [2-(4-bromophenyl)imidazo[1,2-a]pyridin-6-yl]carbamate as a solid (83 mg). MS (ESI+) m/z 388 (M+H)⁺.

The intermediate (83 mg, 0.17 mmol) was treated with 12.3 M formaldehyde in water (113 µL, 1.40 mmol) and sodium acetate (72 mg, 0.87 mmol) in acetic acid (1 mL) at 50 °C overnight. The reaction mixture was added to sat NaHCO₃ (30 mL) and the product was extracted with CH₂Cl₂. The organic phase was dried (Na₂SO₄), filtered and concentrated to give *tert*-butyl [2-(4-bromophenyl)-3-(hydroxymethyl)imidazo[1,2-a]pyridin-6-yl]carbamate, which was then dissolved in a mixture of CH₂Cl₂ (1 mL) and TFA (1 mL). The mixture was stirred at rt for 1 h and then concentrated. The product was purified by preparative HPLC (Xterra C18, 50 mM NH₄HCO₃ (pH 10) - CH₃CN) to give the title compound as a solid (5 mg). HRMS (ESI+) calcd for C₁₄H₁₂BrN₃O 317.0164, found 317.0175.

### EXAMPLE 79

### [6-chloro-2-(4-chlorophenyl)imidazo[1,2-b]pyridazin-3-yl]methanol

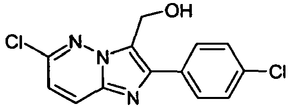

3-Amino-6-chloropyridazine (0.50 g, 3.86 mmol) and 2-bromo-4'-chloroacetophenone (0.60 g, 2.57 mmol) were dissolved in ethanol (5 mL) and stirred at 60 °C overnight. The precipitate that formed was collected by filtration, washed with ethanol and dried *in vacuo* to give intermediate compound 6-chloro-2-(4-chlorophenyl)imidazo[1,2-b]pyridazine hydrobromide (532 mg). MS (ESI+) m/z 264 [M+H]⁺.

This intermediate (526 mg, 1.52 mmol) was treated with 12.3 M formaldehyde in water (991 µL, 12.2 mmol) and sodium acetate (625 mg, 7.62 mmol) in acetic acid (10 mL) at 50 °C for five days. The solvent was evaporated and to the residue was added 5% NaHCO₃ (30 mL) and the product was extracted with ethyl acetate (50 mL). The organic phase was dried (Na₂SO₄), filtered and concentrated to give the crude product as a white solid (0.6 g). A small sample was purified by preparative HPLC (Xterra C18, 50 mM NH₄HCO₃ (pH 10) - CH₃CN) to give the title compound as a white solid. (6.3 mg). HRMS (ESI+) calcd for C₁₃H₉Cl₂N₃O 293.0123, found 293.0129.

### EXAMPLE 80

### [2-(4-Chlorophenyl)imidazo[1,2-a]pyrazin-3-yl]methanol

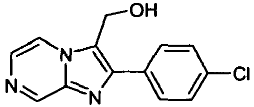

Aminopyrazine (29 mg, 0.30 mmol) and 4-chlorophenacyl bromide (47 mg, 0.20 mmol) were mixed in ethanol (0.5 mL) and stirred at rt over the weekend. The mixture was then heated at 70 °C overnight. 0.5 M NaOH (1.2 mL) was added slowly with stirring. The precipitate that formed was collected by centrifugation and washed with water (1.5 mL) to give the intermediate compound 2-(4-chlorophenyl)imidazo[1,2-a]pyrazine. MS (ESI+) m/z 230, 232 [M+H]+.

To the intermediate was added sodium acetate (82 mg, 1.0 mmol) followed by 12.3 M formaldehyde in water (0.16 mL, 2.0 mmol), acetic acid (0.4 mL) and acetonitrile (0.5 mL). The reaction mixture was heated at 50 °C for 8 h. 4 M NaOH (1.8 mL) was added slowly while stirring and the mixture was extracted with CH₂Cl₂ (2 x 2 mL). The organic phase was washed with brine (2 mL) and concentrated. Part of the material (∼50%) was purified by preparative HPLC (Xterra C18, 50 mM NH₄HCO₃ (pH 10) - CH₃CN) to give the title compound as a light yellow solid (0.6 mg). HRMS (ESI+) calcd for C₁₃H₁₀ClN₃O 259.0512, found 259.0515.

### EXAMPLE 81

### [6-Bromo-2-(3-methoxyphenyl)imidazo[1,2-a]pyrazin-3-yl]methanol

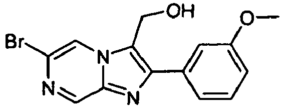

2-Amino-5-bromopyrazine (23 mg, 0.13 mmol) and 2-bromo-3'-methoxyacetophenone (23 mg, 0.10 mmol) were mixed in ethanol (0.5 mL) and stirred at 60 °C for 40 h. 0.5 M NaOH (0.6 mL) was added slowly with stirring. The precipitate that formed was collected by centrifugation and washed with water (1 mL) to give the intermediate compound 6-bromo-2-(3-methoxyphenyl)imidazo[1,2-a]pyrazine. MS (ESI+) m/z 304, 306 [M+H]+.

To the intermediate was added sodium acetate (41 mg, 0.50 mmol) followed by 12.3 M formaldehyde in water (0.10 mL, 1.2 mmol), acetic acid (0.3 mL) and acetonitrile (0.3 mL). The reaction mixture was heated at 50 °C for 4 days and then concentrated. The material was purified by preparative HPLC (Xterra C18, 50 mM NH₄HCO₃ (pH 10) - CH₃CN) to give the title compound as a white solid (7.8 mg). HRMS (ESI+) calcd for C₁₄H₁₂BrN₃O₂ 333.0113, found 333.0124.

### EXAMPLE 82

### {6-Bromo-2-[4-(trifluoromethyl)phenyl}imidazo[1,2-a]pyrazin-3-yl}methanol

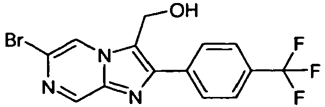

2-Amino-5-bromopyrazine (23 mg, 0.13 mmol) and 4-(trifluoromethyl)phenacyl bromide (27 mg, 0.10 mmol) were mixed in ethanol (0.5 mL) and stirred at 60 °C for 40 h. 0.5 M NaOH (0.6 mL) was added slowly with stirring. The precipitate that formed was collected by centrifugation and washed with water (1 mL) to give intermediate compound 6-bromo-2-[4-(trifluoromethyl)phenyl]imidazo[1,2-a]pyrazine. MS (ESI+) m/z 342, 344 [M+H]+.

To this intermediate was added sodium acetate (41 mg, 0.50 mmol) followed by 12.3 M formaldehyde in water (0.10 mL, 1.2 mmol), acetic acid (0.3 mL) and acetonitrile (0.3 mL). The reaction mixture was heated at 50 °C for 6 days and then at 90 °C for 2 days, and then concentrated. The material was purified by preparative HPLC (Xterra C18, 50 mM NH₄HCO₃ (pH 10) - CH₃CN) to give the title compound as a white solid (6.1 mg). HRMS (ESI+) calcd for C₁₄H₉BrF₃N₃O 370.9881, found 370.9894.

### EXAMPLE 83

### [6-Bromo-2-(4-fluorophenyl)imidazo[1,2-a]pyrazin-3-yl] methanol

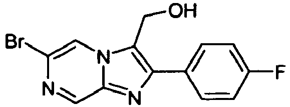

2-Amino-5-bromopyrazine (23 mg, 0.13 mmol) and 4-fluorophenacyl bromide (22 mg, 0.10 mmol) were mixed in ethanol (0.5 mL) and stirred at 60 °C for 40 h. 0.5 M NaOH (0.6 mL) was added slowly with stirring. The precipitate that formed was collected by centrifugation and washed with water (1 mL) to give the intermediate compound 6-bromo-2-(4-fluorophenyl)imidazo[1,2-a]pyrazine. MS (ESI+) m/z 292, 294 [M+H]+.

To the intermediate was added sodium acetate (41 mg, 0.50 mmol) followed by 12.3 M formaldehyde in water (0.10 mL, 1.2 mmol), acetic acid (0.3 mL) and acetonitrile (0.3 mL). The reaction mixture was heated at 50 °C for 4 days and then concentrated. The material was purified by preparative HPLC (Xterra C18, 50 mM NH₄HCO₃ (pH 10) - CH₃CN) to give the title compound as a white solid (4.1 mg). HRMS (ESI+) calcd for C₁₃H₉BrFN₃O 320.9913, found 320.9926.

### EXAMPLE 84

### [6-Bromo-2-(4-chlorophenyl)imidazo[1,2-a]pyrazin-3-yl]methanol

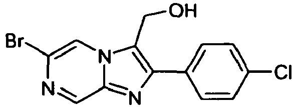

2-Amino-5-bromopyrazine (23 mg, 0.13 mmol) and 4-chlorophenacyl bromide (23 mg, 0.10 mmol) were mixed in ethanol (0.5 mL) and stirred at 60 °C for 40 h. 0.5 M NaOH (0.6 mL) was added slowly with stirring. The precipitate that formed was collected by centrifugation and washed with water (1 mL) to give the intermediate compound 6-bromo-2-(4-chlorophenyl)imidazo[1,2-a]pyrazine. MS (ESI+) m/z 308, 310 [M+H]+.

To the intermediate was added sodium acetate (41 mg, 0.50 mmol) followed by 12.3 M formaldehyde in water (0.10 mL, 1.2 mmol), acetic acid (0.3 mL) and acetonitrile (0.3 mL). The reaction mixture was heated at 50 °C for 6 days and then concentrated. The material was purified by preparative HPLC (Xterra C18, 50 mM NH₄HCO₃ (pH 10) - CH₃CN) to give the title compound as a white solid (8.4 mg). HRMS (ESI+) calcd for C₁₃H₉BrClN₃O 336.9618, found 336.9623.

### EXAMPLE 85

### [6-Bromo-2-(4-bromophenyl)imidazo[1,2-a]pyrazin-3-yl]methanol

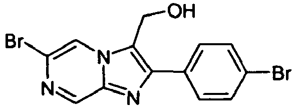

A mixture of 2-bromo-1-(4-bromophenyl)ethanone (1.11 g, 4.0 mmol) and 2-amino-5-bromopyrazine (0.70 g, 4.0 mmol) in ethanol (5 mL) was stirrred at 60 °C overnight. The reaction mixture was cooled to 0 °C and the precipitate was collected by filtration and washed with cold ethanol. Recrystallization from ethanol gave the intermediate compound 6-bromo-2-(4-bromophenyl)imidazo[1,2-a]pyrazine hydrobromide as a brown solid (230 mg).

The intermediate (0.22 g, 0.5 mmol) was treated with 12.3 M formaldehyde in water (0.33 mL, 4 mmol) and sodium acetate (0.21 g, 2.5 mmol) in acetic acid (2 mL) at 50 °C overnight. The mixture was diluted with acetic acid (2 mL) and heated at 70 °C for 6 h and then at 40 °C for 65 h. The reaction mixture was slowly added to 1 M NaOH (100 mL) with stirring and then stirred for 1 h at rt. The solid was collected by filtration and washed with water until the filtrate became neutral. The material was dried *in vacuo* to give the title compound as a light brown solid (210 mg). HRMS (ESI+) calcd for C₁₃H₉Br₂N₃O 380.9112, found 380.9117.

### EXAMPLE 86

### [6-Bromo-2-(2,4-dichlorophenyl)imidazo[1,2-a]pyrazin-3-yl]methanol

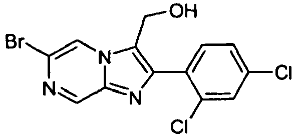

2-Amino-5-bromopyrazine (23 mg, 0.13 mmol) and 2-bromo-2',4'-dichloroacetophenone (26 mg, 0.10 mmol) were mixed in ethanol (0.5 mL) and stirred at 60 °C for 40 h. 0.5 M NaOH (0.6 mL) was added slowly with stirring. The precipitate that formed was collected by centrifugation and washed with water (1 mL) to give the intermediate compound 6-bromo-2-(2,4-dichlorophenyl)imidazo[1,2-a]pyrazine. MS (ESI+) m/z 342, 344 [M+H]+.

To the intermediate was added sodium acetate (41 mg, 0.50 mmol) followed by 12.3 M formaldehyde in water (0.10 mL, 1.2 mmol), acetic acid (0.3 mL) and acetonitrile (0.3 mL). The reaction mixture was heated at 50 °C for 6 days, at 90 °C for 2 days, and then concentrated. The material was purified by preparative HPLC (Xterra C18, 50 mM NH₄HCO₃ (pH 10) - CH₃CN) to give the title compound as a white solid (9 mg). HRMS (ESI+) calcd for C₁₃H₈BrCl₂N₃O 370.9228, found 370.9239.

### EXAMPLE 87

### [6-Bromo-2-(2,4-difluorophenyl)imidazo[1,2-a]pyrazin-3-yl] methanol

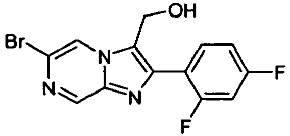

2-Amino-5-bromopyrazine (23 mg, 0.13 mmol) and 2,4-difluorophenacyl bromide (24 mg, 0.10 mmol) were mixed in ethanol (0.5 mL) and stirred at 60 °C for 40 h. 0.5 M NaOH (0.6 mL) was added slowly with stirring. The precipitate that formed was collected by centrifugation and washed with water (1 mL) to give the intermediate compound 6-bromo-2-(2,4-difluorophenyl)imidazo[1,2-a]pyrazine. MS (ESI+) m/z 310, 312 [M+H]+.

To the intermediate was added sodium acetate (41 mg, 0.50 mmol) followed by 12.3 M formaldehyde in water (0.10 mL, 1.2 mmol), acetic acid (0.3 mL) and acetonitrile (0.3 mL). The reaction mixture was heated at 50 °C for 6 days and then concentrated. The material was purified by preparative HPLC (Xterra C18, 50 mM NH₄HCO₃ (pH 10) - CH₃CN) to give the title compound as a white solid (6.5 mg). HRMS (ESI+) calcd for C₁₃H₈BrF₂N₃O 338.9819, found 338.9817.

### EXAMPLE 88

### [6-Bromo-2-(4-chloro-2-fluoro-5-methylphenyl)imidazo[1,2-a]pyrazin-3-yl]methanol

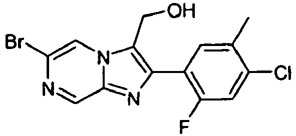

2-Amino-5-bromopyrazine (23 mg, 0.13 mmol) and 4-chloro-2-fluoro-5-methylphenacyl bromide (27 mg, 0.10 mmol) were mixed in ethanol (0.5 mL) and stirred at 60 °C for 40 h. 0.5 M NaOH (0.6 mL) was added slowly with stirring. The precipitate that formed was collected by centrifugation and washed with water (1 mL) to give the intermediate compound 6-bromo-2-(4-chloro-2-fluoro-5-methylphenyl)imidazo[1,2-a]pyrazine. MS (ESI+) m/z 340, 342 [M+H]+.

To the intermediate was added sodium acetate (41 mg, 0.50 mmol) followed by 12.3 M formaldehyde in water (0.10 mL, 1.2 mmol), acetic acid (0.3 mL) and acetonitrile (0.3 mL). The reaction mixture was heated at 50 °C for 6 days, at 90 °C for 2 days and then concentrated. The material was purified by preparative HPLC (Xterra C18, 50 mM NH₄HCO₃ (pH 10) - CH₃CN) to give the title compound as a white solid (9.7 mg).
HRMS (ESI+) calcd for C₁₄H₁₀BrClFN₃O 368.9680, found 368.9696.

### EXAMPLE 89

### [2-(1-Benzofuran-5-yl)-6-bromoimidazo[1,2-a]pyrazin-3-yl]methanol

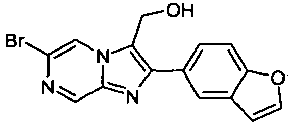

2-Amino-5-bromopyrazine (23 mg, 0.13 mmol) and 5-(2-bromoacetyl)benzofuran (24 mg, 0.10 mmol) were mixed in ethanol (0.5 mL) and stirred at 60 °C for 40 h. 0.5 M NaOH (0.6 mL) was added slowly with stirring. The precipitate that formed was collected by centrifugation and washed with water (1 mL) to give the intermediate compounc 2-(1-benzofuran-5-yl)-6-bromoimidazo[1,2-a]pyrazine. MS (ESI+) m/z 314, 316 [M+H]+.

To the intermediate was added sodium acetate (41 mg, 0.50 mmol) followed by 12.3 M formaldehyde in water (0.10 mL, 1.2 mmol), acetic acid (0.3 mL) and acetonitrile (0.3 mL). The reaction mixture was heated at 50 °C for 24 h and then concentrated. The material was purified by preparative HPLC (Xterra C18, 50 mM NH₄HCO₃ (pH 10) - CH₃CN) to give the title compound as a white solid (11 mg).
HRMS (ESI+) calcd for C₁₅H₁₀BrN₃O₂ 342.9956, found 342.9972.

### EXAMPLE 90

### [6-Bromo-2-(2,3-dihydro-1,4-benzodioxin-5-yl)imidazo[1,2-a]pyrazin-3-yl]methanol

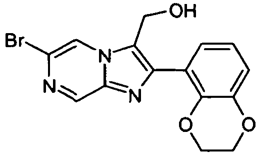

2-Amino-5-bromopyrazine (23 mg, 0.13 mmol) and 2-bromo-1-(2,3-dihydro-1,4-benzodioxin-5-yl)-1-ethanone (26 mg, 0.10 mmol) were mixed in ethanol (0.5 mL) and stirred at 60 °C for 40 h. 0.5 M NaOH (0.6 mL) was added slowly with stirring. The precipitate that formed was collected by centrifugation and washed with water (1 mL) to give the intermediate compound 6-bromo-2-(2,3-dihydro-1,4-benzodioxin-5-yl)imidazo[1,2-a]pyrazine. MS (ESI+) m/z 332, 334 [M+H]+.

To the intermediate was added sodium acetate (41 mg, 0.50 mmol) followed by 12.3 M formaldehyde in water (0.10 mL, 1.2 mmol), acetic acid (0.3 mL) and acetonitrile (0.3 mL). The reaction mixture was heated at 50 °C for 4 days and then concentrated. The material was purified by preparative HPLC (Xterra C18, 50 mM NH₄HCO₃ (pH 10) - CH₃CN) to give the title compound as a yellow solid (2.4 mg).
HRMS (ESI+) calcd for C₁₅H₁₂BrN₃O₃ 361.0062, found 361.0067.

### EXAMPLE 91

### [6-amino-2-(4-fluorophenyl)imidazo[1,2-a] pyrazin-3-yl]methanol

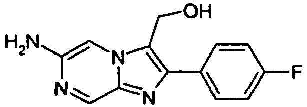

A mixture of [6-bromo-2-(4-fluorophenyl)imidazo[1,2-a]pyrazin-3-yl]methanol (Example 83; 16 mg, 0.050 mmol) and copper(II) sulfate pentahydrate (19 mg, 0.075 mmol) in a 25% aqueous ammonia solution (1 mL) was heated at 90 °C for 3 h in a sealed tube. Methanol (1.5 mL) was added and the mixture was filtered and purified by preparative HPLC (Xterra C 18, 50 mM NH₄HCO₃ (pH 10) - CH₃CN) to give the title compound as an off-white solid (2.9 mg). HRMS (ESI+) calcd for C₁₃H₁₁FN₄O 258.0917, found 258.0920.

### EXAMPLE 92

### [6-amino-2-(4-chlorophenyl)imidazo[1,2-a]pyrazin-3-yl] methanol

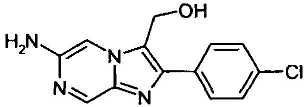

A mixture of [6-bromo-2-(4-chlorophenyl)imidazo[1,2-a]pyrazin-3-yl]methanol (Example 84; 17 mg, 0.050 mmol) and copper(II) sulfate pentahydrate (7.5 mg, 0.030 mmol) in a 25% aqueous ammonia solution (1 mL) was heated at 90 °C for 3 h in a sealed tube. Methanol (1.5 mL) was added and the mixture was filtered and purified by preparative HPLC (Xterra C18, 50 mM NH₄HCO₃ (pH 10) - CH₃CN) to give the title compound as an off-white solid (1.6 mg). HRMS (ESI+) calcd for C₁₃H₁₁ClN₄O 274.0621, found 274.0622.

### EXAMPLE 93

### [6-Amino-2-(4-bromophenyl)imidazo[1,2-a]pyrazin-3-yl]methanol

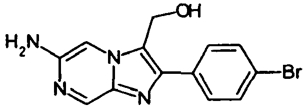

A mixture of [6-bromo-2-(4-bromophenyl)imidazo[1,2-a]pyrazin-3-yl]methanol (Example 85; 10 mg, 0.026 mmol) and 25% aqueous ammonia solution (0.75 mL) in DMF (0.25 mL) was heated at 120 °C for 90 min in a sealed tube. The mixture was purified by preparative HPLC (Xterra C18, 50 mM NH₄HCO₃ (pH 10) - CH₃CN) to give the title compound as a solid (1.2 mg). HRMS (ESI+) calcd for C₁₃H₁₁BrN₄O 318.0116, found 318.0117.

### EXAMPLE 94

### [6-(Azetidin-1-yl)-2-(4-fluorophenyl)imidazo[1,2-a]pyrazin-3-yl]methanol

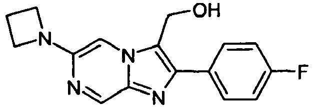

A mixture of [6-bromo-2-(4-fluorophenyl)imidazo[1,2-a]pyrazin-3-yl]methanol (Example 83; 16.1 mg, 0.050 mmol) and azetidine (135 µL, 2.0 mmol) was stirred at rt for 1 h. Methanol (1 mL) and water (1 mL) were added and the mixture was purified by preparative HPLC (Xterra C18, 50 mM NH₄HCO₃ (pH 10) - CH₃CN) to give the title compound as a white solid (5.7 mg). HRMS (ESI+) calcd for C₁₆H₁₅FN₄O 298.1230, found 298.1243.

### EXAMPLE 95

### [2-(4-Chlorophenyl)imidazo[1,2-a]pyrimidin-3-yl]methanol

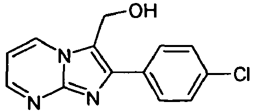

2-Aminopyrimidine (29 mg, 0.30 mmol) and 4-chlorophenacyl bromide (47 mg, 0.20 mmol) were mixed in ethanol (0.5 mL) and stirred at rt over the weekend. The mixture was then heated at 70 °C for 4 h. 0.5 M NaOH (1.2 mL) was added slowly with stirring. The precipitate that formed was collected by centrifugation and washed with water (1.5 mL) to give the intermediate compound 2-(4-chlorophenyl)imidazo[1,2-a]pyrimidine. MS (ESI+) m/z 230, 232 [M+H]+.

To the intermediate was added sodium acetate (82 mg, 1.0 mmol) followed by 12.3 M formaldehyde in water (0.16 mL, 2.0 mmol), acetic acid (0.4 mL) and acetonitrile (0.5 mL). The reaction mixture was heated at 50 °C for 8 h. 4 M NaOH (1.8 mL) was added slowly with stirring and the mixture was extracted with CH₂Cl₂ (2 x 2 mL). The organic phase was washed with brine (2 mL) and concentrated. Part of the material (∼50%) was purified by preparative HPLC (Xterra C18, 50 mM NH₄HCO₃ (pH 10) - CH₃CN) to give the title compound as a white solid (15 mg). HRMS (ESI+) calcd for C₁₃H₁₀ClN₃O 259.0512, found 259.0522.

### EXAMPLE 96

### [2-(2,4-Dichlorophenyl)imidazo[1,2-a]pyrimidin-3-yl]methanol

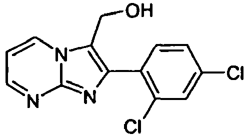

2-Aminopyrimidine (29 mg, 0.30 mmol) and 2,4-dichlorophenacyl chloride (45 mg, 0.20 mmol) were mixed in ethanol (0.5 mL) and stirred at rt over the weekend. The mixture was then heated at 70 °C for 2 days. 0.5 M NaOH (1.2 mL) was added slowly with stirring. The precipitate that formed was collected by centrifugation and washed with water (1.5 mL) to give the intermediate compound 2-(2,4-dichlorophenyl)imidazo[1,2-a]pyrimidine. MS (ESI+) m/z 264, 266 [M+H]+.

To the intermediate was added sodium acetate (82 mg, 1.0 mmol) followed by 12.3 M formaldehyde in water (0.16 mL, 2.0 mmol), acetic acid (0.4 mL) and acetonitrile (0.5 mL). The reaction mixture was heated at 50 °C for 4 days and then concentrated. Part of the material (∼50%) was purified by preparative HPLC (Xterra C18, 50 mM NH₄HCO₃ (pH 10) - CH₃CN) to give the title compound as a white solid (4.2 mg). HRMS (ESI+) calcd for C₁₃H₉Cl₂N₃O 293.0123, found 293.0135.

### EXAMPLE 97

### [6-(4-fluorophenyl)-2-methylimidazo[2,1-b][1,3]oxazol-5-yl]methanol

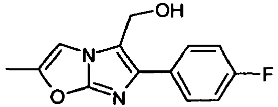

2-Amino-5-methyloxazole (55 mg, 0.56 mmol) and 2-bromo-4'-fluoroacetophenone (97 mg, 0.45 mmol) were mixed in dry CH₂Cl₂ (1.5 mL) and stirred at rt overnight. The solid material was isolated, washed with CH₂Cl₂ (1.5 mL) and dried *in vacuo* to give 1-(4-fluorophenyl)-2-(2-imino-5-methyl-1,3-oxazol-3(2H)-yl)ethanone hydrobromide as a beige solid (39 mg).

To this compound (39 mg, 0.12 mmol) in dry 1,2-dichloroethane (2 mL) under nitrogen was added titanium(IV) chloride (34 µL, 0.31 mmol). The mixture was stirred in a sealed tube at 100 °C for 2 h. The mixture was partitioned between CH₂Cl₂ and water. The organic phase was collected, washed with sat K₂CO₃ solution and water, and dried (MgSO₄). The solvents were evaporated and the residue was dried *in vacuo* to give the intermediate compound 6-(4-fluorophenyl)-2-methylimidazo[2,1-b][1,3]oxazole as a beige solid (18.4 mg).

To the intermediate (11 mg, 0.051 mmol) was added sodium acetate (17 mg, 0.20 mmol) and 12.3 M formaldehyde in water (99 µL, 1.2 mmol) followed by acetic acid (0.2 mL) and acetonitrile (0.3 mL). The mixture was heated at 50 °C for 2 h and then diluted with methanol (1 mL) and basified with 5 M NaOH (0.8 mL). The solution was filtered and purified by preparative HPLC (Xterra C18, 50 mM NH₄HCO₃ (pH 10) - CH₃CN) to give the title compound as a white solid (10.9 mg). HRMS (ESI+) calcd for C₁₃H₁₁FN₂O₂ 246.0805, found 246.0810.

### EXAMPLE 98

### [6-(4-Chlorophenyl)imidazo[2,1-b][1,3]thiazol-5-yl] methanol

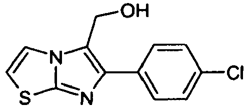

A solution of 2-aminothiazole (20 mg, 0.2 mmol) and 2-bromo-4'-chloroacetophenone (47 mg, 0.2 mmol) in acetone (2 mL) was heated at reflux overnight. The precipitate that formed was isolated by centrifugation and dissolved in dry acetonitrile (5 mL). K₂CO₃ (56 mg, 0.4 mmol) and molecular sieves were added and the mixture was heated at 80 °C for 4 h. Filtration and evaporation gave a crude material which was dissolved in CH₂Cl₂ and washed with water. Drying (MgSO₄), filtration and evaporation afforded the intermediate 6-(4-chlorophenyl)imidazo[2,1-b][1,3]thiazole, which was used without further purification.

To this intermediate was added sodium acetate (82 mg, 1.0 mmol), 12.3 M formaldehyde in water (0.16 mL, 2.0 mmol) and acetic acid (2.0 mL). The reaction mixture was heated at 50 °C for 4 h. The solvent was removed under reduced pressure and the crude was dissolved in CH₂Cl₂ and washed with 1 M NaOH (5 mL). The organic layer was collected and the solvent was removed under reduced pressure. The crude product was purified by preparative HPLC (Xterra C18, 50 mM NH₄HCO₃ (pH 10) - CH₃CN) to give the title compound as a white solid (23 mg). HRMS (ESI+) calcd for C₁₂H₉ClN₂OS 264.0124, found 264.0133.

### EXAMPLE 99

### [6-(4-Bromophenyl)imidazo[2,1-b][1,3]thiazol-5-yl]methanol

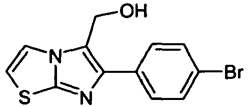

A solution of 2-amino-thiazole (20 mg, 0.2 mmol) and 2,4'-dibromoacetophenone (56 mg, 0.2 mmol) in acetone (2 mL) was heated at reflux for 30 min. The precipitate that formed was collected and dissolved in hot water (1 mL). K₂CO₃ (55 mg, 0.4 mmol) was added and the reaction mixture was heated at 80 °C overnight. The reaction mixture was allowed to cool to rt and extracted with CH₂Cl₂. The organic layer was collected and concentrated to give the intermediate compound 6-(4-bromophenyl)imidazo[2,1-b][1,3]thiazole.

To the intermediate was added sodium acetate (82 mg, 1.0 mmol) followed by 12.3 M formaldehyde in water (0.16 mL, 2.0 mmol) and acetic acid (2.0 mL). The reaction mixture was heated at 50 °C for 3 h. 1 M NaOH (5 mL) was added slowly with stirring and the reaction mixture was extracted with CH₂Cl₂ (3 mL). The crude product was purified by preparative HPLC (Xterra C18, 50 mM NH₄HCO₃ (pH 10) - CH₃CN) to give the title compound as a white solid (1 mg). HRMS (ESI+) calcd for C₁₂H₉BrN₂OS 307.9619, found 307.9624.

### EXAMPLE 100

### [6-(2,4-dichlorophenyl)imidazo[2,1-b][1,3]thiazol-5-yl]methanol

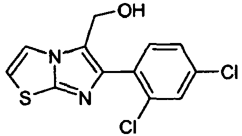

A solution of 2-amino-thiazole (20 mg, 0.2 mmol) and 2-bromo-2',4'-dichloroacetophenone (56 mg, 0.2 mmol) in acetone (2 mL) was heated at reflux for 2 h. The precipitate that formed was isolated by centrifugation and then dissolved in hot water (1 mL). K₂CO₃ (56 mg, 0.4 mmol) was added and the reaction mixture was heated at 80 °C overnight. The reaction mixture was allowed to cool to rt and extracted with CH₂Cl₂ (2 x 5 mL). The organic layers were collected and the solvent was removed under reduced pressure. The intermediate compound 6-(2,4-dichlorophenyl)imidazo[2,1-b][1,3]thiazole was used without further purification.

To the intermediate was added sodium acetate (82 mg, 1.0 mmol), 12.3 M formaldehyde in water (0.16 mL, 2.0 mmol) and acetic acid (2.0 mL). The reaction mixture was heated at 50 °C for 2 h. 1 M NaOH (5 mL) was added whereupon the product precipitated. After centrifugation the precipitate was collected, dissolved in methanol and purified by preparative HPLC (Xterra C18, 50 mM NH₄HCO₃ (pH 10) - CH₃CN) to give the title compound as a white solid (1.3 mg). MS (ESI+) m/z 299, 301 [M+H]+.

### EXAMPLE 101

### [6-(4-Bromophenyl)-2-methylimidazo[2,1-b][1,3]thiazol-5-yl]methanol

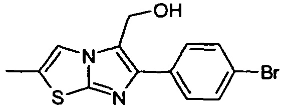

A solution of 2-amino-5-methylthiazole (23 mg, 0.2 mmol) and 2,4'-dibromoacetophenone (56 mg, 0.2 mmol) in acetone (2 mL) was heated at reflux overnight. The precipitate that formed was isolated by centrifugation and then dissolved in dry acetonitrile (5 mL). K₂CO₃ (56 mg, 0.4 mmol) and molecular sieves were added and the reaction mixture was heated at 80 °C for 4 h. Filtration and evaporation gave a crude material which was dissolved in CH₂Cl₂ and washed with water. Drying (MgSO₄), filtration and evaporation afforded the intermediate compound 6-(4-bromophenyl)-2-methylimidazo[2,1-b][1,3]thiazole, which was used without further purification.

To the intermediate was added sodium acetate (82 mg, 1.0 mmol), 12.3 M formaldehyde in water (0.16 mL, 2.0 mmol) and acetic acid (2.0 mL). The reaction mixture was heated at 50 °C for 4 h. The solvent was removed under reduced pressure and the residue was dissolved in CH₂Cl₂ and washed with 1 M NaOH (5 mL). The organic layer was collected and the solvent was removed under reduced pressure. The crude product was purified by preparative HPLC (Xterra C18, 50 mM NH₄HCO₃ (pH 10) - CH₃CN) to give the title compound as a white solid (4 mg). HRMS (ESI+) calcd for C₁₃H₁₁BrN₂OS 321.9775, found 321.9784.

### EXAMPLE 102

### [6-(2,4-Dichlorophenyl)-2-methylimidazo[2,1-b][1,3]thiazol-5-yl]methanol

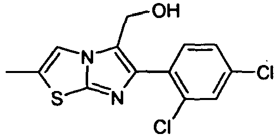

A solution of 2-amino-5-methylthiazole (23 mg, 0.2 mmol) and 2-bromo-2',4'-dichloroacetophenone (53 mg, 0.2 mmol) in acetone (2 mL) was heated at reflux overnight.

The precipitate that formed was isolated by centrifugation and then dissolved in dry acetonitrile (5 mL). K₂CO₃ (56 mg, 0.4 mmol) and molecular sieves were added. The reaction mixture was heated at 80 °C for 4 h. Following filtration and evaporation, the material was dissolved in CH₂Cl₂ and washed with water. Drying (MgSO₄), filtration and evaporation afforded the intermediate compound 6-(2,4-dichlorophenyl)-2-methylimidazo[2,1-b][1,3]thiazole, which was used without further purification.

To the intermediate was added sodium acetate (82 mg, 1.0 mmol), 12.3 M formaldehyde in water (0.16 mL, 2.0 mmol) and acetic acid (2.0 mL). The reaction mixture was heated at 50 °C for 4 h. The solvent was removed under reduced pressure and the residue was dissolved in CH₂Cl₂ and washed with 1 M NaOH (5 mL). The organic layer was collected and the solvent was removed under reduced pressure. The crude product was purified by preparative HPLC (Xterra C18, 50 mM NH₄HCO₃ (pH 10) - CH₃CN) to give the title compound as a white solid (24 mg). HRMS (ESI+) calcd for C₁₃H₁₀Cl₂N₂OS 311.9891, found 311.9902.

### EXAMPLE 103

### [2-Chloro-6-(4-chlorophenyl)imidazo[2,1-b][1,3]thiazol-5-yl]methanol

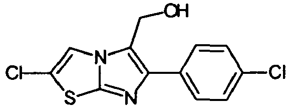

A mixture of 2-amino-5-chlorothiazole (436 mg, 2.55 mmol) and 2-bromo-1-(4-chlorophenyl)ethanone (476 mg, 2.04 mmol) in ethanol (6 mL) was heated at 70 °C for 2 h. After cooling, the product was collected by filtration, washed with ethanol and dried in vacuo to give the intermediate compound 2-chloro-6-(4-chlorophenyl)imidazo[2,1-b][1,3]thiazole hydrobromide as an off-white solid (325 mg).

The intermediate (316 mg, 0.90 mmol) was treated with 12.3 M formaldehyde in water (1.17 mL, 14 mmol) and sodium acetate (0.74 g, 9 mmol) in acetic acid (5 mL) at 80 °C for 2 h. The solvents were evaporated and the residue was dried *in vacuo.* The material was dissolved in methanol (50 mL) and treated with 1 M NaOH (5mL) at rt for 1 h. Water was added and the precipitate that formed was collected by filtration. The crude material was triturated in methanol, collected by filtration and dried *in vacuo* to give the title compound as an off-white solid (258 mg). HRMS (ESI+) calcd for C₁₂H₈C₁₂N₂OS 297.9734, found 297.9741.

### EXAMPLE 104

### Methyl 6-(4-chlorophenyl)-5-(hydroxymethyl)imidazo[2,1-b][1,3]thiazole-2-carboxylate

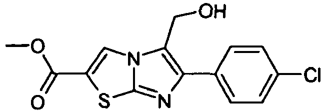

A solution of methyl-2-aminothiazole-5-carboxylate (1.0 g, 7.8 mmol) and 2-bromo-4'-chloroacetophenone (1.8 g, 7.8 mmol) in acetone (200 mL) was heated at reflux for 72 h. The remaining intermediate was filtered off and the filtrate was collected. The solvent was removed under reduced pressure to afford the intermediate compound methyl 6-(4-chlorophenyl)imidazo[2,1-b][1,3]thiazole-2-carboxylate as a yellow solid (1.5 g).

To the intermediate (1.0 g, 3.4 mmol) was added sodium acetate (1.4 g, 17 mmol), 12.3 M formaldehyde in water (2.2 mL, 27 mmol) and acetic acid (10 mL). The reaction mixture was heated at 50 °C for 3 h. The solvent was removed under reduced pressure and the crude material was dissolved in ethyl acetate (30 mL) and washed with 1 M NaOH (15 mL). The organic layer was collected and the solvent removed under reduced pressure to give crude product (1.0 g). A small amount (15 mg) was purified by preparative HPLC (Xterra C18, 50 mM NH₄HCO₃ (pH 10) - CH₃CN) to give the title compound as a white solid (3 mg). HRMS (ESI+) calcd for C₁₄H₁₁ClN₂O₃S 322.0179, found 322.0179.

### INTERMEDIATE 4

### 6-(4-Chlorophenyl)-5-(hydroxymethyl)imidazo[2,1-b][1,3]thiazole-2-carbaldehyde

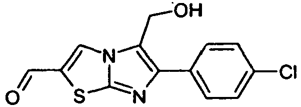

A solution of 2-aminothiazole-5-carboxaldehyde (1.0 g, 7.8 mmol) and 2-bromo-4'-chloroacetophenone (1.8 g, 7.8 mmol) in acetone (5 mL) was heated at reflux for 5 h. The solvent was evaporated and the crude material purified by preparative HPLC (Xterra C18, 50 mM NH₄HCO₃ (pH 10) - CH₃CN) to give the intermediate compound 6-(4-chlorophenyl)imidazo[2,1-b][1,3]thiazole-2-carbaldehyde as a yellow solid (176 mg).

To the intermediate was added sodium acetate (0.2 g, 2.8 mmol), 12.3 M formaldehyde in water (0.4 mL, 4.5 mmol) and acetic acid (2.0 mL). The reaction mixture was heated at 50 °C overnight. The solvent was removed under reduced pressure and the residue was dissolved in ethyl acetate (20 mL) and washed with 2 M NaOH (10 mL). The organic layer was collected and the solvent was removed under reduced pressure to give the crude title compound (125 mg). This material was used without further purification. MS (ESI+) m/z 293 [M+H]+.

### EXAMPLE 105

### [6-(4-Chlorophenyl)imidazo[2,1-b][1,3]thiazole-2,5-diyl]dimethanol

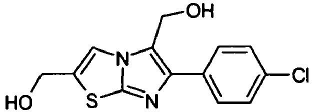

To a solution of 6-(4-chlorophenyl)-5-(hydroxymethyl)imidazo[2,1-b][1,3]thiazole-2-carbaldehyde (Intermediate 4; 10 mg, 34 µmol) in THF (1 mL) was added lithium aluminum hydride (3 mg, 0.8 mmol). The reaction mixture was stirred at rt for 30 min and then quenched by the addition of water (0.1 mL). The solvent was removed under reduced pressure and the crude product was purified by preparative HPLC (Xterra C18, 50 mM NH₄HCO₃ (pH 10) - CH₃CN) to give the title compound as a white solid (7 mg). HRMS (ESI+) calcd for C₁₃H₁₁ClN₂O₂S 294.0230, found 294.0235.

### EXAMPLE 106

### 1-[6-(4-Chlorophenyl)-5-(hydroxymethyl)imidazo[2,1-b][1,3]thiazol-2-yl]ethanol

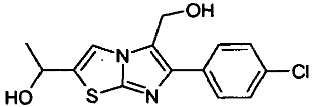

To 6-(4-chlorophenyl)-5-(hydroxymethyl)imidazo[2,1-b][1,3]thiazole-2-carbaldehyde (Intermediate 4; 14 mg, 46 µmol) was added 3 M methyl magnesium bromide in diethyl ether (0.5 mL) at rt. The reaction mixture was stirred at rt for 24 h. The reaction was then quenched by addition of sat.aq. NH₄Cl (2 mL) and the mixture was extracted with ethyl acetate (3 x 5 mL). The solvent was removed under reduced pressure and the crude product was purified by preparative HPLC (Xterra C18, 50 mM NH₄HCO₃ (pH 10) - CH₃CN) to give the title compound as a white solid (3 mg). HRMS (ESI+) calcd for C₁₄H₁₃ClN₂O₂S 308.0386, found 308.0389.

### EXAMPLE 107

### [6-(4-Chlorophenyl)-5-(hydroxymethyl)imidazo[2,1-b][1,3]thiazol-2-yl](cyclopropyl)methanol

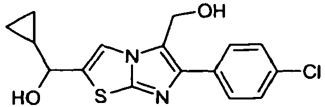

To 6-(4-chlorophenyl)-5-(hydroxymethyl)imidazo[2,1-b][1,3]thiazole-2-carbaldehyde (Intermediate 4; 14 mg, 46 µmol) was added 0.5 M cyclopropyl magnesium bromide in THF (1.5 mL) at rt. The reaction mixture was stirred at rt for 24 h. The reaction was then quenched by addition of sat. aq. NH₄Cl (2 mL) and the mixture was extracted with ethyl acetate (3 x 5 mL). The solvent was removed under reduced pressure and the crude product was purified by preparative HPLC (Xterra C18, 50 mM NH₄HCO₃ (pH 10) - CH₃CN) to give the title compound as a white solid (1 mg). HRMS (ESI+) calcd for C₁₆H₁₅ClN₂O₂S 334.0543, found 334.0545.

### EXAMPLE 108

### 2-[6-(4-Chlorophenyl)-5-(hydroxymethyl)imidazo[2,1-b][1,3]thiazol-2-yl]propan-2-ol

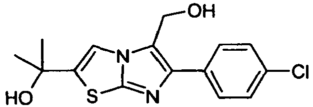

To methyl 6-(4-chlorophenyl)-5-(hydroxymethyl)imidazo[2,1-b][1,3]thiazole-2-carboxylate (Example 104; 20 mg, 62 µmol) was added 3 M methyl magnesium bromide in diethyl ether (1.5 mL) at rt. The reaction mixture was stirred at rt overnight. The reaction was quenched by addition of sat. aq. NH₄Cl and the mixture was extracted with ethyl acetate. The solvent was removed under reduced pressure and the crude product was purified by preparative HPLC (Xterra C18, 50 mM NH₄HCO₃ (pH 10) - CH₃CN) to give the title compound as a white solid (3 mg). HRMS (ESI+) calcd for C₁₅H₁₅ClN₂O₂S 322.0543, found 322.0549.

### INTERMEDIATE 5

### Sodium 6-(4-chlorophenyl)-5-(hydroxymethyl)imidazo[2,1-b][1,3]thiazole-2-carboxylate

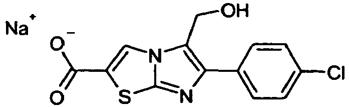

To methyl 6-(4-chlorophenyl)-5-(hydroxymethyl)imidazo[2,1-b][1,3]thiazole-2-carboxylate (Example 104; 1.0 g, 3.1 mmol) in methanol (30 mL) was added 2 M NaOH (10 mL). The reaction mixture was heated at 50°C overnight. The methanol was removed under reduced pressure and the remaining aqueous layer was washed with CH₂Cl₂. Crystallization of the product from the aqueous layer gave the title compound as a light yellow solid (280 mg). MS (ESI+) m/z 309 [M+H]+.

### EXAMPLE 109

### 6-(4-Chlorophenyl)-N-ethyl-5-(hydroxymethyl)-N-methylimidazo[2,1-b][1,3]thiazole-2-carboxamide

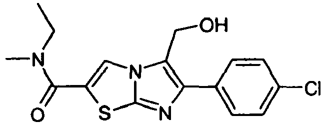

To a solution of sodium 6-(4-chlorophenyl)-5-(hydroxymethyl)imidazo[2,1-b][1,3]thiazole-2-carboxylate (Intermediate 5; 15 mg, 45 µmol) in DMF (0.5 mL) was added N-ethylmethylamine (5 mg, 90 µmol), TBTU (20 mg, 62 µmol) and triethylamine (19 µL, 0.13 mmol) at rt. The reaction mixture was stirred at rt for 2 h. The reaction was quenched by the addition of water (0.2 mL) and the mixture was purified by preparative HPLC (Xterra C18, 50 mM NH₄HCO₃ (pH 10) - CH₃CN) to give the title compound as a white solid (2 mg). MS (ESI+) m/z 350, 352 [M+H]+.

### EXAMPLE 110

### [6-(4-Chlorophenyl)-2-(morpholin-4-ylcarbonyl)imidazo[2,1-b][1,3]thiazol-5-yl]methanol

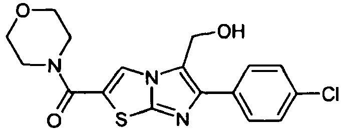

To a solution of sodium 6-(4-chlorophenyl)-5-(hydroxymethyl)imidazo[2,1-b][1,3]thiazole-2-carboxylate (Intermediate 5; 15 mg, 45 µmol) in DMF (0.5 mL) was added morpholine (8 mg, 90 µmol), TBTU (20 mg, 62 µmol) and triethylamine (19 µL, 0.13 mmol) at rt. The reaction mixture was stirred at rt for 2 h. The reaction was quenched by the addition of water (0.2 mL) and the mixture was purified by preparative HPLC (Xterra C18, 50 mM NH₄HCO₃ (pH 10) - CH₃CN) to give the title compound as a white solid (5 mg). MS (ESI+) m/z 378, 380 [M+H]+.

### EXAMPLE 111

### {6-(4-Chlorophenyl)-2-[(4-methylpiperazin-1-yl)carbonyl]imidazo[2,1-b][1,3]thiazol-5-yl}methanol

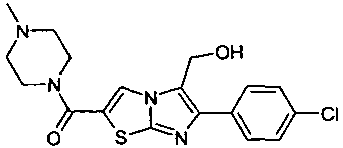

To a solution of sodium 6-(4-chlorophenyl)-5-(hydroxymethyl)imidazo[2,1-b][1,3]thiazole-2-carboxylate (Intermediate 5; 15 mg, 45 µmol) in DMF (0.5 mL) was added N-methylpiperazine (9 mg, 90 µmol), TBTU (20 mg, 62 µmol) and triethylamine (19 µL, 0.13 mmol) at rt. The reaction mixture was stirred at rt for 2 h. The reaction was quenched by the addition of water (0.2 mL) and the mixture was purified by preparative HPLC (Xterra C18, 50 mM NH₄HCO₃ (pH 10) - CH₃CN) to give the title compound as a yellow solid (2 mg). MS (ESI+) m/z 391, 393 [M+H]+.

### EXAMPLE 112

### 6-(4-Chlorophenyl)-5-(hydroxymethyl)-N-propylimidazo[2,1-b][1,3]thiazole-2-carboxamide

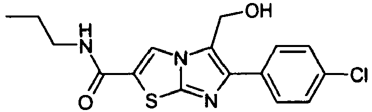

To a solution of sodium 6-(4-chlorophenyl)-5-(hydroxymethyl)imidazo[2,1-b][1,3]thiazole-2-carboxylate (Intermediate 5; 15 mg, 45 µmol) in DMF (0.5 mL) was added propylamine (5 mg, 90 µmol), TBTU (20 mg, 62 µmol) and triethylamine (19 µL, 0.13 mmol) at rt. The reaction mixture was stirred at rt for 2 h. The reaction was quenched by the addition of water (0.2 mL) and the mixture was purified by preparative HPLC (Xterra C18, 50 mM NH₄HCO₃ (pH 10) - CH₃CN) to give the title compound as a white solid (2 mg). MS (ESI+) m/z 350, 352 [M+H]+.

### BIOLOGICAL TESTS

### Biological Assays of the SSAO Enzyme Inhibitors

All primary assays were performed in rt with purified recombinantly expressed human SSAO. Enzyme was prepared essentially as described in Öhman et al. (Protein Expression and Purification 46 (2006) 321-331). In addition, secondary- and selectivity assays were performed using SSAO prepared from various tissues or purified rat recombinant SSAO. The enzyme activity was assayed with benzylamine as substrate by measuring either benzaldehyde production, using ¹⁴C-labeled substrate, or by utilizing the production of hydrogen peroxide in a horseradish peroxidise (HRP) coupled reaction. Briefly, test compounds were dissolved in dimethyl sulfoxide (DMSO) to a concentration of 10 mM. Dose-response measurements were assayed by either creating 1:10 serial dilutions in DMSO to produce a 7 point curve or by making 1:3 serial dilutions in DMSO to produce 11 point curves. The top concentrations were adjusted depending on the potency of the compounds and subsequent dilution in reaction buffer yielded a final DMSO concentration ≤2%.

### Hydrogen peroxide detection:

In a horseradish peroxidise (HRP) coupled reaction, hydrogen peroxide oxidation of 10-acetyl-3,7-dihydroxyphenoxazine produced resorufin, which is a highly fluorescent compound (Zhout and Panchuk-Voloshina. Analytical Biochemistry 253 (1997) 169-174; Amplex^{®} Red Hydrogen Peroxide/peroxidise Assay kit, Invitrogen A22188). Enzyme and compounds in 50 mM sodium phosphate, pH 7.4 were set to pre-incubate in flat-bottomed microtiter plates for approximately 15 minutes before initiating the reaction by addition of a mixture of HRP, benzylamine and Amplex reagent. Benzylamine concertration was fixed at a concentration corresponding to the Michaelis constant, determined using standard procedures. Fluorescence intensity was then measured at several time points during 1 - 2 hours, exciting at 544 nm and reading the emission at 590 nm. For the human SSAO assay final concentrations of the reagents in the assay wells were: SSAO enzyme 1 □g/ml, benzylamine 100 µM, Amplex reagent 20 µM, HRP 0.1 U/mL and varying concentrations of test compound. The inhibition was measured as % decrease of the signal compared to a control without inhibitor (only diluted DMSO). The background signal from a sample containing no SSAO enzyme was subtracted from all data points. Data was fitted to a four parameter logistic model and IC₅₀ values were calculated using the GraphPad Prism 4 or XLfit 4 programs.

### Aldehyde detection:

SSAO activity was assayed using 14C-labeled benzylamine and analysed by measuring radioactive benzaldehyde. In a white 96-well optiplate (Packard), 20 µL of diluted test compound was pre-incubated at rt with 20 µL SSAO enzyme for approximately 15 minutes with continuous agitation. All dilutions were made with PBS. The reaction was initiated by adding 20 µL of the benzylamine substrate solution containing [7-14C] Benzylamine hydrochloride (CFA589, GE Healthcare). The plate was incubated for 1 hour as above after which the reaction was stopped by acidification (10 µL 1 M HCl). Then 90 µL Micro Scint-E solution (Perkin-Elmer) was added to each well and the plate was continuously mixed for 15 minutes. Phase separation occurred instantly and activity was read in a Topcount scintillation counter (Perkin-Elmer). In the final reaction well, human recombinant SSAO concentration was 10 µg/ml. In order to optimize sensitivity, the substrate concentration was decreased as compared to the HRP coupled assay in order to get a higher fraction of radioactive product. In the human SSAO assay, benzylamine concentration was 40 µM (0.2 □Ci/mL). Data was analysed as above.

The exemplified compounds of the invention generally had an IC₅₀ value of 1-1000 nM. Obtained IC₅₀ values for representative compounds are shown in the table below:

| **Compound** | **IC₅₀ (nM)** |
|---|---|
| Example 23 | 88 |
| Example 62 | 155 |
| Example 81 | 248 |
| Example 106 | 89 |

## Claims

1. A compound of formula (I), 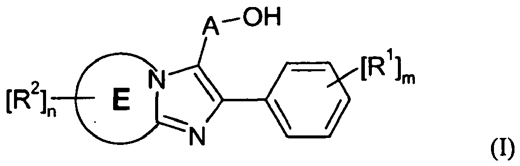 or a pharmaceutically acceptable salt, solvate, hydrate, geometrical isomer, tautomer, optical isomer or N-oxide thereof, wherein:
E is a 5- or 6-membered heteroaromatic ring;
A is C₁₋₃-alkylene, which is optionally substituted with C₁₋₃-alkyl;
each R¹ is independently selected from the group consisting of halogen, cyano, nitro, C₁₋₄-alkyl, fluoro-C₁₋₄-alkyl, C₃₋₆-cycloalkyl, heterocyclyl, phenyl, heteroaryl, -C₀₋₄-alkylene-OR³, -C₀₋₄-alkylene-SR³, -C₀₋₄-alkylene-NR^{4A}R^{4B}, -N(R⁵)-C₂₋₄-alkylene-NR^{4A}R^{4B}, -C(O)R³, -C(O)OR³, -C(O)NR^{4A}R^{4B}, -N(R⁵)C(O)R⁶, -N(R⁵)C(O)NR^{4A}R^{4B}, -N(R⁵)C(O)OR⁶, -N(R⁵)S(O)₂R⁶, -S(O)₂NR^{4A}R^{4B} and -S(O)₂R⁶, and wherein any ring residue is optionally substituted with one or more substituents independently selected from halogen, cyano, hydroxy, C₁₋₄-alkyl, C₁₋₄-alkoxy, trifluoromethyl and trifluoromethoxy;
or two neighbouring substituents R¹, together with the carbon atoms to which they are attached, form a 5- or 6-membered aromatic or partially unsaturated ring, which optionally contains one or two heteroatoms selected from nitrogen, sulphur or oxygen, and which ring is optionally substituted with one or more substituents independently selected from halogen, cyano, hydroxy, C₁₋₄-alkyl, C₁₋₄-alkoxy, trifluoromethyl and trifluoromethoxy;
each R² is independently selected from the group consisting of halogen, cyano, nitro, C₁₋₄-alkyl, fluoro-C₁₋₄-alkyl, C₃₋₆-cycloalkyl, heterocyclyl, phenyl, heteroaryl, C₃₋₆-cycloalkyl-C₁₋₄-alkyl, C₃₋₆-cycloalkyl(hydroxy)C₁₋₄-alkyl, heterocyclyl-C₁₋₄-alkyl, C₃₋₆-cycloalkylcarbonyl, heterocyclylcarbonyl, benzoyl, heteroarylcarbonyl, -C₀₋₄-alkylene-OR³, -C₀₋₄-alkylene-SR³, -C₀₋₄-alkylene-NR^{7A}R^{7B}, -C₀₋₄-alkylene-C(O)R⁶, -C₀₋₄-alkylene-C(O)OR³, -C₀₋₄-alkylene-C(O)NR^{7A}R^{7B}, -C₀₋₄-alkylene-N(R⁵)C(O)R⁶, -C₀₋₄-alkylene-N(R⁵)C(O)NR^{7A}R^{7B}, -C₀₋₄-alkylene-N(R⁵)C(O)OR⁶, -C₀₋₄-alkylene-N(R⁵)S(O)₂R⁶, -C₀₋₄-alkylene-S(O)₂NR^{7A}R^{7B}, -C₀₋₄-alkylene-S(O)₂R⁶, amidino and guanidino, and wherein any ring residue is optionally substituted with one or more substituents independently selected from halogen, cyano, hydroxy, C₁₋₄-alkyl, C₁₋₄-alkoxy, trifluoromethyl, trifluoromethoxy and -C(O)NR^{8A}R^{8B};
each R³ is selected from hydrogen, C₁₋₄-alkyl, fluoro-C₁₋₄-alkyl, hydroxy-C₁₋₄-alkyl and C₃₋₆-cycloalkyl;
each R^{4A} and R^{4B} is independently selected from hydrogen, C₁₋₄-alkyl, hydroxy-C₁₄-alkyl and -C₁₋₄-alkylene-NR^{8A}R^{8B};
or independently R^{4A} and R^{4B} together with the nitrogen atom to which they are attached form a 4- to 6-membered saturated heterocyclic ring, which is optionally substituted with one or more substituents independently selected from halogen, hydroxy, C₁₋₄-alkyl, C₁₋₄-alkoxy, trifluoromethyl and trifluoromethoxy;
each R⁵ is independently selected from hydrogen and C₁₋₄-alkyl;
each R⁶ is independently selected from C₁₋₄-alkyl, fluoro-C₁₋₄-alkyl, hydroxy-C₁₋₄-alkyl and C₃₋₆-cycloalkyl;
each R^{7A} and R^{7B} is independently selected from the group consisting of hydrogen, C₁₋₄-alkyl, hydroxy-C₁₋₄-alkyl, C₁₋₄-alkoxy-C₁₋₄-alkyl, C₃₋₆-cycloalkyl, phenyl-C₁₋₄-alkyl, heteroaryl-C₁₋₄-alkyl, C₃₋₆-cycloalkyl-C₁₋₄-alkyl and heterocyclyl-C₁₋₄-alkyl, and wherein any ring residue is optionally substituted with one or more substituents independently selected from halogen, cyano, hydroxy, C₁₋₄-alkyl, C₁₋₄-alkoxy, trifluoromethyl and trifluoromethoxy;
or independently R^{7A} and R^{7B}, together with the nitrogen atom to which they are attached, form a 4- to 6-membered saturated heterocyclic ring, which optionally contains an additional heteroatom selected from nitrogen, sulphur or oxygen, and which ring is optionally substituted with one or more substituents independently selected from halogen, hydroxy, C₁₋₄-alkyl, C₁₋₄-alkoxy, hydroxy-C₁₋₄-alkyl, -S(O)₂NR^{8A}R^{8B} and -C(O)NR^{8A}R^{8B};
each R^{8A} and R^{8B} is independently selected from hydrogen and C₁₋₄-alkyl;
m is 1, 2, 3 or 4; and
n is 0, 1 or 2;
with the provision that the compound is not selected from the group consisting of:
● 4-[3-(hydroxymethyl)-6-methylimidazo[1,2-a]pyridin-2-yl]-benzonitrile;
● 2-(4-ethylphenyl)-6-methyl-imidazo[1,2-a]pyridine-3-methanol;
● 2-(2-nitrophenyl)-imidazo[ 1,2-a]pyridine-3-methanol;
● 2-(3,4-dimethylphenyl)-6-methyl-imidazo[1,2-a]pyridine-3-methanol;
● 6-chloro-2-(4-chlorophenyl)-imidazo[1,2-a]pyridine-3-methanol;
● 2-(4-fluorophenyl)-imidazo[ 1,2-a]pyridine-3-methanol;
● 2-(2-chloro-4-methoxyphenyl)-6-(1-ethylpropyl)-8-methyl-imidazo[1,2-a]pyridine-3-methanol;
● 2-(4-chlorophenyl)-imidazo[1,2-a]pyridine-3-propanol;
● 2-(4-methylphenyl)-6-(trifluoromethyl)-imidazo[1,2-a]pyridine-3-methanol;
● 7-methyl-2-(2-naphthalenyl)-imidazo[1,2-a]pyridine-3-methanol;
● 2-(4-methylphenyl)-6-nitro-imidazo[1,2-a]pyridine-3-methanol;
● 2-[1,1'-biphenyl]-4-yl-7-methyl-imidazo[1,2-a]pyridine-3-methanol;
● 6-methyl-2-[4-(trifluoromethyl)phenyl]-imidazo[1,2-a]pyridine-3-methanol;
● 2-(4-chlorophenyl)-imidazo[1,2-a]pyridine-3-methanol;
● 6-methyl-2-(4-methylphenyl)-imidazo[1,2-a]pyridine-3-ethanol;
● 6-methyl-2-(4-methylphenyl)-imidazo[1,2-a]pyridine-3-methanol;
● ethyl 4-[3-(hydroxymethyl)-6-methylimidazo[1,2-a]pyridin-2-yl]-benzoate;
● 2-[4-(methylsulfonyl)phenyl]-imidazo[1,2-a]pyridine-3-methanol;
● 2-(4-chlorophenyl)-6-methyl-imidazo[1,2-a]pyridine-3-methanol;
● 6-methyl-2-(4-nitrophenyl)-imidazo[1,2-a]pyridine-3-methanol;
● 6-chloro-2-(4-methylphenyl)-imidazo[1,2-b]pyridazine-3-methanol;
● 3-(diethylamino)-2-methyl-6-(4-methylphenyl)-imidazo[1,2-b][1,2,4]triazine-7-methanol;
● 6-(4-chlorophenyl)-2,3-diphenyl-imidazo[1,2-b][1,2,4]triazine-7-methanol;
● 6-(4-bromophenyl)-imidazo[2,1-b]thiazole-5-methanol;
● 6-(4-chlorophenyl)-imidazo[2,1-b]thiazole-5-methanol;
● 6-(4-chlorophenyl)-2-methyl-imidazo[2,1-b]thiazole-5-methanol;
● 6-(4-methylphenyl)-imidazo[2,1-b]thiazole-5-methanol;
● 6-[1,1'-biphenyl]-4-yl.imidazo[2,1-b]thiazole-5-methanol;
● 6-(4-bromophenyl)-2-(2-furanyl)-imidazo[2,1-b]-1,3,4-thiadiazole-5-methanol;
● 6-(4-bromophenyl)-2-(2-thienyl)-imidazo[2,1-b]-1,3,4-thiadiazole-5-methanol; and
● 6-(4-bromophenyl)-2-cyclohexyl-imidazo[2,1-b]-1,3,4-thiadiazole-5-methanol.

2. A compound according to claim 1, wherein heteroaromatic ring E is selected from:

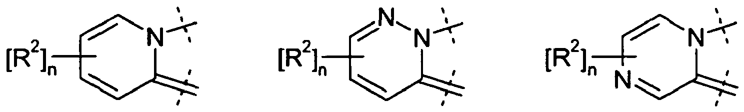

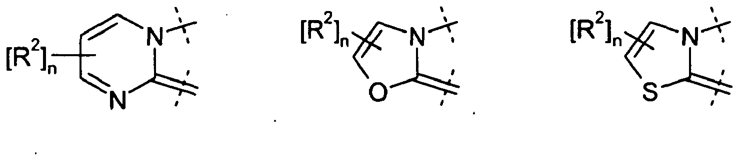

3. A compound according to claim 1 or 2, wherein A is -CH₂-.

4. A compound according to any one of claims 1 to 3, wherein R¹ is independently selected from halogen, C₁₋₄-alkyl, fluoro-C₁₋₄-alkyl and C₁₋₄-alkoxy.

5. A compound according to any one of claims 1 to 4, wherein R² is independently selected from halogen, cyano, nitro, C₁₋₄-alkyl, fluoro-C₁₋₄-alkyl, hydroxy-C₁₋₄-alkyl and C₁₋₄-alkoxycarbonyl and -C(O)NR^{7A}R^{7B}.

6. A compound according to claim 1, which is selected from:
● [2-(4-Methylphenyl)imidazo[1,2-a]pyridin-3-yl]methanol;
● [2-(2,4-Dichlorophenyl)imidazo[1,2-a]pyridin-3-yl]methanol;
● [2-(4-Bromophenyl)-8-methylimidazo[1,2-a]pyridin-3-yl]methanol;
● [7-Methyl-2-(4-methylphenyl)imidazo[1,2-a]pyridin-3-yl]methanol;
● [2-(4-Bromophenyl)-7-methylimidazo[1,2-a]pyridin-3-yl]methanol;
● [2-(4-Bromophenyl)-7-ethylimidazo[1,2-a]pyridin-3-yl]methanol;
● [2-(2-Chlorophenyl)-7-methylimidazo[1,2-a]pyridin-3-yl]methanol;
● [2-(2,4-Dichlorophenyl)-7-methylimidazo[1,2-a]pyridin-3-yl]methanol;
● [2-(3,4-Dichlorophenyl)-7-methylimidazo[1,2-a]pyridin-3-yl]methanol;
● [6-Methyl-2-(2-naphthyl)imidazo[1,2-a]pyridin-3-yl]methanol;
● [2-(3-Methoxyphenyl)-6-methylimidazo[1,2-a]pyridin-3-yl]methanol;
● 4-[3-(Hydroxymethyl)-6-methylimidazo[1,2-a]pyridin-2-yl]benzonitrile;
● [6-Methyl-2-(3-nitrophenyl)imidazo[1,2-a]pyridin-3-yl]methanol;
● [2-(4-Fluorophenyl)-6-methylimidazo[1,2-a]pyridin-3-yl]methanol;
● [2-(4-Iodophenyl)-6-methylimidazo[1,2-a]pyridin-3-yl]methanol;
● [2-(2-Chlorophenyl)-6-methylimidazo[1,2-a]pyridin-3-yl]methanol;
● (2-{4-[(2-Aminoethyl)amino]phenyl}-6-methylimidazo[1,2-a]pyridin-3-yl)methanol;
● 1-[2-(4-Chlorophenyl)-6-methylimidazo[1,2-a]pyridin-3-yl]ethanol;
● [2-(2,4-Dichlorophenyl)-6-methylimidazo[1,2-a]pyridin-3-yl]methanol;
● [2-(3-Methoxyphenyl)-6-(trifluoromethyl)imidazo[1,2-a]pyridin-3-yl]methanol;
● [2-(4-Chlorophenyl)-6-(trifluoromethyl)imidazo[1,2-a]pyridin-3-yl]methanol;
● [2-(4-Bromophenyl)-6-(trifluoromethyl)imidazo[1,2-a]pyridin-3-yl]methanol;
● [7-Chloro-2-(4-chlorophenyl)imidazo[1,2-a]pyridin-3-yl]methanol;
● [2-(4-Bromophenyl)-7-chloroimidazo[1,2-a]pyridin-3-yl]methanol;
● [7-Chloro-2-(2,4-dichlorophenyl)imidazo[1,2-a]pyridin-3-yl]methanol;
● [7-Chloro-2-(2,4-difluorophenyl)imidazo[1,2-a]pyridin-3-yl]methanol;
● [6-Bromo-2-(3-methoxyphenyl)imidazo[1,2-a]pyridin-3-yl]methanol;
● [6-Bromo-2-(4-fluorophenyl)imidazo[1,2-a]pyridin-3-yl]methanol;
● [6-Bromo-2-(4-bromophenyl)imidazo[1,2-a]pyridin-3-yl]methanol;
● [2-(4-Bromophenyl)-6-chloroimidazo[1,2-a]pyridin-3-yl]methanol;
● [2-(4-Chlorophenyl)-6-fluoroimidazo[1,2-a]pyridin-3-yl]methanol;
● [6-Bromo-2-(2,4-difluorophenyl)imidazo[1,2-a]pyridin-3-yl]methanol;
● [6-Chloro-2-(2,4-difluorophenyl)imidazo[1,2-a]pyridin-3-yl]methanol;
● [6-Bromo-2-(2,4-dichlorophenyl)imidazo[1,2-a]pyridin-3-yl]methanol;
● [6-Chloro-2-(2,4-dichlorophenyl)imidazo[1,2-a]pyridin-3-yl]methanol;
● [6-Bromo-2-(3,4-difluorophenyl)imidazo[1,2-a]pyridin-3-yl]methanol;
● [6-Bromo-2-(3-chloro-4-fluorophenyl)imidazo[1,2-a]pyridin-3-yl]methanol;
● [6-Chloro-2-(3-chloro-4-fluorophenyl)imidazo[1,2-a]pyridin-3-yl]methanol;
● [6,8-Dichloro-2-(3-methoxyphenyl)imidazo[1,2-a]pyridin-3-yl]methanol;
● [2-(4-Bromophenyl)-6,8-dichloroimidazo[1,2-a]pyridin-3-yl]methanol;
● 2-(4-Bromophenyl)-3-(hydroxymethyl)imidazo[1,2-a]pyridine-6-carbonitrile;
● Methyl 2-(4-bromophenyl)-3-(hydroxymethyl)imidazo[1,2-a]pyridine-6-carboxylate;
● Methyl 2-(4-chlorophenyl)-3-(hydroxymethyl)imidazo[1,2-a]pyridine-6-carboxylate;
● [2-(4-Bromophenyl)imidazo[1,2-a]pyridine-3,7-diyl]dimethanol;
● [2-(4-Chlorophenyl)imidazo[1,2-a]pyridine-3,6-diyl]dimethanol;
● [2-(4-Chlorophenyl)-6-nitroimidazo[1,2-a]pyridin-3-yl]methanol;
● [2-(4-Bromophenyl)-6-nitroimidazo[1,2-a]pyridin-3-yl]methanol;
● {2-(4-Chlorophenyl)-6-[(4-methoxypiperidin-1-yl)carbonyl]imidazo[1,2-a]pyridin-3-yl}methanol;
● 2-(4-Chlorophenyl)-3-(hydroxymethyl)-N-(3-methoxypropyl)imidazo[1,2-a]pyridine-6-carboxamide;
● 2-(4-Chlorophenyl)-3-(hydroxymethyl)-N-(2-methoxyethyl)imidazo[1,2-a]pyridine-6-carboxamide;
● [2-(4-Chlorophenyl)-6-(morpholin-4-ylcarbonyl)imidazo[1,2-a]pyridin-3-yl]methanol;
● 2-(4-Chlorophenyl)-3-(hydroxymethyl)-N,N-dimethylimidazo[1,2-a]pyridine-6-carboxamide;
● 2-(4-Chlorophenyl)-3-(hydroxymethyl)-N-methylimidazo[1,2-a]pyridine-6-carboxamide;
● 2-(4-Chlorophenyl)-3-(hydroxymethyl)-N-[2-(1-methylpyrrolidin-2-yl)ethyl]imidazo[1,2-a]pyridine-6-carboxamide;
● {2-(4-Chlorophenyl)-6-[(4-methylpiperazin-1-yl)carbonyl]imidazo[1,2-a]pyridin-3-yl}methanol;
● 2-(4-Chlorophenyl)-N-(3,4-dimethoxybenzyl)-3-(hydroxymethyl)imidazo[1,2-a]pyridine-6-carboxamide;
● 2-(4-Chlorophenyl)-3-(hydroxymethyl)-N-[2-(1H-imidazol-4-yl)ethyl]imidazo[1,2-a]pyridine-6-carboxamide;
● 2-(4-Chlorophenyl)-3-(hydroxymethyl)-N-(pyridin-3-ylmethyl)imidazo[1,2-a]pyridine-6-carboxamide;
● 2-(4-Chlorophenyl)-3-(hydroxymethyl)-N-(3-hydroxypropyl)imidazo[1,2-a]pyridine-6-carboxamide;
● (1-{[2-(4-Chlorophenyl)-3-(hydroxymethyl)imidazo[1,2-a]pyridin-6-yl]carbonyl}piperidin-4-yl)methanol;
● 2-(4-Chlorophenyl)-3-(hydroxymethyl)-N-(2-hydroxypropyl)imidazo[1,2-a]pyridine-6-carboxamide;
● 2-(4-Chlorophenyl)-N-(*trans*-4-hydroxycyclohexyl)-3-(hydroxymethyl)imidazo[1,2-a]pyridine-6-carboxamide;
● 1-{[2-(4-Chlorophenyl)-3-(hydroxymethyl)imidazo[1,2-a]pyridin-6-yl]carbonyl}piperidin-4-ol;
● (3R)-1-{[2-(4-Chlorophenyl)-3-(hydroxymethyl)imidazo[1,2-a]pyridin-6-yl]carbonyl}pyrrolidin-3-ol;
● 1- {[2-(4-Chlorophenyl)-3-(hydroxymethyl)imidazo[1,2-a]pyridin-6-yl]carbonyl}pyrrolidin-3-ol;
● 1-{[2-(4-Chlorophenyl)-3-(hydroxymethyl)imidazo[1,2-a]pyridin-6-yl]carbonyl}azetidin-3-ol;
● 2-(4-Chlorophenyl)-3-(hydroxymethyl)imidazo[1,2-a]pyridine-7-carboxamide;
● 3-(Hydroxymethyl)-2-(3-methoxyphenyl)imidazo[1,2-a]pyridine-6-carboxamide;
● 2-(4-Chlorophenyl)-3-(hydroxymethyl)imidazo[1,2-a]pyridine-6-carboxamide;
● 2-(4-Fluorophenyl)-3-(hydroxymethyl)imidazo[1,2-a]pyridine-6-carboxamide;
● 2-(2,4-Difluorophenyl)-3-(hydroxymethyl)imidazo[1,2-a]pyridine-6-carboxamide;
● 2-(2,4-Dichlorophenyl)-3-(hydroxymethyl)imidazo[1,2-a]pyridine-6-carboxamide;
● 2-(3,4-Difluorophenyl)-3-(hydroxymethyl)imidazo[1,2-a]pyridine-6-carboxamide;
● [6-amino-2-(4-chlorophenyl)imidazo[1,2-a]pyridin-3-yl]methanol;
● N-[2-(4-chlorophenyl)-3-(hydroxymethyl)imidazo[1,2-a]pyridin-6-yl]acetamide;
● [6-amino-2-(4-bromophenyl)imidazo[1,2-a]pyridin-3-yl]methanol;
● [6-chloro-2-(4-chlorophenyl)imidazo[1,2-b]pyridazin-3-yl]methanol;
● [2-(4-Chlorophenyl)imidazo[1,2-a]pyrazin-3-yl]methanol;
● [6-Bromo-2-(3-methoxyphenyl)imidazo[1,2-a]pyrazin-3-yl]methanol;
● {6-Bromo-2-[4-(trifluoromethyl)phenyl]imidazo[1,2-a]pyrazin-3-yl}methanol;
● [6-Bromo-2-(4-fluorophenyl)imidazo[1,2-a]pyrazin-3-yl]methanol;
● [6-Bromo-2-(4-chlorophenyl)imidazo[1,2-a]pyrazin-3-yl]methanol;
● [6-Bromo-2-(4-bromophenyl)imidazo[1,2-a]pyrazin-3-yl]methanol;
● [6-Bromo-2-(2,4-dichlorophenyl)imidazo[1,2-a]pyrazin-3-yl]methanol;
● [6-Bromo-2-(2,4-difluorophenyl)imidazo[1,2-a]pyrazin-3-yl]methanol;
● [6-Bromo-2-(4-chloro-2-fluoro-5-methylphenyl)imidazo[1,2-a]pyrazin-3-yl]methanol;
● [2-(1-Benzofuran-5-yl)-6-bromoimidazo[1,2-a]pyrazin-3-yl]methanol;
● [6-Bromo-2-(2,3-dihydro-1,4-benzodioxin-5-yl)imidazo[1,2-a]pyrazin-3-yl]methanol;
● [6-amino-2-(4-fluorophenyl)imidazo[1,2-a]pyrazin-3-yl]methanol;
● [6-amino-2-(4-chlorophenyl)imidazo[1,2-a]pyrazin-3-yl]methanol;
● [6-Amino-2-(4-bromophenyl)imidazo[1,2-a]pyrazin-3-yl]methanol;
● [6-(Azetidin-1-yl)-2-(4-fluorophenyl)imidazo[1,2-a]pyrazin-3-yl]methanol;
● [2-(4-Chlorophenyl)imidazo[1,2-a]pyrimidin-3-yl]methanol;
● [2-(2,4-Dichlorophenyl)imidazo[1,2-a]pyrimidin-3-yl]methanol;
● [6-(4-fluorophenyl)-2-methylimidazo[2,1-b][1,3]oxazol-5-yl]methanol;
● [6-(2,4-dichlorophenyl)imidazo[2, 1-b][1,3]thiazol-5-yl]methanol;
● [6-(4-Bromophenyl)-2-methylimidazo[2,1-b][1,3]thiazol-5-yl]methanol;
● [6-(2,4-Dichlorophenyl)-2-methylimidazo[2,1-b][1,3]thiazol-5-yl]methanol;
● [2-Chloro-6-(4-chlorophenyl)imidazo[2,1-b][1,3]thiazol-5-yl]methanol;
● Methyl 6-(4-chlorophenyl)-5-(hydroxymethyl)imidazo[2,1-b][1,3]thiazole-2-carboxylate;
● [6-(4-Chlorophenyl)imidazo[2,1-b][1,3]thiazole-2,5-diyl]dimethanol;
● 1-[6-(4-Chlorophenyl)-5-(hydroxymethyl)imidazo[2,1-b][1,3]thiazol-2-yl]ethanol;
● [6-(4-Chlorophenyl)-5-(hydroxymethyl)imidazo[2,1-b][1,3]thiazol-2-yl](cyclopropyl)methanol;
● 2-[6-(4-Chlorophenyl)-5-(hydroxyrnethyl)imidazo[2,1-b][1,3]thiazol-2-yl]propan-2-ol;
● 6-(4-Chlorophenyl)-N-ethyl-5-(hydroxymethyl)-N-methylimidazo[2,1-b] [1,3]thiazole-2-carboxamide;
● [6-(4-Chlorophenyl)-2-(morpholin-4-ylcarbonyl)imidazo[2,1-b][1,3]thiazol-5-yl]methanol;
● {6-(4-Chlorophenyl)-2-[(4-methylpiperazin-1-yl)carbonyl]imidazo[2,1-b][1,3]thiazol-5-yl}methanol; and
● 6-(4-Chlorophenyl)-5-(hydroxymethyl)-N-propylimidazo[2,1-b][1,3]thiazole-2-carboxamide.

7. A pharmaceutical formulation comprising a compound according to any one of claims 1 to 6 as an active ingredient, in combination with a pharmaceutically acceptable diluent or carrier.

8. A compound according to any one of claims 1 to 6 for use in therapy.

9. A compound of formula (I), 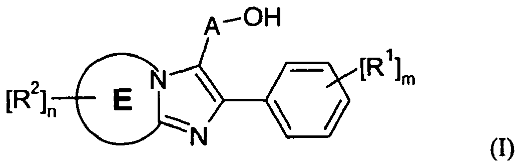 or a pharmaceutically acceptable salt, solvate, hydrate, geometrical isomer, tautomer, optical isomer or N-oxide thereof, wherein:
E is a 5- or 6-membered heteroaromatic ring;
A is C₁₋₃-alkylene, which is optionally substituted with C₁₋₃-alkyl;
each R¹ is independently selected from the group consisting of halogen, cyano, nitro, C₁₋₄-alkyl, fluoro-C₁₋₄-alkyl, C₃₋₆-cycloalkyl, heterocyclyl, phenyl, heteroaryl, -C₀₋₄-alkylene-OR³, -C₀₋₄-alkylene-SR³, -C₀₋₄-alkylene-NR^{4A}R^{4B}, -N(R⁵)-C₂₋₄-alkylene-NR^{4A}R^{4B}, -C(O)R³, -C(O)OR³, -C(O)NR^{4A}R^{4B}, -N(R⁵)C(O)R⁶, -N(R⁵)C(O)NR^{4A}R^{4B}, -N(R⁵)C(O)OR⁶, -N(R⁵)S(O)₂R⁶, -S(O)₂NR^{4A}R^{4B} and -S(O)₂R⁶, and wherein any ring residue is optionally substituted with one or more substituents independently selected from halogen, cyano, hydroxy, C₁₋₄-alkyl, C₁₋₄-alkoxy, trifluoromethyl and trifluoromethoxy;
or two neighbouring substituents R', together with the carbon atoms to which they are attached, form a 5- or 6-membered aromatic or partially unsaturated ring, which optionally contains one or two heteroatoms selected from nitrogen, sulphur or oxygen, and which ring is optionally substituted with one or more substituents independently selected from halogen, cyano, hydroxy, C₁₋₄-alkyl, C₁₋₄-alkoxy,
trifluoromethyl and trifluoromethoxy;
each R² is independently selected from the group consisting of halogen, cyano, nitro, C₁₋₄-alkyl, fluoro-C₁₋₄-alkyl, C₃₋₆-cycloalkyl, heterocyclyl, phenyl, heteroaryl, C₃₋₆-cycloalkyl-C₁₋₄-alkyl, C₃₋₆-cycloalkyl(hydroxy)C₁₋₄-alkyl, heterocyclyl-C₁₋₄-alkyl, C₃₋₆-cycloalkylcarbonyl, heterocyclylcarbonyl, benzoyl, heteroarylcarbonyl, -C₀₋₄-alkylene-OR³, -C₀₋₄-alkylene-SR³, -C₀₋₄-alkylene-NR^{7A}R^{7B}, -C₀₋₄-alkylene-C(O)R⁶, -C₀₋₄-alkylene-C(O)OR³, -C₀₋₄-alkylene-C(O)NR^{7A}R^{7B}, -C₀₋₄-alkylene-N(R⁵)C(O)R⁶, -C₀₋₄-alkylene-N(R⁵)C(O)NR^{7A}R^{7B}, -C₀₋₄-alkylene-N(R⁵)C(O)OR⁶, -C₀₋₄-alkylene-N(R⁵)S(O)₂R⁶, -C₀₋₄-alkylene-S(O)₂NR^{7A}R^{7B}, -C₀₋₄-alkylene-S(O)₂R⁶, amidino and guanidino, and wherein any ring residue is optionally substituted with one or more substituents independently selected from halogen, cyano, hydroxy, C₁₋₄-alkyl, C₁₋₄-alkoxy, trifluoromethyl, trifluoromethoxy and -C(O)NR^{8A}R^{8B};
each R³ is independently selected from hydrogen, C₁₋₄-alkyl, fluoro-C₁₋₄-alkyl, hydroxy-C₁₋₄-alkyl and C₃₋₆-cycloalkyl;
each R^{4A} and R^{4B} is independently selected from hydrogen, C₁₋₄-alkyl, hydroxy-C₁₋₄-alkyl and -C₁₋₄-alkylene-NR^{8A}R^{8B};
or independently R^{4A} and R^{4B} together with the nitrogen atom to which they are attached form a 4- to 6-membered saturated heterocyclic ring, which is optionally substituted with one or more substituents independently selected from halogen, hydroxy, C₁₋₄-alkyl, C₁₋₄-alkoxy, trifluoromethyl and trifluoromethoxy;
each R⁵ is independently selected from hydrogen and C₁₋₄-alkyl;
each R⁶ is independently selected from C₁₋₄-alkyl, fluoro-C₁₋₄-alkyl, hydroxy-C₁₋₄-alkyl and C₃₋₆-cycloalkyl;
each R^{7A} and R^{7B} is independently selected from the group consisting of hydrogen, C₁₋₄-alkyl, hydroxy-C₁₋₄-alkyl, C₁₋₄-alkoxy-C₁₋₄-alkyl, C₃₋₆-cycloalkyl, phenyl-C₁₋₄-alkyl, heteroaryl-C₁₋₄-alkyl, C₃₋₆-cycloalkyl-C₁₋₄-alkyl and heterocyclyl-C₁₋₄-alkyl, and wherein any ring residue is optionally substituted with one or more substituents independently selected from halogen, cyano, hydroxy, C₁₋₄-alkyl, C₁₋₄-alkoxy, trifluoromethyl and trifluoromethoxy;
or independently R^{7A} and R^{7B} together with the nitrogen atom to which they are attached, form a 4- to 6-membered saturated heterocyclic ring, which optionally contains an additional heteroatom selected from nitrogen, sulphur or oxygen, and
which ring is optionally substituted with one or more substituents independently selected from halogen, hydroxy, C₁₋₄-alkyl, C₁₋₄-alkoxy, hydroxy-C₁₋₄-alkyl, -S(O)₂NR^{8A}R^{8B} and -C(O)NR^{8A}R^{8B};
each R^{8A} and R^{8B} is independently selected from hydrogen and C₁₋₄-alkyl;
m is 1, 2, 3 or 4; and
n is 0, 1 or 2,
for use in the treatment or prevention of inflammation, an inflammatory disease, an immune or an autoimmune disorder.

10. A compound according to claim 9, wherein the inflammation or inflammatory disease or immune or autoimmune disorder is arthritis (including rheumatoid arthritis, juvenile rheumatoid arthritis, osteoarthritis and psoriatic arthritis), synovitis, vasculitis, a condition associated with inflammation of the bowel (including Crohn's disease, ulcerative colitis, inflammatory bowel disease and irritable bowel syndrome), atherosclerosis, multiple sclerosis, Alzheimer's disease, vascular dementia, a pulmonary inflammatory disease (including asthma, chronic obstructive pulmonary disease and acute respiratory distress syndrome), a fibrotic disease (including idiopathic pulmonary fibrosis, cardiac fibrosis and systemic sclerosis (scleroderma)), an inflammatory disease of the skin (including contact dermatitis, atopic dermatitis and psoriasis), systemic inflammatory response syndrome, sepsis, an inflammatory and/or autoimmune condition of the liver (including autoimmune hepatitis, primary biliary cirrhosis, alcoholic liver disease, sclerosing cholangitis, and autoimmune cholangitis), diabetes (type I or II) and/or the complications thereof, chronic heart failure, congestive heart failure, an ischemic disease (including stroke and ischemia-reperfusion injury) or myocardial infarction and/or the complications thereof.

11. A compound according to claim 9, wherein the inflammatory disease is vasculitis.

## Patentansprüche

1. Verbindung der Formel (I), 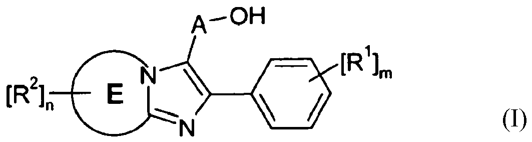 oder ein pharmazeutisch verträgliches Salz, Solvat, Hydrat, geometrisches Isomer, Tautomer, optisches Isomer oder N-Oxid davon, wobei:
E ein 5- oder 6-gliedriger heteroaromatischer Ring ist;
A C₁₋₃-Alkylen ist, das gegebenenfalls mit C₁₋₃-Alkyl substituiert ist;
jedes R¹ unabhängig aus der Gruppe ausgewählt ist, bestehend aus Halogen, Cyano, Nitro, C₁₋₄-Alkyl, Fluor-C₁₋₄-alkyl, C₃₋₆-Cycloalkyl, Heterocyclyl, Phenyl, Heteroaryl, -C₀₋₄-Alkylen-OR³, -C₀₋₄-Alkylen-SR³, -C₀₋₄-Alkylen-NR^{4A}R^{4B}, -N(R⁵)-C₂₋₄-Alkylen-NR^{4A}R^{4B}, -C(O)R³, -C(O)OR³, -C(O)NR^{4A}R^{4B}, -N(R⁵)C(O)R⁶, -N(R⁵)C(O)NR^{4A}R^{4B}, -N(R⁵)C(O)OR⁶, -N(R⁵)S(O)₂R⁶, -S(O)₂NR^{4A}R^{4B} und -S(O)₂R⁶, und wobei jedweder Ringrest gegebenenfalls mit einem oder mehr Substituenten substituiert ist, der/die unabhängig aus Halogen, Cyano, Hydroxy, C₁₋₄-Alkyl, C₁₋₄-Alkoxy, Trifluormethyl und Trifluormethoxy ausgewählt ist/sind;
oder zwei benachbarte Substituenten R¹, zusammen mit den Kohlenstoffatomen, an die sie angelagert sind, einen 5- oder 6-gliedrigen aromatischen oder teilungesättigen Ring bilden, der gegebenenfalls ein oder zwei Heteroatom(e) enthält, der/die aus Stickstoff, Schwefel oder Sauerstoff ausgewählt ist/sind, und welcher Ring gegebenenfalls mit einem oder mehr Substituenten substituiert ist, der/die unabhängig aus Halogen, Cyano, Hydroxy, C₁₋₄-Alkyl, C₁₋₄-Alkoxy, Trifluormethyl und Trifluormethoxy ausgewählt ist/sind;
jedes R² unabhängig aus der Gruppe ausgewählt ist, bestehend aus Halogen, Cyano, Nitro, C₁₋₄-Alkyl, Fluor-C₁₋₄-alkyl, C₃₋₆-Cycloalkyl, Heterocyclyl, Phenyl, Heteroaryl, C₃₋₆-Cycloalkyl-C₁₋₄₋alkyl, C₃₋₆-Cycloalkyl(hydroxy)-C₁₋₄-alkyl, Heterocyclyl-C₁₋₄-alkyl, C₃₋₆-Cycloalkylcarbonyl, Heterocyclylcarbonyl, Benzoyl, Heteroarylcarbonyl, -C₀₋₄-Alkylen-OR³, -C₀₋₄-Alkylen-SR³, -C₀₋₄-Alkylen-NR^{7A}R^{7B}, -C₀₋₄-Alkylen-C(O)R⁶, -C₀₋₄-Alkylen-C(O)OR³, -C₀₋₄-Alkylen-C(O)NR^{7A}R^{7B}, -C₀₋₄-Alkylen-N(R⁵)C(O)R⁶, -C₀₋₄-Alkylen-N(R⁵)C(O)NR^{7A}R^{7B}, -C₀₋₄-Alkylen-N(R⁵)C(O)OR⁶, -C₀₋₄-Alkylen-N(R⁵)S(O)₂R⁶, -C₀₋₄-Alkylen-S(O)₂NR^{7A}R^{7B}, -C₀₋₄-Alkylen-S(O)₂R⁶, Amidino und Guanidino, und wobei jeder Ringrest gegebenenfalls mit einem oder mehr Substituenten substituiert ist, der/die unabhängig aus Halogen, Cyano, Hydroxy, C₁₋₄-Alkyl, C₁₋₄-Alkoxy, Trifluormethyl, Trifluormethoxy und -C(O)NR^{8A}R^{8B} ausgewählt ist/sind;
jedes R³ aus Wasserstoff, C₁₋₄-Alkyl, Fluor-C₁₋₄-alkyl, Hydroxy-C₁₋₄-alkyl und C₃₋₆-Cycloalkyl ausgewählt ist;
jedes R^{4A} und R^{4B} unabhängig aus Wasserstoff, C₁₋₄-Alkyl, Hydroxy-C₁₋₄-alkyl und -C₁₋₄-Alkylen-NR^{8A}R^{8B} ausgewählt ist;
oder R^{4A} und R^{4B} unabhängig, zusammen mit dem Stickstoffatom, an das sie angelagert sind, einen 4- bis 6-gliedrigen gesättigten heterocyclischen Ring bilden, der gegebenenfalls mit einem oder mehr Substituenten substituiert ist, der/die unabhängig aus Halogen, Hydroxy, C₁₋₄-Alkyl, C₁₋₄-Alkoxy, Trifluormethyl und Trifluormethoxy ausgewählt ist/sind;
jedes R⁵ unabhängig aus Wasserstoff und C₁₋₄-Alkyl ausgewählt ist;
jedes R⁶ unabhängig aus C₁₋₄-Alkyl, Fluor-C₁₋₄-alkyl, Hydroxy-C₁₋₄-alkyl und C₃₋₆-Cycloalkyl ausgewählt ist;
jedes R^{7A} und R^{7B} unabhängig aus der Gruppe ausgewählt ist, bestehend aus Wasserstoff, C₁₋₄-Alkyl, Hydroxy-C₁₋₄-alkyl, C₁₋₄-Alkoxy-C₁₋₄-alkyl, C₃₋₆-Cycloalkyl, Phenyl-C₁₋₄-alkyl, Heteroaryl-C₁₋₄-alkyl, C₃₋₆-Cycloalkyl-C₁₋₄-alkyl und Heterocyclyl-C₁₋₄-alkyl, und wobei jeder Ringrest gegebenenfalls mit einem oder mehr Substituenten substituiert ist, der/die unabhängig aus Halogen, Cyano, Hydroxy, C₁₋₄-Alkyl, C₁₋₄-Alkoxy, Trifluormethyl und Trifluormethoxy ausgewählt ist/sind;
oder R^{7A} und R^{7B} unabhängig, zusammen mit dem Stickstoffatom, an das sie angelagert sind, einen 4- bis 6-gliedrigen gesättigten heterocyclischen Ring bilden, der gegebenenfalls ein zusätzliches Heteroatom enthält, das aus Stickstoff, Schwefel oder Sauerstoff ausgewählt ist, und welcher Ring gegebenenfalls mit einem oder mehr Substituenten substituiert ist, der/die unabhängig aus Halogen, Hydroxy, C₁₋₄-Alkyl, C₁₋₄-Alkoxy, Hydroxy-C₁₋₄-alkyl, -S(O)₂NR^{8A}R^{8B} und -C(O)NR^{8A}R^{8B} ausgewählt ist/sind;
jedes R^{8A} und R^{8B} unabhängig aus Wasserstoff und C₁₋₄-Alkyl ausgewählt ist;
m 1, 2, 3 oder 4 ist; und
n 0, 1 oder 2 ist;
mit der Maßgabe, dass die Verbindung nicht aus der Gruppe ausgewählt ist, bestehend aus:
● 4-[3-(Hydroxymethyl)-6-methylimidazo[1,2-a]pyridin-2-yl]-benzonitril;
● 2-(4-Ethylphenyl)-6-methyl-imidazo[1,2-a]pyridin-3-methanol;
● 2-(2-Nitrophenyl)-imidazo[1,2-a]pyridin-3-methanol;
● 2-(3,4-Dimethylphenyl)-6-methyl-imidazo[1,2-a]pyridin-3-methanol;
● 6-Chlor-2-(4-chlorphenyl)-imidazo[1,2-a]pyridin-3-methanol;
● 2-(4-Fluorphenyl)-imidazo[1,2-a]pyridin-3-methanol;
● 2-(2-Chlor-4-methoxyphenyl)-6-(1-ethylpropyl)-8-methyl-imidazo[1,2-a]pyridin-3-methanol;
● 2-(4-Chlorphenyl)-imidazo[1,2-a]pyridin-3-propanol;
● 2-(4-Methylphenyl)-6-(trifluormethyl)-imidazo[1,2-a]pyridin-3-methanol;
● 7-Methyl-2-(2-Naphthalenyl)-imidazo[1,2-a]pyridin-3-methanol;
● 2-(4-Methylphenyl)-6-nitro-imidazo[1,2-a]pyridin-3-methanol;
● 2-[1,1'-Biphenyl]-4-yl-7-methyl-imidazo[1,2-a]pyridin-3-methano;
● 6-Methyl-2-[4-(trifluormethyl)phenyl]-imidazo[1,2-a]pyridin-3-methanol;
● 2-(4-Chlorphenyl)-imidazo[1,2-a]pyridin-3-methanol;
● 6-Methyl-2-(4-methylphenyl)-imidazo[1,2-a]pyridin-3-ethanol;
● 6-Methyl-2-(4-methylphenyl)-imidazo[1,2-a]pyridin-3-methanol;
● Ethyl-4-[3-(hydroxymethyl)-6-methylimidazo[1,2-a]pyridin-2-yl]-benzoat;
● 2-[4-(Methylsulfonyl)phenyl]-imidazo[1,2-a]pyridin-3-methanol;
● 2-(4-Chlorphenyl)-6-methyl-imidazo[1,2-a]pyridin-3-methanol;
● 6-Methyl-2-(4-nitrophenyl)-imidazo[1,2-a]pyridin-3-methanol;
● 6-Chlor-2-(4-methylphenyl)-imidazo[1,2-b]pyridazin-3-methanol;
● 3-(Diethylamino)-2-methyl-6-(4-methylphenyl)-imidazo[1,2-b][1,2,4]triazin-7-methanol;
● 6-(4-Chlorphenyl)-2,3-diphenyl-imidazo[1,2-b][1,2,4]triazin-7-methanol;
● 6-(4-Bromphenyl)-imidazo[2,1-b]thiazol-5-methanol;
● 6-(4-Chlorphenyl)-imidazo[2,1-b]thiazol-5-methanol;
● 6-(4-Chlorphenyl)-2-methyl-imidazo[2,1-b]thiazol-5-methanol;
● 6-(4-Methylphenyl)-imidazo[2,1-b]thiazol-5-methanol;
● 6-[l,l'-Biphenyl]-4-yl-imidazo[2,1-b]thiazol-5-methanol;
● 6-(4-Bromphenyl)-2-(2-furanyl)-imidazo[2,1-b]-1,3,4-thiadiazol-5-methanol;
● 6-(4-Bromphenyl)-2-(2-thienyl)-imidazo[2,1-b]-1,3,4-thiadiazol-5-methanol; und
● 6-(4-Bromphenyl)-2-cyclohexyl-imidazo[2,1-b]-1,3,4-thiadiazol-5-methanol.

2. Verbindung nach Anspruch 1, wobei der heteroaromatische Ring E ausgewählt ist aus:

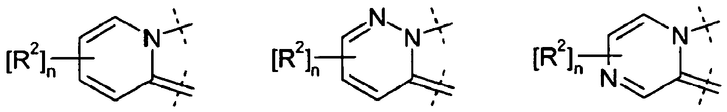

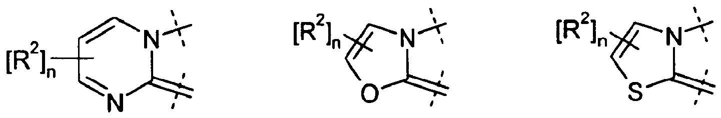

3. Verbindung nach Anspruch 1 oder 2, wobei A -CH₂- ist.

4. Verbindung nach einem der Ansprüche 1 bis 3, wobei R¹ unabhängig aus Halogen, C₁₋₄-Alkyl, Fluor-C₁₋₄-alkyl und C₁₋₄-Alkoxy ausgewählt ist.

5. Verbindung nach einem der Ansprüche 1 bis 4, wobei R² unabhängig aus Halogen, Cyano, Nitro, C₁₋₄-Alkyl, Fluor-C₁₋₄-alkyl, Hydroxy-C₁₋₄-alkyl und C₁₋₄-Alkoxycarbonyl und -C(O)NR^{7A}R^{7B} ausgewählt ist.

6. Verbindung nach Anspruch 1, die ausgewählt ist aus:
• [2-(4-Methylphenyl)imidazo[1,2-a]pyridin-3-yl]methanol,
• [2-(2,4-Dichlorphenyl)imidazo[1,2-a]pyridin-3-yl]methanol,
• [2-(4-Bromphenyl)-8-methylimidazo[1,2-a]pyridin-3-yl]methanol;
• [7-Methyl-2-(4-methylphenyl)imidazo[1,2-a]pyridin-3-yl]methanol,
• [2-(4-Bromphenyl)-7-methylimidazo[1,2-a]pyridin-3-yl]methanol;
• [2-(4-Bromphenyl)-7-ethylimidazo[1,2-a]pyridin-3-yl]methanol;
• [2-(2-Chlorphenyl)-7-methylimidazo[1,2-a]pyridin-3-yl]methanol;
• [2-(2,4-Dichlorphenyl)-7-methylimidazo[1,2-a]pyridin-3-yl]methanol;
• [2-(3,4-Dichlorphenyl)-7-methylimidazo[1,2-a]pyridin-3-yl]methanol;
• [6-Methyl-2-(2-naphthyl)imidazo[1,2-a]pyridin-3-yl]methanol;
• [2-(3-Methoxyphenyl)-6-methylimidazo[1,2-a]pyridin-3-yl]methanol;
• 4-[3-(Hydroxymethyl)-6-methylimidazo[1,2-a]pyridin-2-yl]benzonitril;
• [6-Methyl-2-(3-nitrophenyl)imidazo[1,2-a]pyridin-3-yl]methanol;
• [2-(4-Fluorphenyl)-6-methylimidazo[1,2-a]pyridin-3-yl]methanol;
• [2-(4-Iodphenyl)-6-methylimidazo[1,2-a]pyridin-3-yl]methanol;
• [2-(2-Chlorphenyl)-6-methylimidazo[1,2-a]pyridin-3-yl]methanol;
• (2-{4-[(2-Aminoethyl)amino]phenyl}-6-methylimidazo[1,2-a]pyridin-3-yl)methanol;
• 1-[2-(4-Chlorphenyl)-6-methylimidazo[1,2-a]pyridin-3-yl]ethanol;
• [2-(2,4-Dichlorphenyl)-6-methylimidazo[1,2-a]pyridin-3-yl]methanol;
• [2-(3-Methoxyphenyl)-6-(trifluormethyl)imidazo[1,2-a]pyridin-3-yl]methanol;
• [2-(4-Chlorphenyl)-6-(trifluormethyl)imidazo[1,2-a]pyridin-3-yl]methanol;
• [2-(4-Bromphenyl)-6-(trifluormethyl)imidazo[1,2-a]pyridin-3-yl]methanol;
• [7-Chlor-2-(4-chlorphenyl)imidazo[1,2-a]pyridin-3-yl]methanol;
• [2-(4-Bromphenyl)-7-chlorimidazo[1,2-a]pyridin-3-yl]methanol;
• [7-Chlor-2-(2,4-dichlorphenyl)imidazo[1,2-a]pyridin-3-yl]methanol;
• [7-Chlor-2-(2,4-difluorphenyl)imidazo[1,2-a]pyridin-3-yl]methanol;
• [6-Brom-2-(3-methoxyphenyl)imidazo[1,2-a]pyridin-3-yl]methanol;
• [6-Brom-2-(4-fluorphenyl)imidazo[1,2-a]pyridin-3-yl]methanol;
• [6-Brom-2-(4-bromphenyl)imidazo[1,2-a]pyridin-3-yl]methanol;
• [2-(4-Bromphenyl)-6-chlorimidazo[1,2-a]pyridin-3-yl]methanol;
• [2-(4-Chlorphenyl)-6-fluorimidazo[1,2-a]pyridin-3-yl]methanol;
• [6-Brom-2-(2,4-difluorphenyl)imidazo[1,2-a]pyridin-3-yl]methanol;
• [6-Chlor-2-(2,4-difluorphenyl)imidazo[1,2-a]pyridin-3-yl]methanol;
• [6-Brom-2-(2,4-dichlorphenyl)imidazo[1,2-a]pyridin-3-yl]methanol;
• [6-Chlor-2-(2,4-dichlorphenyl)imidazo[1,2-a]pyridin-3-yl]methanol;
• [6-Brom-2-(3,4-difluorphenyl)imidazo[1,2-a]pyridin-3-yl]methanol;
• [6-Brom-2-(3-chlor-4-fluorphenyl)imidazo[1,2-a]pyridin-3-yl]methanol;
• [6-Chlor-2-(3-chlor-4-fluorphenyl)imidazo[1,2-a]pyridin-3-yl]methanol;
• [6,8-Dichlor-2-(3-methoxyphenyl)imidazo[1,2-a]pyridin-3-yl]methanol;
• [2-(4-Bromphenyl)-6,8-dichlorimidazo[1,2-a]pyridin-3-yl]methanol;
• 2-(4-Bromphenyl)-3-(hydroxymethyl)imidazo[1,2-a]pyridin-6-carbonitril;
• Methyl-2-(4-bromphenyl)-3-(hydroxymethyl)imidazo[1,2-a]pyridin-6-carboxylat;
• Methyl-2-(4-chlorphenyl)-3-(hydroxymethyl)imidazo[1,2-a]pyridin-6-carboxylat;
• [2-(4-Bromphenyl)imidazo[1,2-a]pyridin-3,7-diyl]dimethanol;
• [2-(4-Chlorphenyl)imidazo[1,2-a]pyridin-3,6-diyl]dimethanol;
• [2-(4-Chlorphenyl)-6-nitroimidazo[1,2-a]pyridin-3-yl]methanol;
• [2-(4-Bromphenyl)-6-nitroimidazo[1,2-a]pyridin-3-yl]methanol;
• [2-(4-Chlorphenyl)-6-[(4-methoxypiperidin-1-yl)carbonyl]imidazo[1,2-a]pyridin-3-yl} methanol;
• 2-(4-Chlorphenyl)-3-(hydroxymethyl)-N-(3-methoxypropyl)imidazo[1,2-a]pyridin-6-carboxamid;
• 2-(4-Chlorphenyl)-3-(hydroxymethyl)-N-(2-methoxyethyl)imidazo[1,2-a]pyridin-6-carboxamid;
• [2-(4-Chlorphenyl)-6-(morpholin-4-ylcarbonyl)imidazo[1,2-a]pyridin-3-yl]methanol;
• 2-(4-Chlorphenyl)-3-(hydroxymethyl)-N,N-dimethylimidazo[1,2-a]pyridin-6-carboxamid;
• 2-(4-Chlorphenyl)-3-(hydroxymethyl)-N-methylimidazo[1,2-a]pyridin-6-carboxamid;
• 2-(4-Chlorphenyl)-3-(hydroxymethyl)-N-[2-(1-methylpyrrolidin-2-yl)ethyl]imidazo[1,2-a]pyridin-6-carboxamid;
• {2-(4-Chlorphenyl)-6-[(4-methylpiperazin-1-yl)carbonyl]imidazo[1,2-a]pyridin-3-yl} methanol;
• 2-(4-Chlorphenyl)-N-(3,4-dimethoxybenzyl)-3-(hydroxymethyl)imidazo[1,2-a]pyridin-6-carboxamid;
• 2-(4-Chlorphenyl)-3-(hydroxymethyl)-N-[2-(1H-imidazol-4-yl)ethyl]imidazo[1,2-a]-pyridin-6-carboxamid;
• 2-(4-Chlorphenyl)-3-(hydroxymethyl)-N-(pyridin-3-ylmethyl)imidazo[1,2-a]pyridin-6-carboxamid;
• 2-(4-Chlorphenyl)-3-(hydroxymethyl)-N-(3-hydroxypropyl)imidazo[1,2-a]pyridin-6-carboxamid;
• (1-{[2-(4-Chlorphenyl)-3-(hydroxymethyl)imidazo[1,2-a]pyridin-6-yl]carbonyl}-piperidin-4-yl)methanol;
• 2-(4-Chlorphenyl)-3-(hydroxymethyl)-N-(2-hydroxypropyl)imidazo[1,2-a]pyridin-6-carboxamid;
• 2-(4-Chlorphenyl)-N-(*trans*-4-hydroxycyclohexyl)-3-(hydroxymethyl)imidazo[1,2-a]-pyridin-6-carboxamid;
• 1-{[2-(4-Chlorphenyl)-3-(hydroxymethyl)imidazo[1,2-a]pyridin-6-yl]carbonyl}-piperidin-4-ol;
• (3R)-1-{[2-(4-Chlorphenyl)-3-(hydroxymethyl)imidazo[1,2-a]pyridin-6-yl]carbonyl}-pyrrolidin-3-ol;
• 1-{[2-(4-Chlorphenyl)-3-(hydroxymethyl)imidazo[1,2-a]pyridin-6-yl]carbonyl}-pyrrolidin-3-ol;
• 1-{[2-(4-Chlorphenyl)-3-(hydroxymethyl)imidazo[1,2-a]pyridin-6-yl]carbonyl}-azetidin-3-ol;
• 2-(4-Chlorphenyl)-3-(hydroxymethyl)imidazo[1,2-a]pyridin-7-carboxamid;
• 3-(Hydroxymethyl)-2-(3-methoxyphenyl)imidazo[1,2-a]pyridin-6-carboxamid;
• 2-(4-Chlorphenyl)-3-(hydroxymethyl)imidazo[1,2-a]pyridin-6-carboxamid;
• 2-(4-Fluorphenyl)-3-(hydroxymethyl)imidazo[1,2-a]pyridin-6-carboxamid;
• 2-(2,4-Difluorphenyl)-3-(hydroxymethyl)imidazo[1,2-a]pyridin-6-carboxamid;
• 2-(2,4-Dichlorphenyl)-3-(hydroxymethyl)imidazo[1,2-a]pyridin-6-carboxamid;
• 2-(3,4-Difluorphenyl)-3-(hydroxymethyl)imidazo[1,2-a]pyridin-6-carboxamid;
• [6-Amino-2-(4-chlorphenyl)imidazo[1,2-a]pyridin-3-yl]methanol;
• N-[2-(4-Chlorphenyl)-3-(hydroxymethyl)imidazo[1,2-a]pyridin-6-yl]acetamid;
• [6-Amino-2-(4-bromphenyl)imidazo[1,2-a]pyridin-3-yl]methanol;
• [6-Chlor-2-(4-chlorphenyl)imidazo[1,2-b]pyridazin-3-yl]methanol;
• [2-(4-Chlorphenyl)imidazo[1,2-a]pyrazin-3-yl]methanol;
• [6-Brom-2-(3-methoxyphenyl)imidazo[1,2-a]pyrazin-3-yl]methanol;
• {6-Brom-2-[4-(trifluormethyl)phenyl]imidazo[1,2-a]pyrazin-3-yl} methanol;
• [6-Brom-2-(4-fluorphenyl)imidazo[1,2-a]pyrazin-3-yl]methanol;
• [6-Brom-2-(4-chlorphenyl)imidazo[1,2-a]pyrazin-3-yl]methanol;
• [6-Brom-2-(4-bromphenyl)imidazo[1,2-a]pyrazin-3-yl]methanol;
• [6-Brom-2-(2,4-dichlorphenyl)imidazo[1,2-a]pyrazin-3-yl]methanol;
• [6-Brom-2-(2,4-difluorphenyl)imidazo[1,2-a]pyrazin-3-yl]methanol;
• [6-Brom-2-(4-chlor-2-fluor-5-methylphenyl)imidazo[1,2-a]pyrazin-3-yl]methanol;
• [2-(1-Benzofuran-5-yl)-6-bromimidazo[1,2-a]pyrazin-3-yl]methanol;
• [6-Brom-2-(2,3-dihydro-1,4-benzodioxin-5-yl)imidazo[1,2-a]pyrazin-3-yl]methanol;
• [6-Amino-2-(4-fluorphenyl)imidazo[1,2-a]pyrazin-3-yl]methanol;
• [6-Amino-2-(4-chlorphenyl)imidazo[1,2-a]pyrazin-3-yl]methanol;
• [6-Amin-2-(4-bromphenyl)imidazo[1,2-a]pyrazin-3-yl]methanol;
• [6-(Azetidin-1-yl)-2-(4-fluorphenyl)imidazo[1,2-a]pyrazin-3-yl]methanol;
• [2-(4-Chlorphenyl)imidazo[1,2-a]pyrimidin-3-yl]methanol;
• [2-(2,4-Dichlorphenyl)imidazo[1,2-a]pyrimidin-3-yl]methanol;
• [6-(4-Fluorphenyl)-2-methylimidazo[2,1-b][1,3]oxazol-5-yl]methanol;
• [6-(2,4-Dichlorphenyl)imidazo[2,1-b][1,3]thiazol-5-yl]methanol;
• [6-(4-Bromphenyl)-2-methylimidazo[2,1-b][1,3]thiazol-5-yl]methanol;
• [6-(2,4-Dichlorphenyl)-2-methylimidazo[2,1-b][1,3]thiazol-5-yl]methanol;
• [2-Chlor-6-(4-chlorphenyl)imidazo[2,1-b][1,3]thiazol-5-yl]methanol;
• Methyl-6-(4-chlorphenyl)-5-(hydroxymethyl)imidazo[2,1-b][1,3]thiazol-2-carboxylat;
• [6-(4-Chlorphenyl)imidazo[2,1-b][1,3]thiazol-2,5-diyl]dimethanol;
• 1-[6-(4-Chlorphenyl)-5-(hydroxymethyl)imidazo[2,1-b][1,3]thiazol-2-yl]ethanol;
• [6-(4-Chlorphenyl)-5-(hydroxymethyl)imidazo[2,1-b][1,3]thiazol-2-yl](cyclopropyl)-methanol;
• 2-[6-(4-Chlorphenyl)-5-(hydroxymethyl)imidazo[2,1-b][1,3]thiazol-2-yl]propan-2-ol;
• 6-(4-Chlorphenyl)-N-ethyl-5-(hydroxymethyl)-N-methylimidazo[2,1-b][1,3]thiazol-2-carboxamid;
• [6-(4-Chlorphenyl)-2-(morpholin-4-ylcarbonyl)imidazo[2,1-b][1,3]thiazol-5-yl]methanol;
• {6-(4-Chlorphenyl)-2-[(4-methylpiperazin-1-yl)carbonyl]imidazo[2,1-b][1,3]thiazol-5-yl}methanol; und
• 6-(4-Chlorphenyl)-5-(hydroxymethyl)-N-propylimidazo[2,1-b][1,3]thiazol-2-carboxamid.

7. Pharmazeutische Formulierung, umfassend eine Verbindung nach einem der Ansprüche 1 bis 6 als einen aktiven Bestandteil in Kombination mit einem pharmazeutisch verträglichen Verdünnungsmittel oder Träger.

8. Verbindung nach einem der Ansprüche 1 bis 6 zur Verwendung in der Therapie.

9. Verbindung der Formel (I), 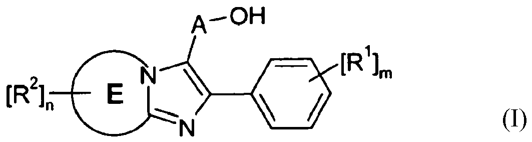 oder ein pharmazeutisch verträgliches Salz, Solvat, Hydrat, geometrisches Isomer, Tautomer, optisches Isomer oder N-Oxid davon, wobei:
E ein 5- oder 6-gliedriger heteroaromatischer Ring ist;
A C₁₋₃-Alkylen ist, das gegebenenfalls mit C₁₋₃-Alkyl substituiert ist;
jedes R¹ unabhängig aus der Gruppe ausgewählt ist, bestehend aus Halogen, Cyano, Nitro, C₁₋₄-Alkyl, Fluor-C₁₋₄-alkyl, C₃₋₆-Cycloalkyl, Heterocyclyl, Phenyl, Heteroaryl, -C₀₋₄-Alkylen-OR³, -C₀₋₄-Alkylen-SR³, -C₀₋₄-Alkylen-NR^{4A}R^{4B}, -N(R⁵)-C₂₋₄-Alkylen-NR^{4A}R^{4B}, -C(O)R³, -C(O)OR³, -C(O)NR^{4A}R^{4B}, -N(R⁵)C(O)R⁶, -N(R⁵)C(O)NR^{4A}R^{4B}, -N(R⁵)C(O)OR⁶, -N(R⁵)S(O)₂R⁶, -S(O)₂NR^{4A}R^{4B} und -S(O)₂R⁶, und wobei jedweder Ringrest gegebenenfalls mit einem oder mehr Substituenten substituiert ist, der/die unabhängig aus Halogen, Cyano, Hydroxy, C₁₋₄-Alkyl, C₁₋₄-Alkoxy, Trifluormethyl und Trifluormethoxy ausgewählt ist/sind;
oder zwei benachbarte Substituenten R¹, zusammen mit den Kohlenstoffatomen, an die sie angelagert sind, einen 5- oder 6-gliedrigen aromatischen oder teilungesättigten Ring bilden, der gegebenenfalls ein oder zwei Heteroatom(e) enthält, der/die aus Stickstoff, Schwefel oder Sauerstoff ausgewählt ist/sind, und welcher Ring gegebenenfalls mit einem oder mehr Substituenten substituiert ist, der/die unabhängig aus Halogen, Cyano, Hydroxy, C₁₋₄-Alkyl, C₁₋₄-Alkoxy, Trifluormethyl und Trifluormethoxy ausgewählt ist/sind;
jedes R² unabhängig aus der Gruppe ausgewählt ist, bestehend aus Halogen, Cyano, Nitro, C₁₋₄-Alkyl, Fluor-C₁₋₄-alkyl, C₃₋₆-Cycloalkyl, Heterocyclyl, Phenyl, Heteroaryl, C₃₋₆-Cycloalkyl-C₁₋₄-alkyl, C₃₋₆-Cycloalkyl(hydroxy)-C₁₋₄-alkyl, Heterocyclyl-C₁₋₄-alkyl, C₃₋₆-Cycloalkylcarbonyl, Heterocyclylcarbonyl, Benzoyl, Heteroarylcarbonyl, -C₀₋₄-Alkylen-OR³, -C₀₋₄-Alkylen-SR³, -C₀₋₄-Alkylen-NR^{7A}R^{7B}, -C₀₋₄-Alkylen-C(O)R⁶, -C₀₋₄-Alkylen-C(O)OR³, -C₀₋₄-Alkylen-C(O)NR^{7A}R^{7B}, -C₀₋₄-Alkylen-N(R⁵)C(O)R⁶, -C₀₋₄-Alkylen-N(R⁵)C(O)NR^{7A}R^{7B}, -C₀₋₄-Alkylen-N(R⁵)C(O)OR⁶, -C₀₋₄-Alkylen-N(R⁵)S(O)₂R⁶, -C₀₋₄-Alkylen-S(O)₂NR^{7A}R^{7B}, -C₀₋₄-Alkylen-S(O)₂R⁶, Amidino und Guanidino, und wobei jedweder Ringrest gegebenenfalls mit einem oder mehr Substituenten substituiert ist, der/die unabhängig aus Halogen, Cyano, Hydroxy, C₁₋₄-Alkyl, C₁₋₄-Alkoxy, Trifluormethyl, Trifluormethoxy und -C(O)NR^{8A}R^{8B} ausgewählt ist/sind;
jedes R³ unabhängig aus Wasserstoff, C₁₋₄-Alkyl, Fluor-C₁₋₄-alkyl, Hydroxy-C₁₋₄-alkyl und C₃₋₆-Cycloalkyl ausgewählt ist;
jedes R^{4A} und R^{4B} unabhängig aus Wasserstoff, C₁₋₄-Alkyl, Hydroxy-C₁₋₄-alkyl und -C₁₋₄-Alkylen-NR^{8A}R^{8B} ausgewählt ist;
oder R^{4A} und R^{4B} unabhängig, zusammen mit dem Stickstoffatom, an das sie angelagert sind, einen 4- bis 6-gliedrigen gesättigten heterocyclischen Ring bilden, der gegebenenfalls mit einem oder mehr Substituenten substituiert ist, der/die unabhängig aus Halogen, Hydroxy, C₁₋₄-Alkyl, C₁₋₄-Alkoxy, Trifluormethyl und Trifluormethoxy ausgewählt ist/sind;
jedes R⁵ unabhängig aus Wasserstoff und C₁₋₄-Alkyl ausgewählt ist;
jedes R⁶ unabhängig aus C₁₋₄-Alkyl, Fluor-C₁₋₄-akyl, Hydroxy-C₁₋₄-alkyl und C₃₋₆-Cycloalkyl ausgewählt ist;
jedes R^{7A} und R^{7B} unabhängig aus der Gruppe ausgewählt ist, bestehend aus Wasserstoff, C₁₋₄-Alkyl, Hydroxy-C₁₋₄-alkyl, C₁₋₄-Alkoxy-C₁₋₄-alkyl, C₃₋₆-Cycloalkyl, Phenyl-C₁₋₄-alkyl, Heteroaryl-C₁₋₄-alkyl, C₃₋₆-Cycloalkyl-C₁₋₄-alkyl und Heterocyclyl-C₁₋₄-alkyl, und wobei jedweder Ringrest gegebenenfalls mit einem oder mehr Substituenten substituiert ist, der/die unabhängig aus Halogen, Cyano, Hydroxy, C₁₋₄-Alkyl, C₁₋₄-Alkoxy, Trifluormethyl und Trifluormethoxy ausgewählt ist/sind;
oder R^{7A} and R^{7B} unabhängig, zusammen mit dem Stickstoffatom, an das sie angelagert sind, einen 4- bis 6-gliedrigen gesättigten Ring bilden, der gegebenenfalls ein zusätzliches Heteroatom enthält, das aus Stickstoff, Schwefel oder Sauerstoff ausgewählt ist, und welcher Ring gegebenenfalls mit einem oder mehr Substituenten substituiert ist, der/die unabhängig aus Halogen, Hydroxy, C₁₋₄-Alkyl, C₁₋₄-Alkoxy, Hydroxy-C₁₋₄-alkyl, -S(O)₂NR^{8A}R^{8B} und -C(O)NR^{8A}R^{8B} ausgewählt ist/sind;
jedes R^{8A} und R^{8B} unabhängig aus Wasserstoff und C₁₋₄-Alkyl ausgewählt ist;
m 1, 2, 3 oder 4 ist; und
n 0, 1 oder 2 ist,
zur Verwendung bei der Behandlung oder Prävention einer Entzündung, einer Entzündungskrankheit, einer Immun- oder einer Autoimmunerkrankung.

10. Verbindung nach Anspruch 9, wobei die Entzündung oder Entzündungskrankheit oder Immun- oder Autoimmunerkrankung Arthritis (einschließlich rheumatoider Arthritis, juveniler rheumatoider Arthritis, Osteoarthritis und psoriatischer Arthritis), Synovitis, Vaskulitis, ein mit einer Entzündung des Darms assoziierter Zustand (einschließlich Morbus Crohn, Colitis ulcerosa, einer entzündlichen Darmerkrankung und eines Reizdarmsyndroms), Atherosklerose, Multiple Sklerose, Alzheimer-Krankheit, vaskuläre Demenz, eine entzündliche Lungenerkrankung (einschließlich Asthma, einer chronischen obstruktiven Lungenkrankheit und eines akuten Atemnotsyndroms), eine fibrotische Erkrankung (einschließlich einer idiopathischen Lungenfibrose, kardialen Fibrose und systemischen Sklerose (Sklerodermie)), eine entzündliche Krankheit der Haut (einschließlich Kontaktdermatitis, atopischer Dermatitis und Psoriasis), ein systemisches inflammatorisches Response-Syndrom, Sepsis, eine Entzündungs- und/oder Autoimmunkrankheit der Leber (einschließlich einer Autoimmunhepatitis, primär biliärer Zirrhose, Alkohol-Lebererkrankung, sklerosierenden Cholangitis und Autoimmuncholangitis), Diabetes (Typ I oder II) und/oder die damit einhergehenden Komplikationen, chronische Herzinsuffizienz, dekompensierte Herzinsuffizienz, eine ischämische Krankheit (einschließlich eines Schlaganfalls und einer ischämischen Reperfusionsverletzung) oder ein Myokardinfarkt und/oder die damit einhergehenden Komplikationen ist.

11. Verbindung nach Anspruch 9, wobei die Entzündungskrankheit Vaskulitis ist.

## Revendications

1. Composé de la formule (1), 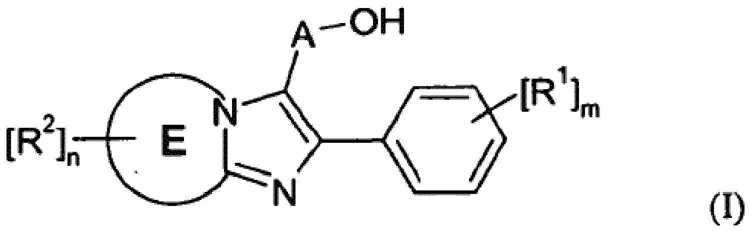 ou un sel pharmaceutiquement acceptable, un solvate, un hydrate, un isomère géométrique, un tautomère, un isomère optique ou un N-oxyde de ces derniers, dans lequel:
E représente un cycle hétéroaromatique à 5 ou 6 chaînons;
A représente alkylène en C₁₋₃, qui est éventuellement substitué par alkyle en C₁₋₃;
chaque R¹ est indépendamment sélectionné parmi le groupe constitué d'halogène, cyano, nitro, alkyle en C₁₋₄, fluoro-alkyle en C₁₋₄, cycloalkyle en C₃₋₆, hétérocyclyle, phényle, hétéroaryle, alkylène en C₀₋₄-OR³, alkylène en C₀₋₄-SR³, alkylène en C₀₋₄-NR^{4A}R^{4B}, -N(R5)-alkylène en C₂₋₄-NR^{4A}R^{4B}, -C(O)R³, -C(O)OR³, -C(O)NR^{4A}R^{4B}, -N(R⁵)C(O)R⁶, -N(R⁵)C(O)NR^{4A}R^{aB}, - N(R⁵)C(O)OR⁶, -N(R⁵)S(O)₂R⁶, -S(O)₂NR^{4A}R^{4B} et -S(O)₂R⁶, et dans lequel tout résidu du cycle est éventuellement substitué par un ou plusieurs substituants indépendamment sélectionnés parmi halogène, cyano, hydroxy, alkyle en C₁₋₄, alkoxy en C₁₋₄, trifluorométhyle et trifluorométhoxy ;
ou deux substituants adjacents R¹, avec les atomes de carbone auxquels ils sont liés, représentent un cycle aromatique ou partiellement insaturé à 5 ou 6 chaînons, qui contient éventuellement un ou deux hétéroatomes sélectionné(s) parmi azote, soufre ou oxygène et lequel cycle est éventuellement substitué par un ou plusieurs substituants indépendamment sélectionné(s) parmi halogène, cyano, hydroxy, alkyle en C₁₋₄, alkoxy en C₁₋₄, trifluorométhyle et trifluorométhoxy ;
chaque R² est indépendamment sélectionné parmi le groupe constitué d'halogène, cyano, nitro, alkyle en C₁₋₄, fluoroalkyle en C₁₋₄, cycloalkyle en C₃₋₆, hétérocyclyle, phényle, hétéroaryle, cycloalkyle en C₃₋₆-alkyle en C₁₋₄, cycloalkyle en C₃₋₆(hydroxy)-alkyle en C₁₋₄, hétérocyclyle-alkyle en C₁₋₄, cycloalkylecarbonyle en C₃₋₆, hétérocyclylcarbonyle, benzoyle, hétéroarylcarbonyle, alkylène en C₀₋₄-OR³, alkylène en C₀₋₄-SR³, alkylène en C₀₋₄-NR^{7A}R^{7B}, alkylène en C₀₋₄-C(O)R⁶, alkylène en C₀₋₄ -C(O)OR³n, alkylène en C₀₋₄-C(O)NR^{7A}R^{7B}, alkylène en C₀₋₄ -N(R⁵)C(O)R⁶, alkylène en C₀₋₄-N(R⁵)C(O)NR^{7A}R^{7B}, alkylène en C₀₋₄-S(O)₂NR^{7A}R^{7B}, alkylène en C₀₋₄-S(O)₂R⁶, amido et guanido, et dans lequel tout résidu du cycle est éventuellement substitué par un ou plusieurs substituants indépendamment sélectionné(s) parmi halogène, cyano hydroxy, alkyle en C₁₋₄, alkoxy en C₁₋₄, trifluorométhyle, trifluorométhoxy et -C(O)NR^{8A}R^{8B};
chaque R³ est sélectionné parmi hydrogène, alkyle en C₁₋₄, fluoro-alkyle en C₁₋₄, hydroxy-alkyle en C₁₋₄ et cycloalkyle en C₃₋₆;
chaque R^{4A} et R^{4B} est indépendamment sélectionné parmi hydrogène, alkyle en C₁₋₄, hydroxy-alkyle en C₁₋₄ et alkylène en C₁₋₄-NR^{8A}R^{8B};
ou indépendamment R^{4A} et R^{4B}, avec l'atome d'azote auquel ils sont respectivement liés, représentent un cycle hétérocyclique saturé à 4 à 6 chaînons, éventuellement substitué par un ou plusieurs substituants indépendamment sélectionné(s) parmi halogène, hydroxy, alkyle en C₁₋₄, alkoxy en C₁₋₄, trifluorométhyle et trifluorométhoxy ;
chaque R⁵ est indépendamment sélectionné parmi hydrogène et alkyle en C₁₋₄;
chaque R⁶ est indépendamment sélectionné parmi alkyle en C₁₋₄, fluoro-alkyle en C₁₋₄, hydroxy-alkyle en C₁₋₄ et cycloalkyle en C₃₋₆;
chaque R^{7A} et R^{7B} est indépendamment sélectionné parmi le groupe constitué d'hydrogène, alkyle en C₁₋₄, hydroxy-alkyle en C₁₋₄, alkoxy en C₁₋₄-alkyle en C₁₋₄, cycloalkyle en C₃₋₆, phényle-alkyle en C₁₋₄, hétéroaryle-alkyle en C₁₋₄, cycloalkyle en C₃₋₆-alkyle en C₁₋₄ et hétérocyclyle-alkyle en C₁₋₄ et dans lequel tout résidu du cycle est éventuellement substitué par un ou plusieurs substituants indépendamment sélectionné(s) parmi halogène, hydroxy, alkyle en C₁₋₄, alkoxy en C₁₋₄, trifluorométhyle et trifluorométhoxy ;
ou indépendamment R^{7A} et R^{7B}, avec l'atome d'azote auquel ils sont liés, représentent un cycle hétérocyclique saturé à 4 ou 6 chaînons, qui éventuellement contient un hétéroatome additionnel sélectionné parmi azote, soufre ou oxygène, et
lequel cycle est éventuellement substitué par un ou plusieurs substituants indépendamment sélectionné(s) parmi halogène, hydroxy, alkyle en C₁₋₄, alkoxy en C₁₋₄, hydroxy-alkyle en C₁₋₄, -S(O)₂NR^{8A}R^{8B} et -C(O)NR^{8A}R^{8B};
chaque R^{8A} et R^{8B} est indépendamment sélectionné parmi hydrogène et alkyle en C ₁₋₄;
m représente 1, 2, 3 ou 4 ; et
n représente 0, 1 ou 2 ;
à condition que le composé n'est pas sélectionné parmi le groupe constitué de:
• 4-[3-(hydroxyméthyle)-6-méthylimidazo[1,2-a]pyridine-2-yl]-benzonitrile ;
• 2-(4-éthylphényle)-6-méthyle-imidazo[1,2-a]pyridine-3-méthanol ;
• 2-(2-nitrophényle)-imidazo[1,2-a]pyridine-3-méthanol ;
• 2-(3,4-diméthylphényle)-6-méthyle-imidazo[1,2-a]pyridine-3-méthanol ;
• 6-chloro-2-(4-chlorophényle)-imidazo[1,2-a]pyridine-3-méthanol ;
• 2-(4-fluorophényle)-imidazo[1,2-a]pyridine-3-méthanol ;
• 2-(2-chloro-4-méthoxyphényle)-6-(1-éthylpropyle)-8-méthyle-imidazo[1,2-a]pyridine-3-méthanol ;
• 2-(4-chlorophényle)-imidazo[1,2-a]pyridine-3-propanol ;
• 2-(4-méthylphényle)-6-(trifluorométhyle)-imidazo[1,2-a]pyridine-3-méthanol ;
• 7-méthyle-2-(2-naphtalényle)-imidazo[1,2-a]pyridine-3-méthanol ;
• 2-(4-méthylphényle)-6-nitro-imidazo[1,2-a]pyridine-3-méthanol ;
• 2-[1,1'-biphényle]-4-yl-7-méthyle-imidazo[1,2-a]pyridine-3-méthanol ;
• 6-méthyle-2-[4-(trifluorométhyle)phényle]-imidazo[1,2-a]pyridine-3-méthanol ;
• 2-(4-chlorophényle)-imidazo[1,2-a]pyridine-3-méthanol ;
• 6-méthyle-2-(4-méthylphényle)-imidazo[1,2-a]pyridine-3-éthanol ;
• 6-méthyle-2-(4-méthylphényle)-imidazo[1,2-a]pyridine-3-méthanol ;
• éthyle 4-[3-(hydroxyméthyle)-6-méthylimidazo[1,2-a]pyridine-2-yI]-benzoate ;
• 2-[4-(méthylsulfonyle)phényle]-imidazo[1,2-a]pyridine-3-méthanol ;
• 2-(4-chlorophényle)-6-méthyle-imidazo[1,2-a]pyridine-3-méthanol ;
• 6-méthyle-2-(4-nitrophényle)-imidazo[1,2-a]pyridine-3-méthanol ;
• 6-chloro-2-(4-méthylphényle)-imidazo[1,2-b]pyridazine-3-méthanol ;
• 3-(diéthylamino)-2-méthyle-6-(4-méthylphényle)-imidazo[1,2-b][1,2,4]triazine-7-méthanol ;
• 6-(4-chlorophényle)-2,3-diphényle-imidazo[1,2-b][1,2,4]triazine-7-méthanol ;
• 6-(4-bromophényle)-imidazo[2,1-b]thiazole-5-méthanol ;
• 6-(4-chlorophényle)-imidazo[2,1-b]thiazole-5-méthanol ;
• 6-(4-chlorophényle)-2-méthyle-imidazo[2,1-b]thiazole-5-méthanol ; • 6-(4-mémylphényle)-imidazo[2,1-b]thiazole-5-méthanol ;
• 6-[1,1'-biphényle]-4-yl-imidazo[2,1-b]thiazole-5-méthanol ;
• 6-(4-bromophényle)-2-(2-furanyle)-imidazo[2,1-b]-1,3,4-thiadiazole-5-méthanol ;
• 6-(4-bromophényle)-2-(2-thiényle)-imidazo[2,1-b]-1,3,4-thiadiazole-5-méthanol ; et
• 6-(4-bromophényle)-2-cyclohexyle-imidazo[2,1-b]-1,3,4-thiadiazole-5-méthanol.

2. Composé selon la revendication 1, dans lequel le cycle hétéroaromatique est sélectionné parmi:

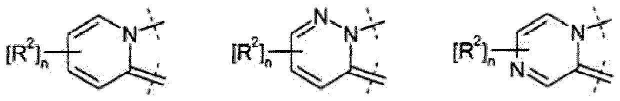

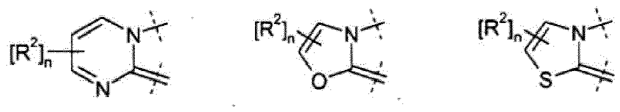

3. Composé selon la revendication 1 ou 2, dans lequel A représente -CH₂-.

4. Composé selon l'une quelconque des revendications 1 à 3, dans lequel R¹ est indépendamment sélectionné parmi halogène, alkyle en C₁₋₄, fluoro-alkyle en C₁₋₄, et alkoxy en C₁₋₄.

5. Composé selon l'une quelconque des revendications 1 à 4, dans lequel R² est indépendamment sélectionné parmi halogène, cyano, nitro, alkyle en C₁₋₄, fluoro-alkyle en C₁₋₄, hydroxy-alkyle en C₁₋₄ et alkoxycarbonyle en C₁₋₄ et -C(O)NR^{7A}R^{7B}.

6. Composé selon la revendication 1, qui est sélectionné parmi:
• [2-(4-Méthylphényle)imidazo[1,2-a]pyridine-3-yl]méthanol ;
• [2-(2,4-Dichlorophényle)imidazo[1,2-a]pyridine-3-yl]méthanol ;
• [2-(4-Bromophényle)-8-méthylimidazo[1,2-a]pyridine-3-yl]méthanol ;
• [7-Méthyle-2-(4-méthylphényle)imidazo[1,2-a]pyridine-3-yl]méthanol ;
• [2-(4-Bromophényle)-7-méthylimidazo[1,2-a]pyridine-3-yl]méthanol ;
• [2-(4-Bromophényle)-7-éthylimidazo[1,2-a]pyridine-3-yl]méthanol ;
• [2-(2-Chlorophényle)-7-méthylimidazo[1,2-a]pyridine-3-yl]méthanol ;
• [2-(2,4-Dichlorophényle)-7-méthylimidazo[1,2-a]pyridine-3-yl]méthanol ;
• [2-(3,4-Dichlorophényle)-7-méthylimidazo[1,2-a]pyridine-3-yl]méthanol ;
• [6-Méthyle-2-(2-naphtyle)imidazo[1,2-a]pyridine-3-yl]méthanol ;
• [2-(3-Mémoxyphényle)-6-méthylimidazo[1,2-a]pyridine-3-yl]méthanol ;
• 4-[3-(Hydroxyméthyle)-6-méthylimidazo[1,2-a]pyridine-2-yl-benzonitrile ;
• [6-Méthyle-2-(3-nitrophényle)imidazo[1,2-a]pyridine-3-yl]méthanol ;
• [2-(4-Fluorophényle)-6-méthylimidazo[1,2-a]pyridine-3-yl]méthanol ;
• [2-(4-Iodophényle)-6-méthylimidazo[1,2-a]pyridine-3-yl]méthanol ;
• [2-(2-Chlorophényle)-6-méthylimidazo[1,2-a]pyridine-3-yl]méthanol ;
• (2-{4-[(2-Aminoéthyle)amino]phényle}-6-méthylimidazo[1,2-a]pyridine-3-yl)méthanol ;
• 1 -[2-(4-Chlorophényle)-6-méthylimidazo[1,2-a]pyridine-3-yl]éthanol ;
• [2-(2,4-Dichlorophényle)-6-méthylimidazo[1,2-a]pyridine-3-yl]méthanol ;
• [2-(3-Mémoxyphényle)-6-(trifluorométhyle)imidazo[1,2-a]pyridine-3-yl]méthanol ;
• [2-(4-Chlorophényle)-6-(trifluorométhyle)imidazo[1,2-a]pyridine-3-yl]méthanol ;
• [2-(4-Bromophényle)-6-(trifluorométhyle)imidazo[1,2-a]pyridine-3-yl]méthanol ;
• [7-Chloro-2-(4-chlorophényle)imidazo[1,2-a]pyridine-3-yl]méthanol ;
• [2-(4-Bromophényle)-7-chloroimidazo[1,2-a]pyridine-3-yl]méthanol ;
• [7-Chloro-2-(2,4-dichlorophényle)imidazo[1,2-a]pyridine-3-yl]méthanol ;
• [7-Chloro-2-(2,4-difluorophényle)imidazo[1,2-a]pyridine-3-yl]méthanol ;
• [6-Bromo-2-(3-mémoxyphényle)imidazo[1,2-a]pyridine-3-yl]méthanol ;
• [6-Bromo-2-(4-fluorophényle)imidazo[1,2-a]pyridine-3-yl]méthanol ;
• [6-Bromo-2-(4-bromophényle)imidazo[1,2-a]pyridine-3-yl]méthanol ;
• [2-(4-Bromophényle)-6-chloroimidazo[1,2-a]pyridine-3-yl]méthanol ;
• [2-(4-Chlorophényle)-6-fluoroimidazo[1,2-a]pyridine-3-yl]méthano] ;
• [6-Bromo-2-(2,4-difluorophényle)imidazo[1,2-a]pyridine-3-yl]méthanol ;
• [6-Chloro-2-(2,4-difluorophényle)imidazo[1,2-a]pyridine-3-yl]méthanol ;
• [6-Bromo-2-(2,4-dichlorophényle)imidazo[1,2-a]pyridine-3-yl]méthanol ;
• [6-Chloro-2-(2,4-dichlorophényle)imidazo[1,2-a]pyridine-3-yl]méthanol ;
• [6-Bromo-2-(3,4-difluorophényle)imidazo[1,2-a]pyridine-3-yl]méthanol ;
• [6-Bromo-2-(3-chloro-4-fluorophényle)imidazo[1,2-a]pyridine-3-yl]méthanol ;
• [6-Chloro-2-(3-chloro-4-fluorophényle)imidazo[1,2-a]pyridine-3-yl]méthanol ;
• [6-8-Dichloro-2-(3-méthoxyphényle)imidazo[1,2-a]pyridine-3-yl]méthanol ;
• [2-(4-Bromophényle)-6-8-dichloroimidazo[1,2-a]pyridine-2-3-yl]méthanol ;
• 2-(4-Bromophényle)-3-(hydroxyméthyle)imidazo[1,2a]pyridine-6-carbonitrile ;
• Méthyle 2-(4-bromophényle)-3-(hydroxyméthyle)imidazo[1,2-a]pyridine-6-carboxylate ;
• Méthyle 2-(4-chlorophényle)-3-(hydroxyméthyle)imidazo[1,2-a]pyridine-6-carboxylate ;
• [2-(4-Bromophényle)imidazo[1, 2-a]pyridine-3,7-diyl]diméthanol ;
• [2-(4-Chlorophényle)imidazo[1,2-a]pyridine-3, 6-diyl]diméthanol ;
• [2-(4-Chlorophényle) -6-nitroimidazo[1,2a]pyridine-3-yl]méthanol ;
• [2-(4-Bromophényle) -6-nitroimidazo[1,2a]pyridine-3-yl]méthanol ;
• {2-(4-Chlorophényle) -6-[(4-méthoxypipéridine-1-yl)carbonyle]imidazo[1,2a]pyridine-3-yl} méthanol ;
• 2-(4-Chlorophényle)- 3-(hydroxyméthyle)-N-(3-méthoxypropyle)imidazo[1,2a]pyridine-6-carboxamide ;
• 2-(4-Chlorophényle)-3-(hydroxyméthyle)-N-(2-méthoxyéthyle)imidazo[1,2-a]pyridine-6-carboxamide ;
• [2-(4-Chlorophényle)- 6-(morpholine-4-ylcarbonyle)imidazo[1,2-a]pyridine-3-yl]méthanol ;
• 2-(4-Chlorophényle)-3-(hydroxyméthyle)-N,N-diméthylimidazo[1,2-a]pyridine-6-carboxamide ;
• 2-(4-Chlorophényle)-3-(hydroxyméthyle)-N-méthylimidazo[1,2-a]pyridine-6-carboxamide ;
• 2-(4-Chlorophényle)-3-(hydroxyméthyle)-N-[-2 (1-méthylpyrrolidine-2-yl)éthyle]imidazo[1,2-a]pyridine-6-carboxamide ;
• {2-(4-Chlorophényle)- 6-[(4-méthylpiperazine-1-yl)carbonyle]imidazo[1,2-a]pyridine-3-yl}méthanol ;
• 2-(4-Chlorophényle)-N-(3, 4-diméthoxybenzyle)- 3-(hydroxyméthyle)imidazo[1,2-a]pyridine-6-carboxamide ;
• 2-(4-Chlorophényle)-3-(hydroxyméthyle)-N-[2-(1H-imidazol-4-yl)éthyle]imidazo[1,2-a]pyridine-6-carboxamide ;
• 2-(4-Chlorophényle)-3-(hydroxyméthyle)-N-(pyridine-3-ylméthyle)imidazo[1,2-a]pyridine-6-carboxamide ;
• 2-(4-Chlorophényle)-3-(hydroxyméthyle)-N-(3-hydroxypropyl)imidazo[1,2-a]pyridine-6-carboxamide ;
• (1-{[2-(4-Chlorophényle)-3-(hydroxyméthyle)imidazo[1,2-a]pyridine-6-yl]carbonyle}pipéridine-4-yl)méthanol ;
• 2-(4-Chlorophényle)-3-(hydroxyméthyle)-N-(2-hydroxypropyle)imidazo[1,2-a]pyridine-6-carboxamide ;
• 2-(4-Chlorophényle)-N-(*trans*-4-hydroxycyclohexyle)-3-(hydroxyméthyle)imidazo[1,2-a]pyridine-6-carboxamide ;
• 1-{[2-(4-Chlorophényle)-3-(hydroxyméthyle)imidazo[1,2-a]pyridine-6-yl]carbonyle}pipéridine-4-ol ;
• (3R)-1-{[2-(4-Chlorophényle)-3-(hydroxyméthyle)imidazo[1,2-a]pyridine-6-yl]carbonyle} pyrrolidine-3-ol ;
• 1-{[2-(4-Chlorophényle)-3-(hydroxyméthyle)imidazo[1,2-a]pyridine-6-yl]carbonyle}pyrrolidme-3-ol ;
• 1-{[2-(4-Chlorophényle)-3-(hydroxyméthyle)imidazo[1,2-a]pyridine-6-yl] carbonyle}azétidine-3-ol ;
• 2-(4-Chlorophényle)-3-(hydroxyméthyle)imidazo[1,2-a]pyridine-7-carboxarnide ;
• 3-(Hydroxyméthyle)-2-(3-mémoxyphényle)imidazo[1,2-a]pyridine-6-carboxamide ;
• 2-(4-Chlorophényle)-3-(hydroxyméthyle)imidazo[1,2-a]pyridine-6-carboxamide ;
• 2-(4-Fluorophényle)-3-(hydroxyméthyle)imidazo[1,2-a]pyridine-6-carboxamide ;
• 2-(2,4-Difluorophényle)-3-(hydroxyméthyle)imidazo[1,2-a]pyridine-6-carboxamide ;
• 2-(2,4-Dichlorophényle)-3-(hydroxyméthyle)imidazo[1,2-a]pyridine-6-carboxamide ;
• 2-(3,4-Difluorophényle)-3-(hydroxyméthyle)imidazo[1,2-a]pyridine-6-carboxamide ;
• [6-amino-2-(4-chlorophényle)imidazo[1,2-a]pyridine-3-yl]méthanol ;
• N-[2-(4-chlorophényle)-3-(hydroxyméthyle)imidazo[1,2-a]pyridine-6-yl]acétamide ;
• [6-amino-2-(4 bromophényle)imidazo[1,2-a]pyridine-3-yl]méthanol ;
• [6-chloro-2-(4-chlorophényle)imidazo[1,2-b]pyridazine-3-yl]méthanol ;
• [2-(4-Chlorophényle)imidazo[1,2-a]pyrazine-3-yl]méthanol ;
• [6-Bromo-2-(3-méthoxyphényle)imidazo[1,2-a]pyrazine-3-yl]méthanol ;
• {6-Bromo-2-[4-(trifluorométhyle)phényle]imidazo[l,2-a]pyrazine-3-yl}méthanol ;
• [6-Bromo-2-(4-fluorophényle)imidazo[1,2-a]pyrazine-3-yl]méthanol ;
• [6-Bromo-2-(4-chlorophényle)imidazo[1,2-a]pyrazine-3-yl]méthanol ;
• [6-Bromo-2-(4-bromophényl)imidazo[1,2-a]pyrazine-3-yl]méthanol ;
• [6-Bromo-2-(2,4-dichlorophényle)imidazo[1,2-a]pyrazine-3-yl]méthanol;
• [6-Bromo-2-(2,4-difluorophényle)imidazo[1,2-a]pyrazine-3-yl]méthanol ;
• [6-Bromo-2-(4-chloro-2-fluoro-5-méthylphényle)imidazo[1,2-a]pyrazine-3-yl]méthanol ;
• [2-(1-Benzofurane-5-yl)-6-bromoimidazo[1,2-a]pyrazine-3-yl]méthanol ;
• [6-Bromo-2-(2,3-dihydro-1,4-benzodioxine-5-yl)imidazo[1,2-a]pyrazine-3-]méthanol ;
• [6-amino-2-(4-fluorophényle)imidazo[1,2-a]pyrazine-3-yl]méthanol ;
• [6-amino-2-(4-chlorophényle)imidazo[1,2-a]pyrazine-3-yl]méthanol ;
• [6-Amino-2-(4-bromophényle)imidazo[1,2-a]pyrazine-3-yl]méthanol ;
• [6-(Azétidine-1-yl)-2-(4-fluorophényle)imidazo[1,2-a]pyrazine-3-yl]méthanol ;
• [2-(4-Chlorophényle)imidazo[1,2-a]pyrimidine-3-yl]méthanol ;
• [2-(2,4-Dichlorophényle)imidazo[1,2-a]pyrimidine-3-yl]méthanol ;
• [6-(4-fluorophényle)-2-méthylimidazo[2,1-b][1,3]oxazol-5-yl]méthanol ;
• [6-(2,4-dichlorophényle)imidazo[2,1-b][1,3]thiazol-5-yl]méthanol ;
• [6-(4-Bromophényle)-2-méthlimidazo[2,1-b][1,3]thiazol-5-yl]méthanol;
• [6-(2,4-Dichlorophényle)-2-méthylimidazo[2,1-b][1,3]thiazol-5-yl]méthanol ;
• [2-Chloro-6-(4-chlorophényle)imidazo[2,1-b][1,3]thiazol-5-yl]méthanol ;
• Méthyle 6-(4-chlorophényle)-5-(hydroxyméthyle)imidazo[2,1-b][1,3]thiazole-2-carboxylate ;
• [6-(4-Chlorophényle)imidazo[2,1-b][1,3]thiazole-2,5-diyl]diméthanol ;
• 1-[6-(4-Chlorophényle)-5-(hydroxyméthyle)imidazo[2,1-b][1,3]thiazol-2-yl]éthanol ;
• [6-(4-Chlorophényle)-5-(hydroxyméthyle)imidazo[2,1-b][1,3]thiazol-2-yl](cyclopropyle)méthanol ;
• 2-[6-(4-Chlorophényle)-5-(hydroxyméthyle)imidazo[2,1-b][1,3]thiazol-2-yl]propane-2-ol;
• 6-(4-Chlorophényle)-N-éthyle-5-(hydroxyméthyle)-N-méthylimidazo[2,1-b] [1,3]thiazole-2-carboxamide ;
• [6-(4-Chlorophényle)-2-(morpholine-4-ylcarbonyle)imidazo[2,1-b][1,3]thiazol-5-yl]méthanol ;
• {6-(4-Chlorophényle)-2-[(4-méthylpipérazine-1-yl)carbonyle]imidazo[2,1-b][1,3]thiazol-5-yl}méthanol ; et
• 6-(4Chlorophényle)-5-(hydroxyméthyle)-N-propylimidazol[2,1-b][1,3]thiazole-2-carboxamide.

7. Formulation pharmaceutique renfermant un composé selon l'une quelconque des revendications 1 à 6 en tant que principe actif, en association à un diluant ou à un excipient pharmaceutiquement acceptable.

8. Composé selon l'une quelconque des revendications 1 à 6 destiné à être utilisé en thérapie.

9. Composé de la formule (I), 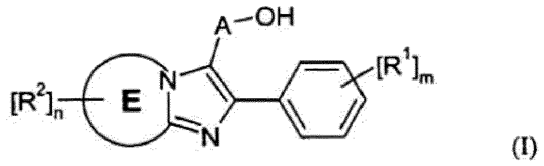 ou un sel pharmaceutiquement acceptable, un solvate, un hydrate, un isomère géométrique, un tautomère, un isomère optique ou un N-oxyde de ces derniers, dans lequel :
E est un cycle hétéroaromatique à 5 ou 6 chaînons ;
A est un alkylène en C₁₋₃, qui est éventuellement substitué par alkyle en C₁₋₃ ;
chaque R¹ est indépendamment sélectionné parmi le groupe constitué d'halogène, cyano, nitro, alkyle en C₁₋₄, fluoro-alkyle en C₁₋₄, cycloalkyle en C₃₋₆, hétérocyclyle, phényle, hétéroaryle, alkylène en C₀₋₄-OR³, alkylène en C₀₋₄-SR³, alkylène en C₀₋₄-NR^{4A}R^{4B}, -N(R⁵)-alkylène en C₂₋₄-NR^{4A}R^{4B}, -C(O)R³, -C(O)OR³, -C(O)NR^{4A}R^{4B}, -N(R^{s})C(O)R⁶, -N(R⁵)C(O)NR^{4A}R^{4B}, - N(R⁵)C(O)OR⁶, -N(R⁵)S(O)₂R⁶, -S(O)₂NR^{4A}R^{4B} et -S(0)₂R⁶, et dans lequel tout reste du cycle est éventuellement substitué par un ou plusieurs substituants indépendamment sélectionnés parmi halogène, cyano, hydroxy, alkyle en C₁₋₄, alkoxy en C₁₋₄, trifluorométhyle et trifluorométhoxy ; ou
deux substituants adjacents R¹, qui avec les atomes de carbone auxquels ils sont liés, représentent un cycle aromatique ou partiellement insaturé à 5 ou 6 chaînons qui contient éventuellement un ou deux hétéroatomes sélectionnés parmi azote, soufre ou oxygène et lequel cycle est éventuellement substitué par un ou plusieurs substituants indépendamment sélectionné(s) parmi halogène, cyano, hydroxy, alkyle en C₁₋₄, alkoxy en C₁₋₄, trifluorométhyle et trifluorométhoxy ;
chaque R² est indépendamment sélectionné parmi le groupe constitué de halogène, cyano, nitro, alkyle en C₁₋₄, fluoro-alkyle en C₁₋₄, cycloalkyle en C₃₋₆, hétérocyclyle, phényle, hétéroaryle, cycloalkyle en C₃₋₆-alkyle en C₁₋₄, cycloalkyle en C₃₋₆(hydroxy)-alkyle en C₁₋₄, hétérocyclyle-alkyle en C₁₋₄, cycloalkylcarbonyle en C₃₋₆, hétérocyclylcarbonyle, benzoyle, hétéroarylcarbonyle, alkylène en C₀₋₄-OR³, alkylène en C₀₋₄-SR³, alkylène en C₀₋₄, alkylène en C₀₋₄-C(O)R⁶, alkylène en C₀₋₄-C(O)OR³, alkylène en C₀₋₄-C(O)NR^{7A}R^{7B}, alkylène en C₀₋₄-N(R⁵)C(O)R⁶, alkylène en C₀₋₄-N(R⁵)C(O)NR^{7A}R^{7B}, alkylène en C₀₋₄N(R⁵)C(O)OR⁶, alkylène en C₀₋₄-N(R⁵)S(O)₂R⁶, alkylène en C₀₋₄-S(O)2NR^{7A}R^{7B}, alkylène en C₀₋₄-S(O)₂R⁶, amidino et guanidino, et dans lequel tout reste du cycle est éventuellement substitué par un ou plusieurs substituants indépendamment sélectionnés parmi halogène, cyano, hydroxy, alkyle en C₁₋₄, alkoxy en C₁₋₄, trifluorométhyle, trifluorométhoxy et -C(O)NR^{8A}R^{8B} ;
chaque R³ est indépendamment sélectionné parmi hydrogène, alkyle en C₁₋₄, fluoro-alkyle en C₁₋₄, hydroxy-alkyl en C₁₋₄ et cycloalkyle en C₃₋₆;
chaque R^{4A} et R^{4B} est indépendamment sélectionné parmi hydrogène, alkyle en C₁₋₄, hydroxy-alkyle en C₁₋₄ et alkylènene-NR^{8A}R^{8B};
ou indépendamment R^{4A} et R^{4B}, avec l'atome d'azote auquel ils sont liés, représentent un cycle hétérocyclique saturé à 4 à 6 chaînons, qui est éventuellement substitué par un ou plusieurs substituants indépendamment sélectionné(s) parmi halogène, hydroxy, alkyle en C₁₋₄, alkoxy en C₁₋₄, trifluorométhyle et trifluorométhoxy ;
chaque R⁵ est indépendamment sélectionné parmi hydrogène et alkyle en C₁₋₄ ;
chaque R⁶ est indépendamment sélectionné parmi alkyle en C₁₋₄, fluoro-alkyle en C₁₋₄, hydroxy-alkyle en C₁₋₄ et cycloalkyle en C₃₋₆ ;
chaque R^{7A} et R^{7B} est indépendamment sélectionné parmi le groupe constitué d'hydrogène, alkyle en C₁₋₄, hydroxy-alkyle en C₁₋₄, alkoxy en C₁₋₄-alkyle en C₁₋₄, cycloalkyle en C₃₋₆, phényle-alkyle en C₁₋₄, hétéroaryle-alkyle en C₁₋₄, cycloalkyle en C₃₋₆-alkyle en C₁₋₄ et hétérocyclyle-alkyle en C₁₋₄, et dans lequel tout résidu du cycle est éventuellement substitué par un ou plusieurs substituants indépendamment sélectionnés parmi halogène, cyano, hydroxy, alkyle en C₁₋₄, alkoxy en C₁₋₄, trifluorométhyle et trifluorométhoxy ;
ou indépendamment R^{7A} et R^{7B} , avec l'atome d'azote auquel ils sont liés, représentent un cycle hétérocyclique saturé à 4 à 6 chaînons, qui contient éventuellement un hétéroatome additionnel sélectionné parmi azote, soufre ou oxygène, et lequel cycle est éventuellement substitué par un ou plusieurs substituants indépendamment sélectionné(s) parmi halogène, hydroxy, alkyle en C₁₋₄, alkoxy en C₁₋₄, hydroxy-alkyle en C₁₋₄, -S(O)₂NR^{8A}R^{8B} et -C(O)NR^{8A}R^{8B} ;
chaque R^{8A} et R^{8B} est indépendamment sélectionné parmi hydrogène et alkyle en C₁₋₄ ;
m représente 1, 2, 3 ou 4 ; et
n représente 0, 1 ou 2,
pour utilisation dans le traitement ou la prévention d'une inflammation, d'une maladie inflammatoire, d'un trouble immun ou auto-immun.

10. Composé selon la revendication 9, dans lequel l'inflammation ou la maladie inflammatoire ou le trouble immun ou auto-immun est arthrite (y compris arthrite rhumatoïde, arthrite rhumatoïde juvénile, ostéoarthrite et arthrite psoriasique), synovite, vasculite, une condition associé à une inflammation des intestins (y compris maladie de Crohn, colite ulcérative, maladie inflammatoire des intestins et syndrome du côlon irritable), athérosclérose, sclérose multiple, maladie d'Alzheimer, démence vasculaire, maladie inflammatoire pulmonaire (y compris asthme, broncho-pneumopathie chronique obstructive et le syndrome de détresse respiratoire aiguë), maladie fibreuse (y compris fibrose pulmonaire idiopathique, fibrose cardiaque et sclérose systémique (sclérodermie), une maladie inflammatoire de la peau (y compris dermatite de contact, dermatite atopique et psoriasis), syndrome de réponse inflammatoire systémique, sepsis, condition inflammatoire et/ou auto-immune du foie (y compris hépatite auto-immune, cirrhose biliaire primitive, maladie du foie liée à l'alcool, cholangite sclérosante et la cholangite auto-immune), diabète (type I ou II) et/ou les complications de ces maladies, insuffisance cardiaque chronique, insuffisance cardiaque congestive, maladie ischémique (y compris congestion cérébrale et lésion de reperfusion due à l'ischémie) ou infarctus du myocarde et/ou des complications de ces maladies.

11. Composé selon la revendication 9, dans lequel la maladie inflammatoire est la vasculite.
